# EUROPEAN PATENT APPLICATION

(11) **EP 4 447 056 A1**
(43) Date of publication of application: **16.10.2024**
(21) Application number: 24170216.6
(22) Date of filing: 15.04.2024
(51) Int. Cl.: G16B 25/10, G16B 40/20

(54) **SYSTEMS AND METHODS FOR PREDICTING CLINICAL RESPONSE**

(30) Priority: 13.04.2023 US 202363495975 P
(71) Applicant: Tempus AI, Inc., Chicago, IL 60654 (US)
(72) Inventor: CARTY, Mark, Chicago, Illinois (US); PELOSSOF, Raphael, Chicago, Illinois (US); AHMED, Talal, Chicago, Illinois (US); SALAHUDEEN, Ameen, Chicago, Illinois (US)
(74) Representative: Grund, Martin

(57) **Abstract**

The disclosure provides methods and systems for predicting an effect of a pharmaceutical agent in a test subject of a first species. Information about the test subject is input into a multi-task model comprising a plurality of parameters. The model applies the plurality of parameters to the information about the test subject through a plurality of instructions to generate, as output from the multi-task model, a plurality of outputs including a predicted effect of the pharmaceutical agent in the test subject and, for each respective cell type variable in a set of one or more cell type variables, a corresponding cell type classification. The information about the test subject includes a plurality of abundance values including, for each respective cellular constituent in a plurality of cellular constituents, a corresponding abundance value for the abundance of the respective cellular constituent in a biological sample of the test subject.

## Description

### BACKGROUND

The statements in this section merely provide background information related to the present disclosure and may not constitute prior art.

Precision oncology is the practice of tailoring cancer therapy to the unique genomic, epigenetic, and/or transcriptomic profile of an individual tumor. This is in contrast to conventional methods for treating a cancer patient based merely on the type of cancer the patient is afflicted with, e.g., treating all breast cancer patients with a first therapy and all lung cancer patients with a second therapy. Precision oncology was borne out of many observations that different patients diagnosed with the same type of cancer responded very differently to common treatment regimes. Over time, researchers have identified genomic, epigenetic, and transcriptomic markers that facilitate some level of prediction as to how an individual cancer will respond to a particular treatment modality.

Genomic analysis of tumors is rapidly becoming routine clinical practice to provide tailored patient treatments and improve outcomes. *See* Femandes et al. 2017 Clinics 72, 588-594. Indeed, recent studies indicate that clinical care is guided by NGS assay results for 30-40% of patients receiving such testing. *See* Hirshfield et al. 2016 Oncologist 21, 1315-1325; Groisberg et al. 2017 Oncotarget 8, 39254-39267; Ross et al. JAMA Oncol. 1, 40-49; and Ross et al. 2015 Arch. Pathol. Lab Med. 139, 642-649. There is growing evidence that patients who receive therapeutic advice guided by genetics have better outcomes. *See,* for example Wheler *et al.* who used matching scores (*e.g.,* scores based on the number of therapeutic associations and genomic aberrations per patient) to demonstrate that patients with higher matching scores have a greater frequency of stable disease, longer time to treatment failure, and greater overall survival (2016 Cancer Res. 76, 3690-3701). Such methods may be particularly useful for patients who have already failed multiple lines of therapy.

Targeted therapies have shown significant improvements in patient outcomes, especially in terms of progression-free survival. *See* Radovich et al. 2016 Oncotarget 7, 56491-56500. Further, recent evidence reported from the IMPACT trial, which involved genetic testing of advanced stage tumors from 3,743 patients and where approximately 19% of patients received matched targeted therapies based on their tumor biology, showed a response rate of 16.2% in patients with matched treatments versus 5.2% in patients with non-matched treatments. *See* Bankhead. "IMPACT Trial: Support for Targeted Cancer Tx Approaches." MedPageToday. June 5, 2018. The IMPACT study further found that the three-year overall survival for patients given a molecularly matched therapy was more than twice that of non-matched patients (15% vs. 7%). *See Id.* and ASCO Post. "2018 ASCO: IMPACT Trial Matches Treatment to Genetic Changes in the Tumor to Improve Survival Across Multiple Cancer conditions." The ASCO POST. June 6, 2018. Estimates of the proportion of patients for whom genetic testing changes the trajectory of their care vary widely, from approximately 10% to more than 50%. *See* Femandes et al. 2017 Clinics 72, 588-594.

In fact, therapy targeted to specific genomic alterations is already the standard of care in several tumor types (*e.g.,* as suggested in the National Comprehensive Cancer Network (NCCN) guidelines for melanoma, colorectal cancer, and non-small cell lung cancer). These few, yet well-known mutations in the NCCN guidelines can be addressed with individual assays or small next generation sequencing (NGS) panels. However, for targeted therapy to be most effective, the patient's individual genomic and transcriptomic should be considered more wholistically. However, it is more difficult to model therapeutic effects against large portions of genome and/or transcriptome than one or a few genomic loci or mRNA expression levels. Moreover, human patient data for a therapy, particularly for a candidate therapy prior to regulatory approval, is limited.

Tumor organoids (TOs) are three dimensional cultures of cancerous cells derived from tumor tissues and are similar to the original cancer source in terms of genome and function. Tumor organoids can imitate the pathological characteristics of primary tissue at the organ level and better simulate the tumor in vivo as compared to traditional models. Further, tumor organoid inheritance and morphology remain stable after many generations. Thus, tumor organoids potentially provide a powerful tool in cancer research and personalized treatment. Such tumor organoid systems can be used, for example, for drug discovery and clinical treatment response studies, as well as for disease modelling and development studies. Tumor organoid models have been developed for several different types of cancers including stomach cancer (Vlachogiannis et al., Science 359: 920-6 (2018)), intestinal cancer (Vlachogiannis et al., Science 359: 920-6 (2018)), liver cancer (Broutier et al., Nat. Med. 23:1424-34 (2017)), pancreatic cancer (Boj et al., Cell 160: 324-38 (2015)), breast cancer (Sachs et al., Cell 172:373-86 (2018)), bladder cancer (Lee et al., Cell 173:515-28 (2018)),prostate cancer (Gao et al., Cell. 159: 176-187 (2014); and Puca et al., Nat. Commun. 9:2404 (2018)), and head and neck cancer (Driehuis et al., Cancer Discovery 9:852-871 (2019)). However, it is known that transcriptomic patterns in tumor organoid models vary significantly from transcriptomic patterns of the disease in vivo.

### SUMMARY

As evident from the description above, there remains a need in the art for methods and systems for modeling the effects of therapies on personalized patient genomes. The methods and systems described herein satisfy these and other needs by providing a method that (i) corrects technical biases between organoid and human RNA-expression datasets, (ii) exploits organoid-specific-drug responses to predict drug response on human expression data, and (iii) accounts for the confounders within the organoid and human samples. In some embodiments, confounders are accounted for by modeling the confounder variables (e.g., histology, stage, grade, etc.) as tasks similar to the task of predicting drug response. In some embodiments, a multi-task model is described herein that not only addresses the confounders and technical biases across organoid and human samples but also learns a transferable drug-response mapping from organoids to patients. In some embodiments, the algorithm's output is a recommendation for patients that are similar to sensitive and resistant organoids. The algorithm also allows for the integration of organoid and human molecular datasets into a combined database, improving the ability to detect drug-specific biomarkers and model drug response.

In one aspect, a method is provided for predicting an effect of a pharmaceutical agent in a test subject of a first species. The method includes inputting information about the test subject into a multi-task model including a plurality of parameters, where the multi-task model applies the plurality of parameters to the information about the test subject through a plurality of instructions to generate, as output from the multi-task model, a plurality of outputs including (i) a predicted effect of the pharmaceutical agent in the test subject and (ii) for each respective cell type variable in a set of one or more cell type variables, a corresponding cell type classification, where the information about the test subject includes a plurality of abundance values, the plurality of abundance values including, for each respective cellular constituent in a plurality of cellular constituents, a corresponding abundance value for the abundance of the respective cellular constituent in a biological sample of the test subject.

In another aspect, a method is provided for identifying one or more tissue organoids in a plurality of tissue organoids matching a biological property of a tissue in a subject. The method includes inputting information about the test subject into a multi-task model including a plurality of parameters and one or more hidden layers. The multi-task model is trained to apply the plurality of parameters to the information about the test subject through a plurality of instructions to generate, as output from the multi-task model, a plurality of outputs including (i) a predicted effect of the pharmaceutical agent in the test subject and (ii) for each respective cell type variable in a set of one or more cell type variables, a corresponding cell type classification. The information about the test subject includes, for each respective cellular constituent in a plurality of cellular constituents, a corresponding abundance value for the abundance of the respective cellular constituent in a biological sample of the test subject. The method also includes obtaining a latent representation of the information about the test subject from a respective hidden layer in the one or more hidden layers. The method also includes comparing the latent representation of the information about the test subject to a plurality of latent representations, where each respective latent representation in the plurality of latent representations is of information about a respective tissue organoid, in a plurality of tissue organoids, obtained from the multi-task model. The method also includes identifying one or more respective tissue organoids, in the plurality of tissue organoids, that satisfy a set of one or more similarity criterion based on the comparing, thereby identifying the one or more tissue organoids matching the biological property of the tissue in the subject.

In another aspect, a method is provided for training a model to predict an effect of a candidate pharmaceutical agent in a test subject of a first species. The method includes obtaining, for each respective training sample in a first plurality of training samples, where each respective training sample in the first plurality of training samples includes a tissue organoid or tissue organoid culture, formed from cells of the first species, that has been exposed to a perturbation (i) a corresponding plurality of abundance values including, for each respective cellular constituent in a plurality of cellular constituents, a corresponding abundance value for the abundance of the respective cellular constituent in the respective training sample after exposure to the candidate pharmaceutical agent, (ii) a corresponding experimentally measured effect of the candidate pharmaceutical agent on the respective training sample, and (iii) a corresponding set of one or more cell type classifications including, for each respective cell type variable in a set of one or more cell type variables, a corresponding cell type classification for the respective cell type variable. The method also includes obtaining, for each respective training sample in a second plurality of training samples, where each respective training sample in the second plurality of training samples includes a biological sample from a respective subject in a plurality of subjects of the first species (i) a corresponding plurality of abundance values including, for each respective cellular constituent in the plurality of cellular constituents, a corresponding abundance value for the abundance of the respective cellular constituent in the respective training sample, and (ii) a corresponding set of one or more cell type classifications including, for each respective cell type variable in the set of one or more cell type variables, a corresponding cell type classification for the respective cell type variable. The method also includes performing a first dimensionality reduction analysis across the corresponding plurality of abundance values for each respective training sample in the first plurality of training samples, thereby learning a first mapping function that maps a corresponding plurality of abundance values including, for each respective cellular constituent in the plurality of cellular constituents, a corresponding abundance value for the abundance of the respective cellular constituent in a tissue organoid or tissue organoid culture, formed from cells of the first species, that has been exposed to a perturbation, into a first latent feature space including a first plurality of dimensions that is less than the number of cellular constituents in the plurality of constituents, and generating, for each respective training sample in the first plurality of training samples, a first corresponding representation of the corresponding plurality of abundance values in the first latent feature space according to the first mapping function. The method also includes performing a second dimensionality reduction analysis across the corresponding plurality of abundance values for each respective training sample in the second plurality of training samples, thereby learning a second mapping function that maps a corresponding plurality of abundance values including, for each respective cellular constituent in the plurality of cellular constituents, a corresponding abundance value for the abundance of the respective cellular constituent in a biological sample of the first species, into a second latent feature space including the first plurality of dimensions, and generating, for each respective training sample in the second plurality of training samples, a corresponding representation of the corresponding plurality of abundance values in the second latent feature space according to the second mapping function. The method also includes learning a third mapping function that maps a representation of a corresponding plurality of abundance values in the first latent feature space to the second latent feature space. The method also includes generating, for each respective training sample in the first plurality of training samples, a second corresponding representation of the corresponding plurality of abundance values in the second latent feature space according to the third mapping function. The method also includes inputting, for each respective training sample in the first plurality of training samples, corresponding information about the respective training sample into a multi-task model including a plurality of parameters, where the multi-task model applies the plurality of parameters to the information about the training subject through a plurality of instructions to generate, as output from the multi-task model, a corresponding plurality of outputs. The corresponding plurality of outputs includes (i) a predicted effect of the candidate pharmaceutical agent on the respective training sample and (ii) for each respective cell type variable in the set of one or more cell type variables, a corresponding cell type classification for the respective training sample. The information about the respective training sample includes the second corresponding representation of the corresponding plurality of abundance values in the second latent feature space. The method also includes inputting, for each respective training sample in the second plurality of training samples, corresponding information about the respective training sample into the multi-task model, where the information about the respective training sample includes the corresponding representation of the corresponding plurality of abundance values in the second latent feature space. The method also includes adjusting the plurality of parameters based on (A) for each respective training sample in the first plurality of training samples, one or more differences between (i) the corresponding plurality of outputs and (ii) a set of labels including (a) the corresponding experimentally measured effect of the candidate pharmaceutical agent on the respective training sample and (b) the corresponding cell type classification for each respective cell type variable in the set of one or more cell type variables, and (B) for each respective training sample in the second plurality of training samples, one or more differences between (i) the corresponding plurality of outputs and (ii) a set of labels including the corresponding cell type classification for each respective cell type variable in the set of one or more cell type variables.

### BRIEF DESCRIPTION OF THE FIGURES

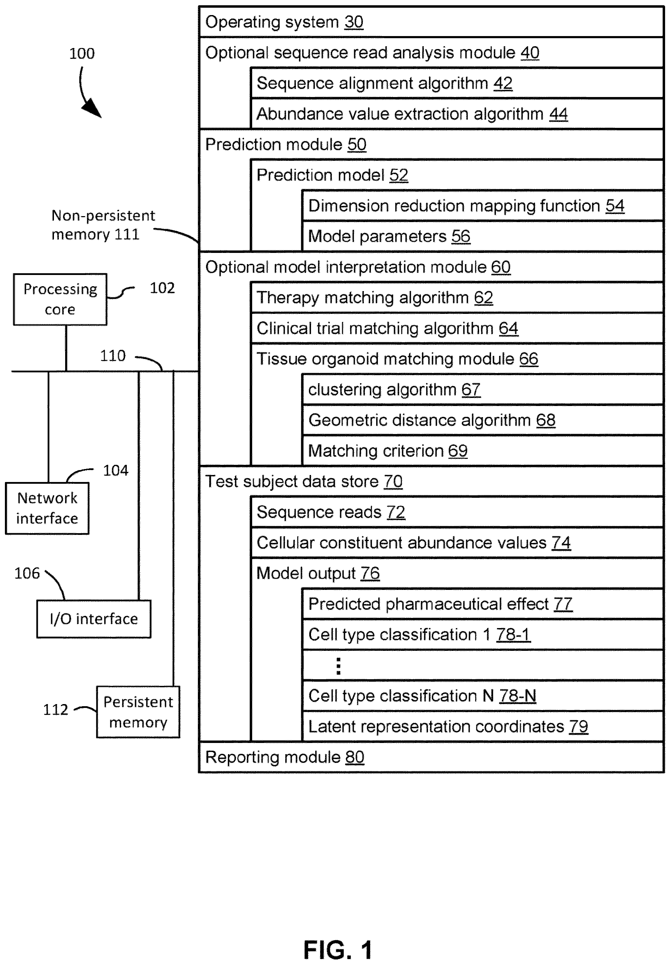

Figure 1 illustrates a block diagram of an example of a computing device for predicting an effect of a pharmaceutical agent in a test subject and/or for identifying one or more tissue organoids in a plurality of tissue organoids matching a biological property of a tissue in a subject, in accordance with some embodiments of the present disclosure.

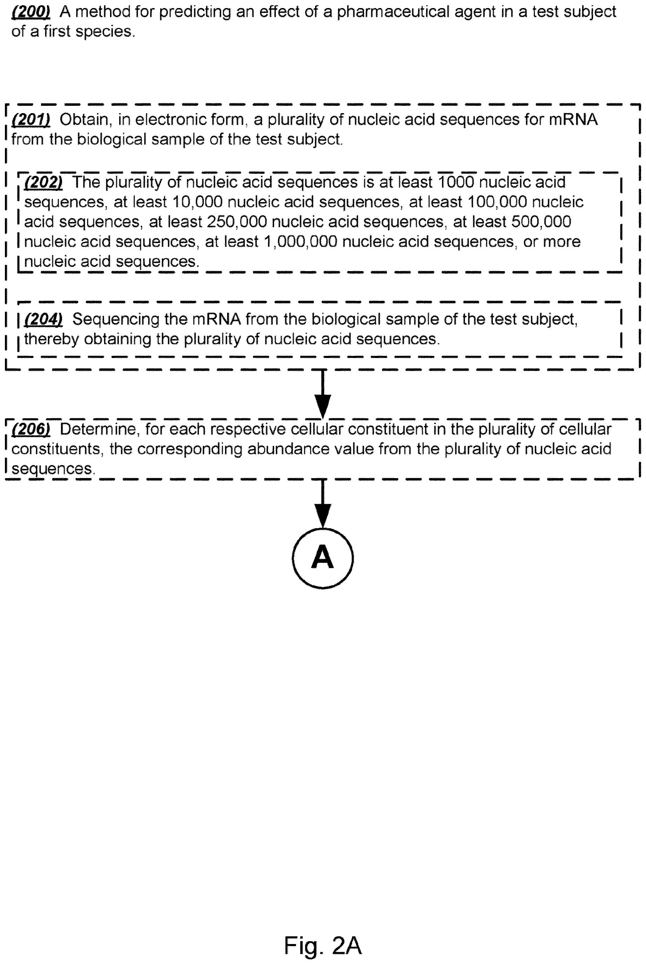

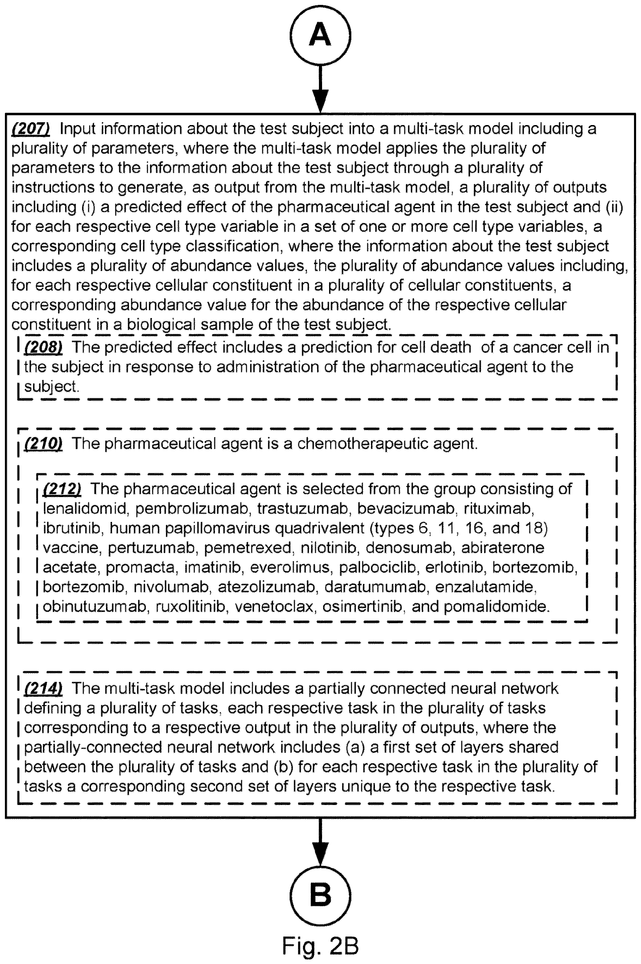

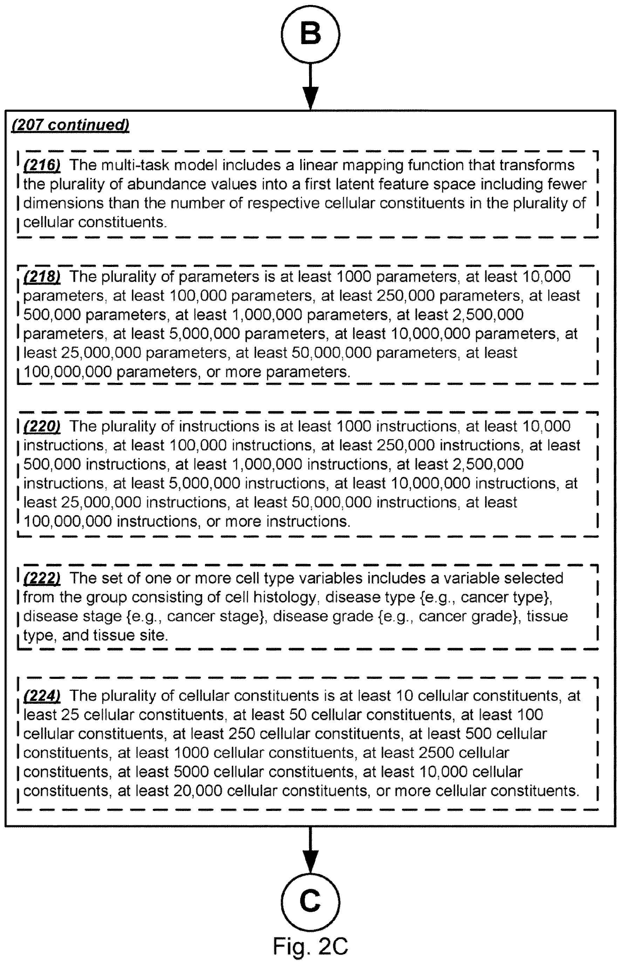

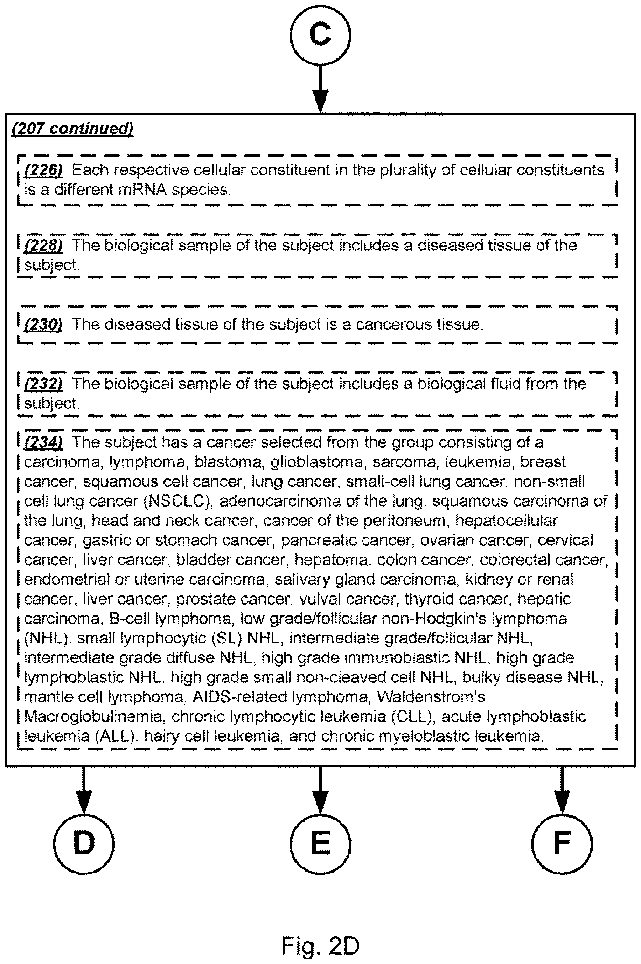

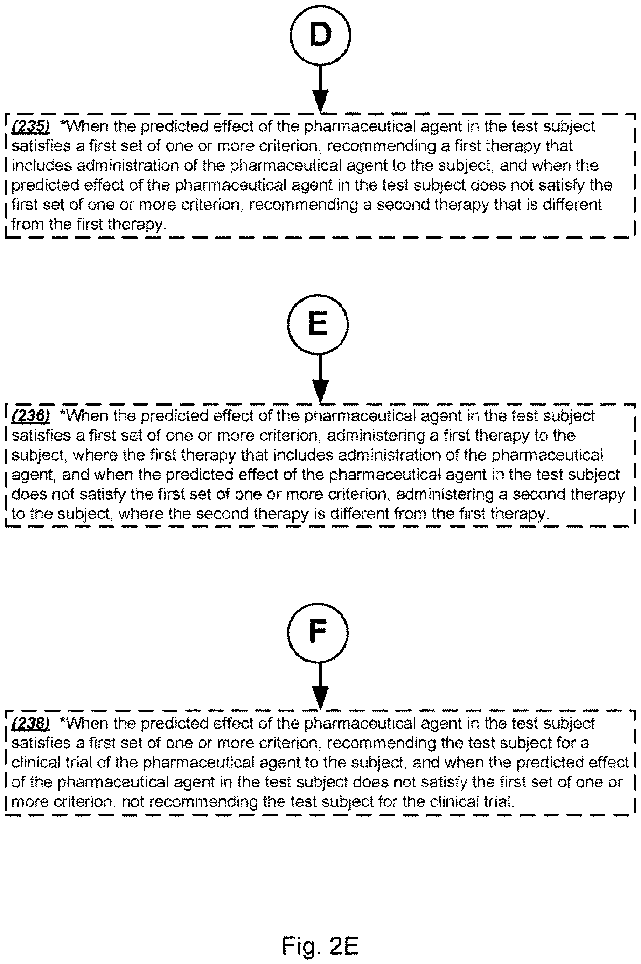

Figures 2A, 2B, 2C, 2D, and 2E collectively provide a flow chart of processes and features for predicting an effect of a pharmaceutical agent in a test subject, in which dashed boxes represent optional portions of the method, in accordance with some embodiments of the present disclosure.

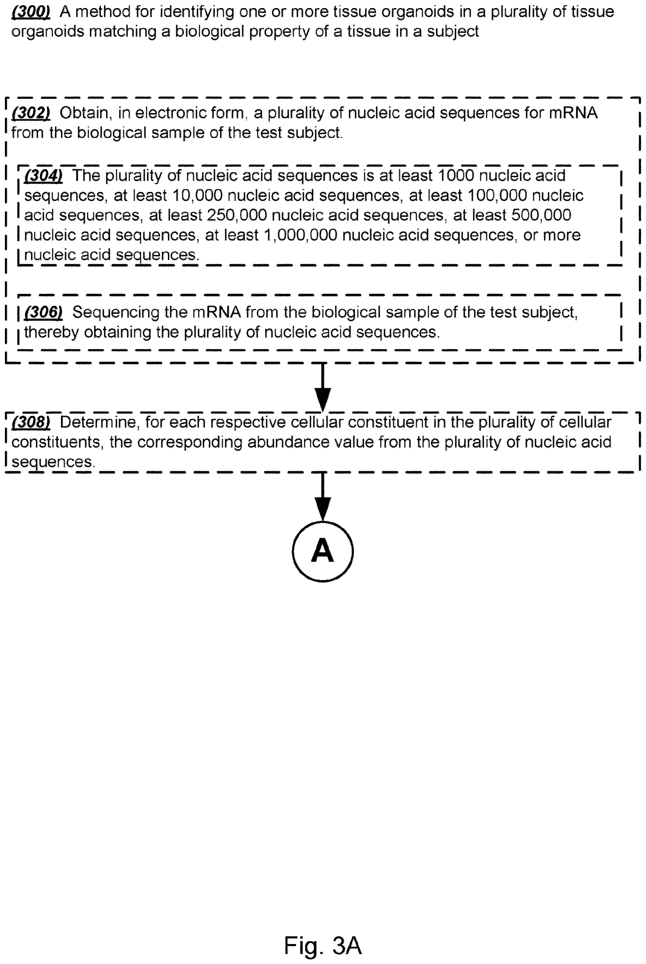

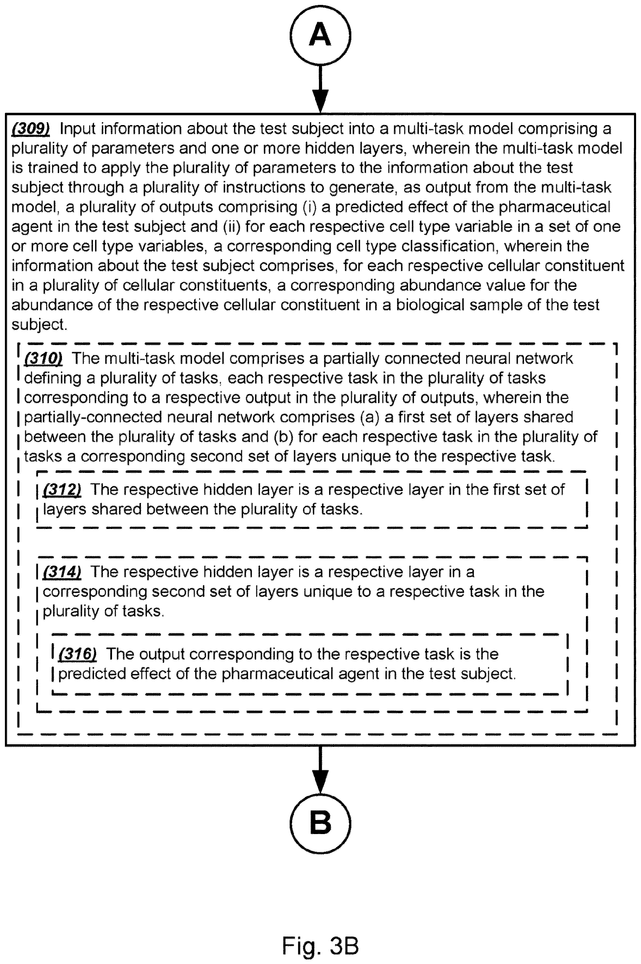

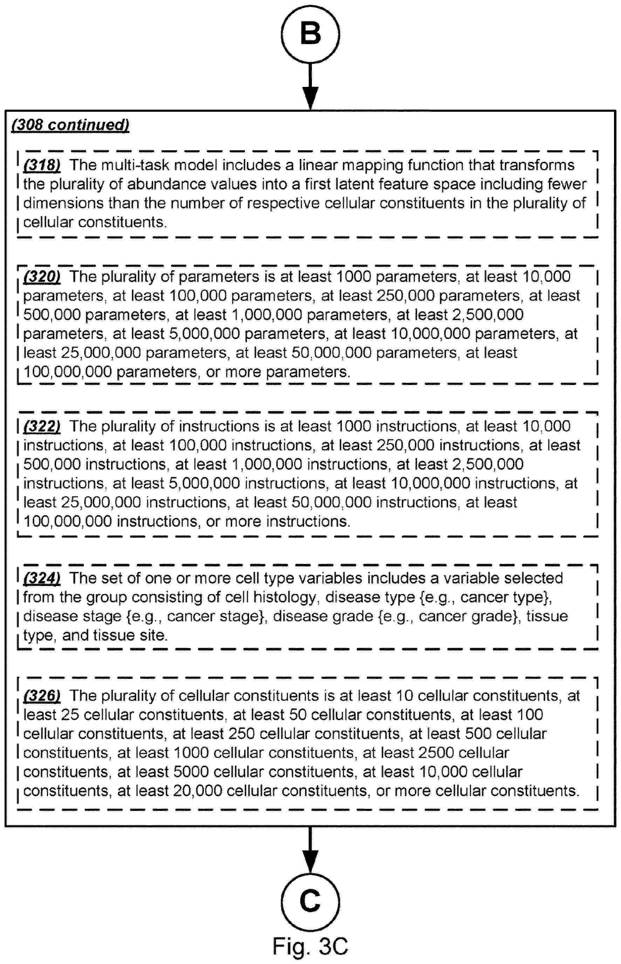

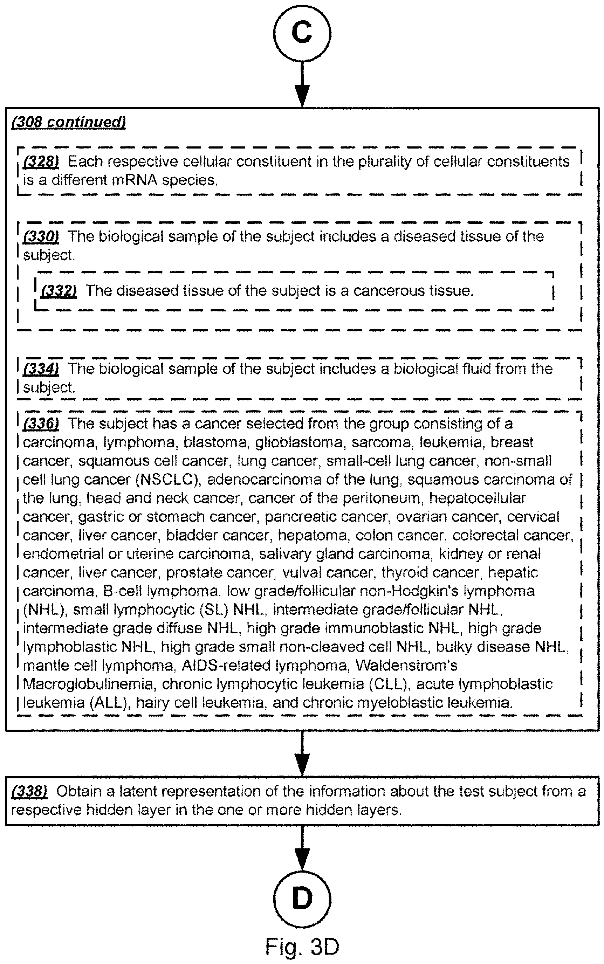

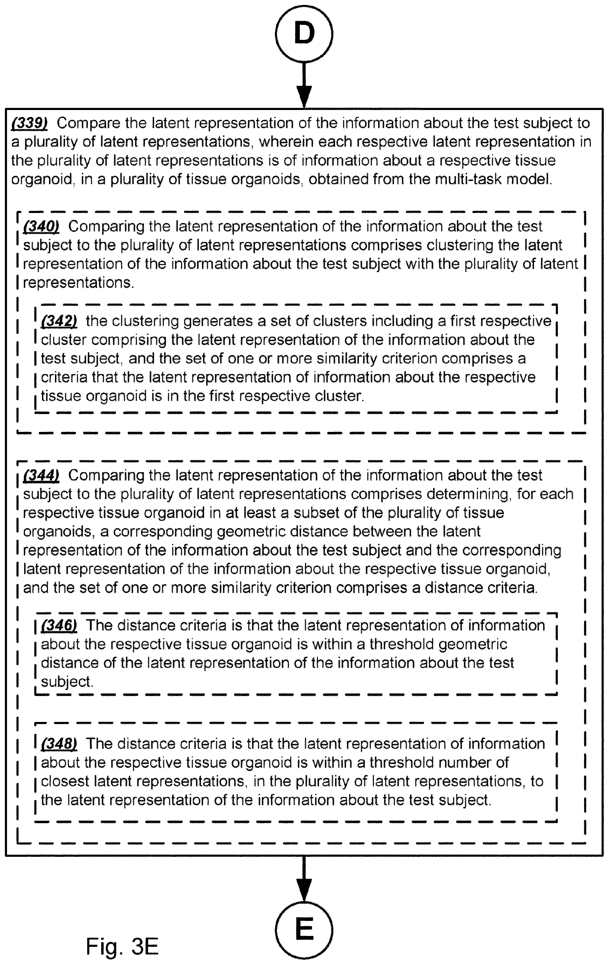

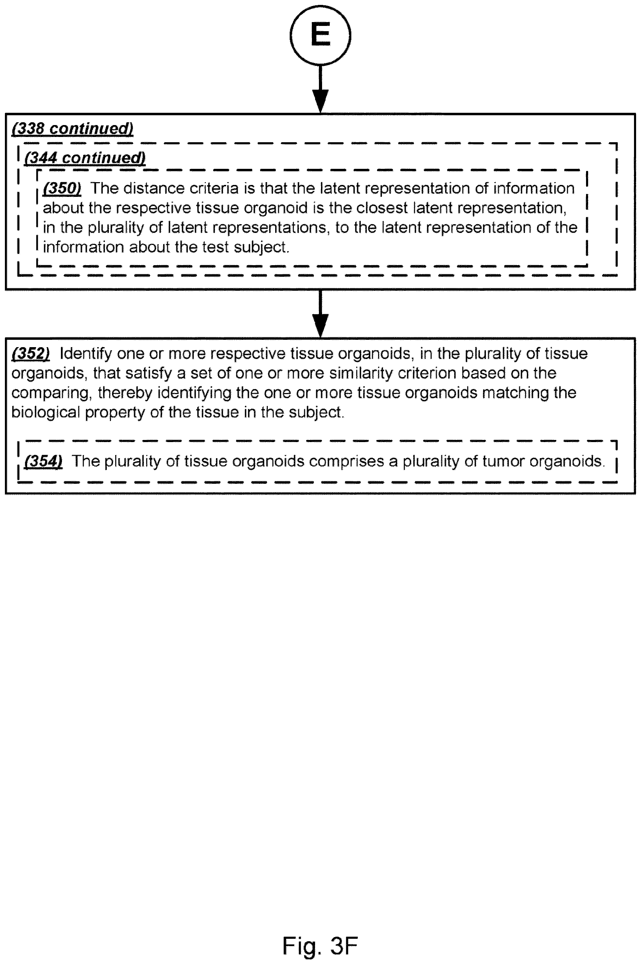

Figures 3A, 3B, 3C, 3D, 3E, and 3F collectively provide a flow chart of processes and features for identifying one or more tissue organoids in a plurality of tissue organoids matching a biological property of a tissue in a subject, in which dashed boxes represent optional portions of the method, in accordance with some embodiments of the present disclosure.

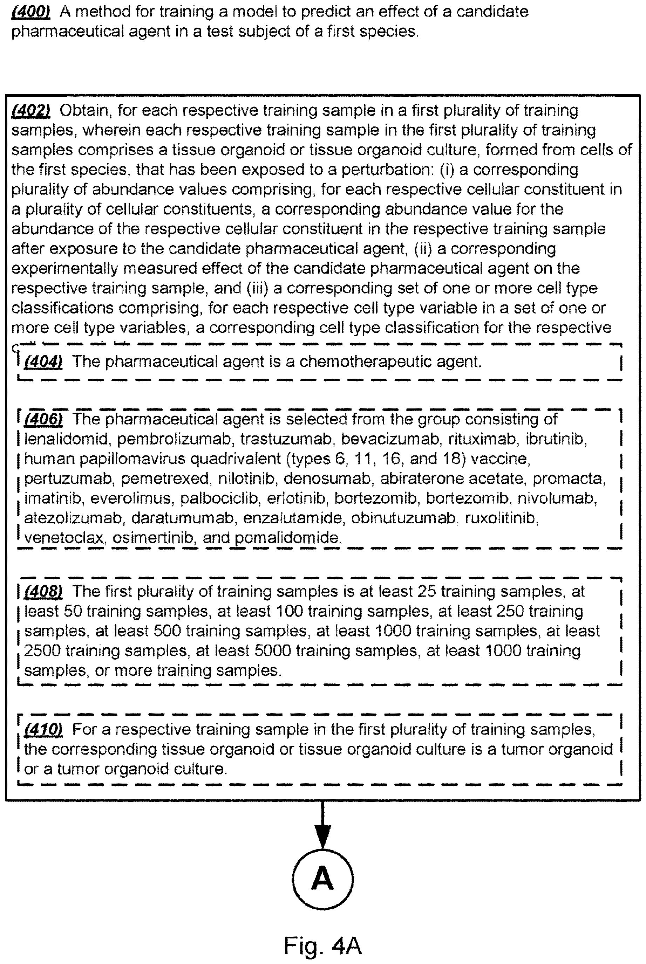

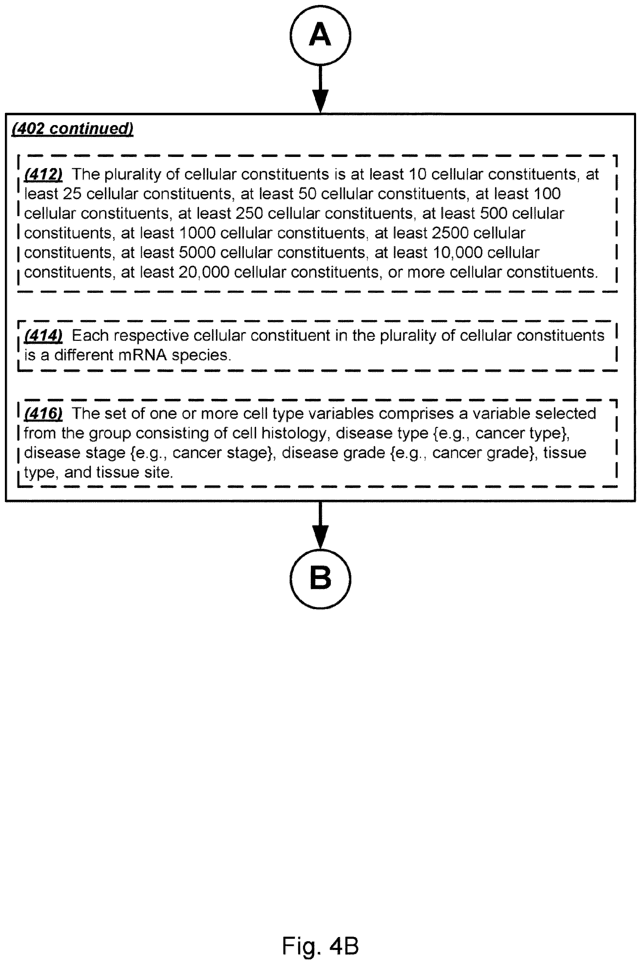

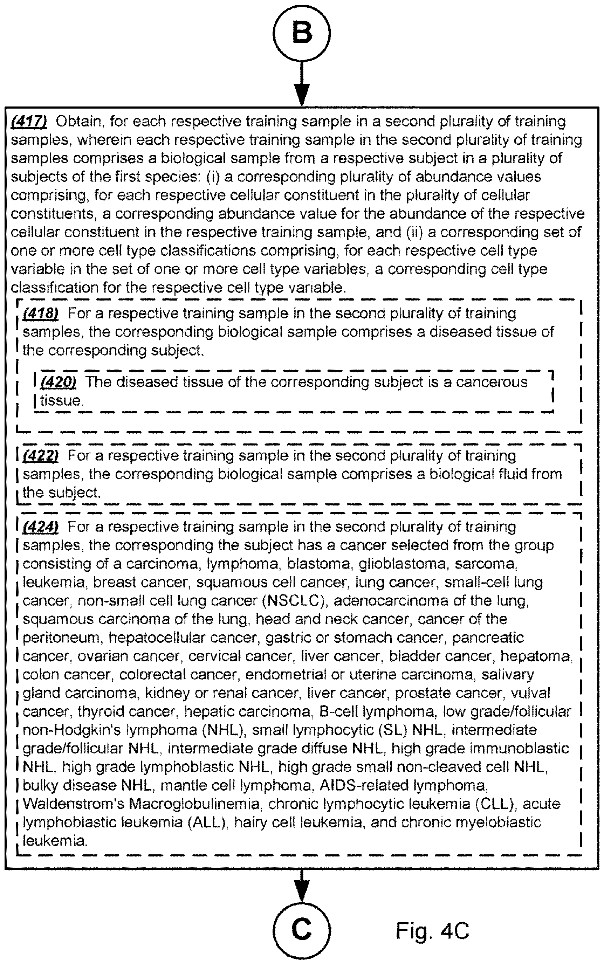

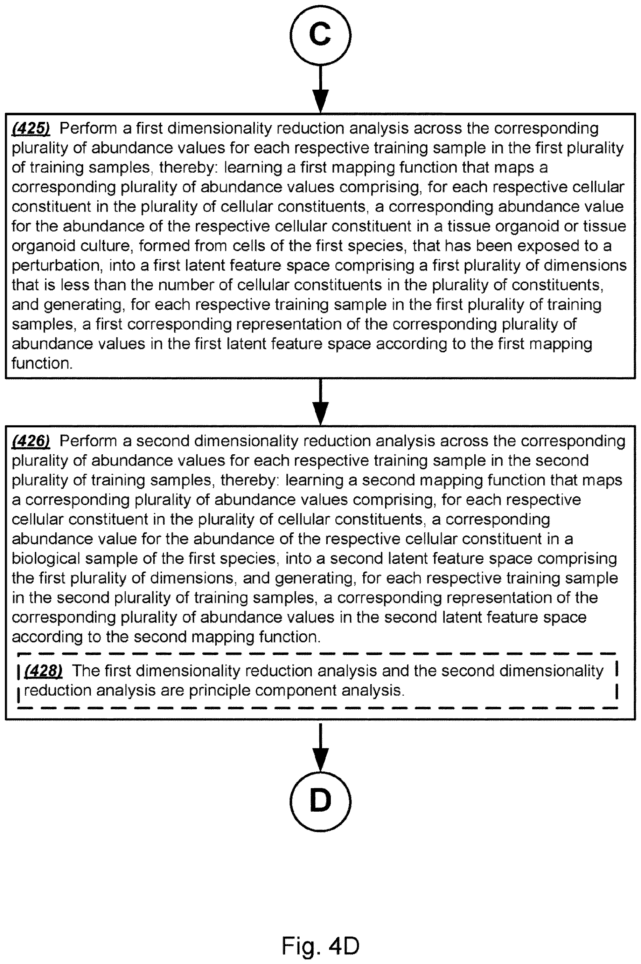

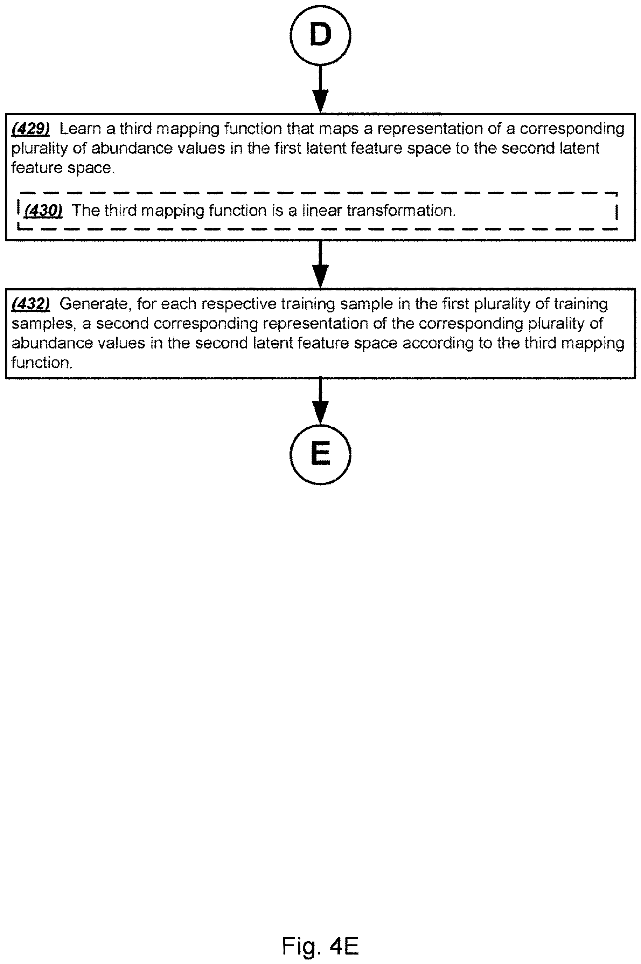

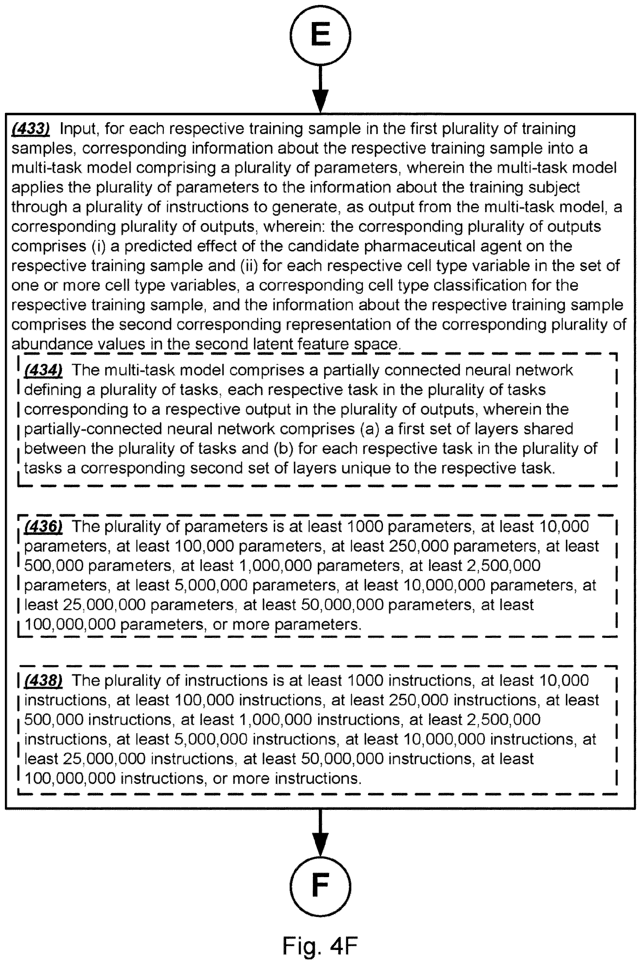

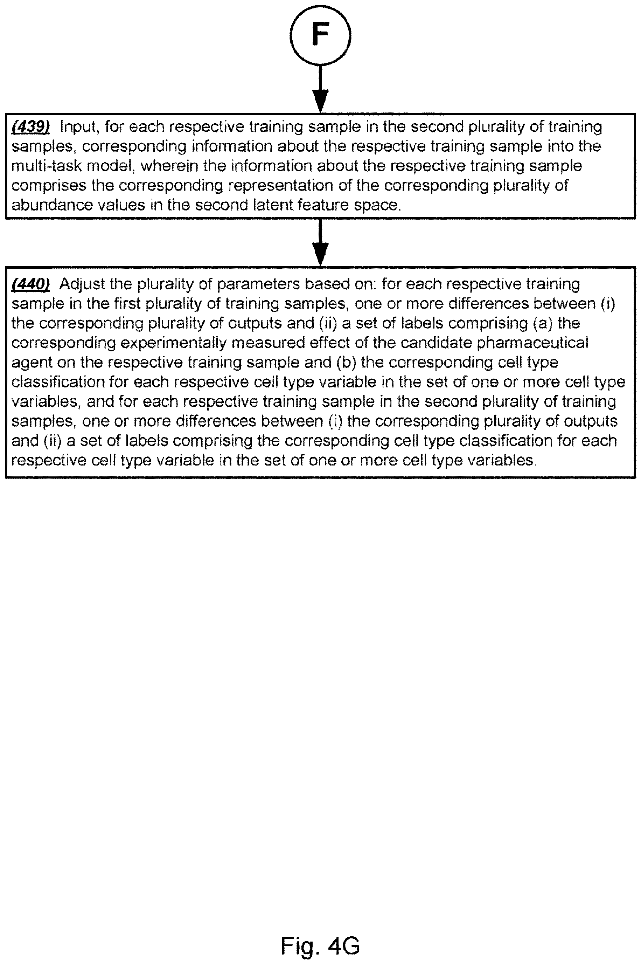

Figures 4A, 4B, 4C, 4D, 4E, 4F, and 4G collectively provide a flow chart of processes and features for training a model to predict an effect of a candidate pharmaceutical agent in a test subject, in which dashed boxes represent optional portions of the method, in accordance with some embodiments of the present disclosure.

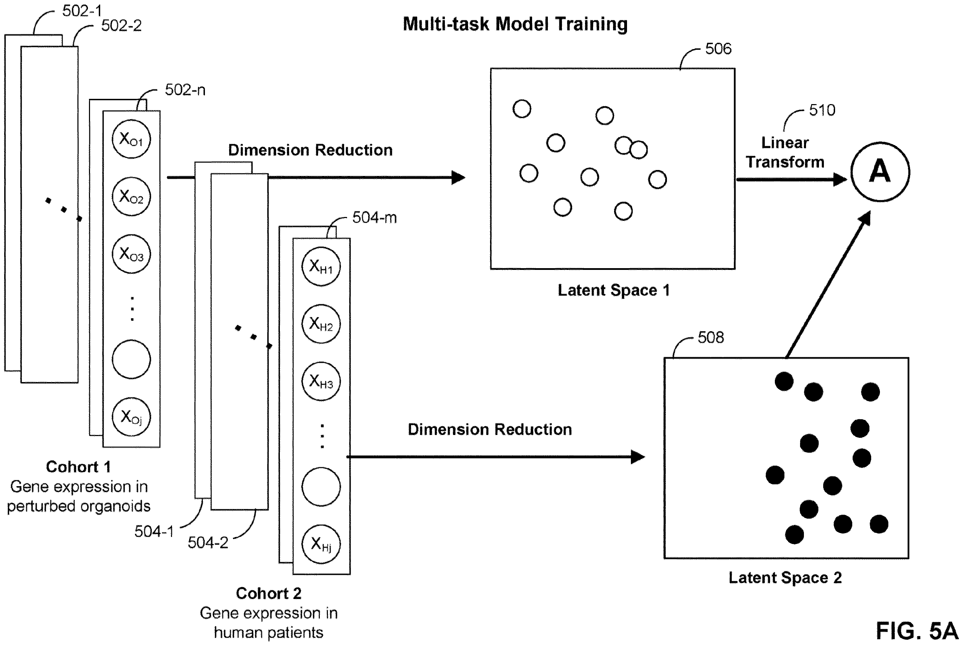

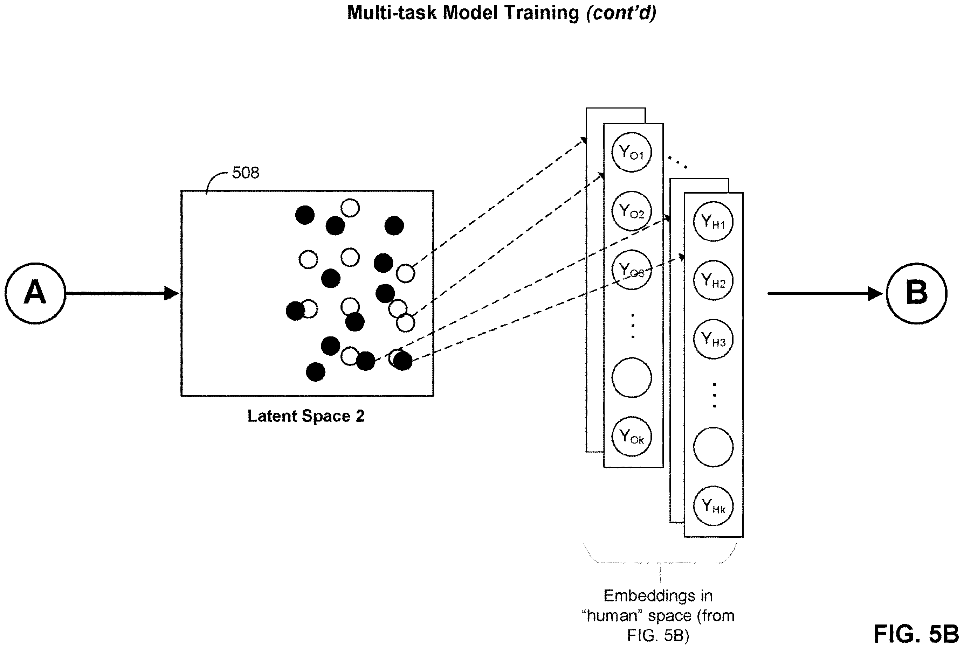

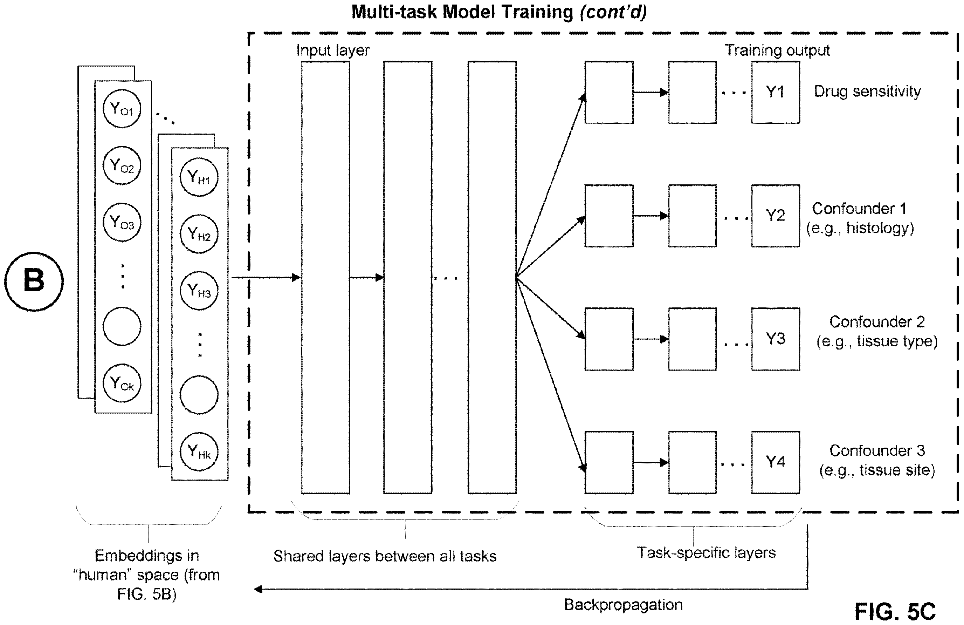

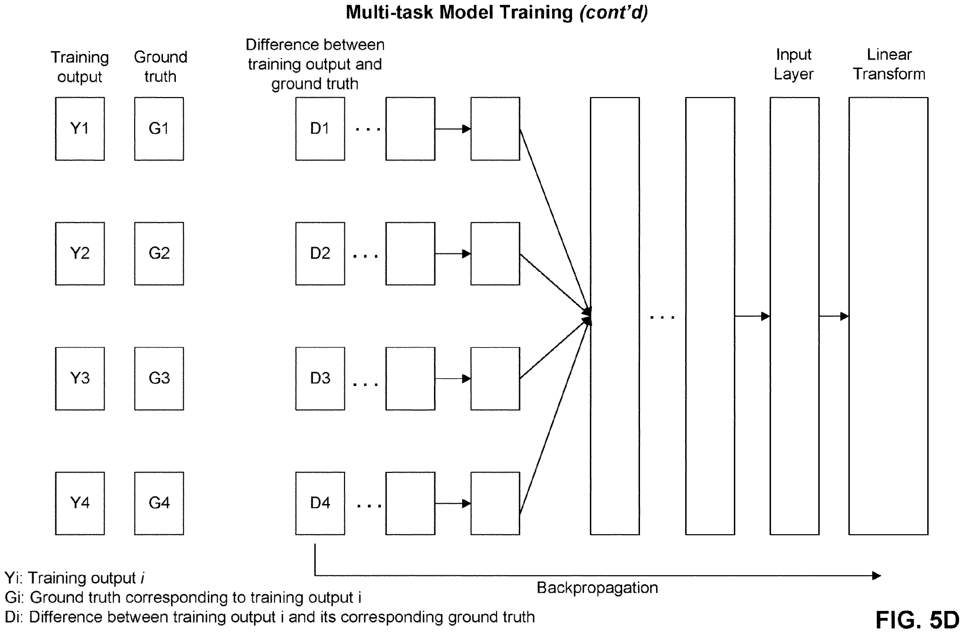

Figures 5A, 5B, 5C, and 5D collectively illustrate processes and features for training a model to predict an effect of a candidate pharmaceutical agent in a test subject, in accordance with some embodiments of the present disclosure.

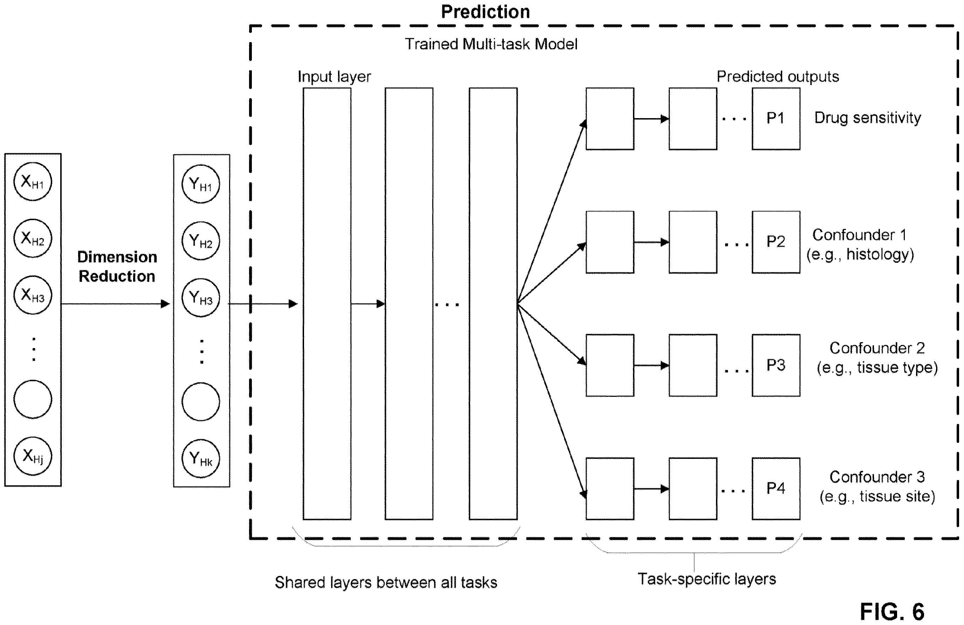

Figure 6 illustrates processes and features for predicting an effect of a pharmaceutical agent in a test subject, in accordance with some embodiments of the present disclosure.

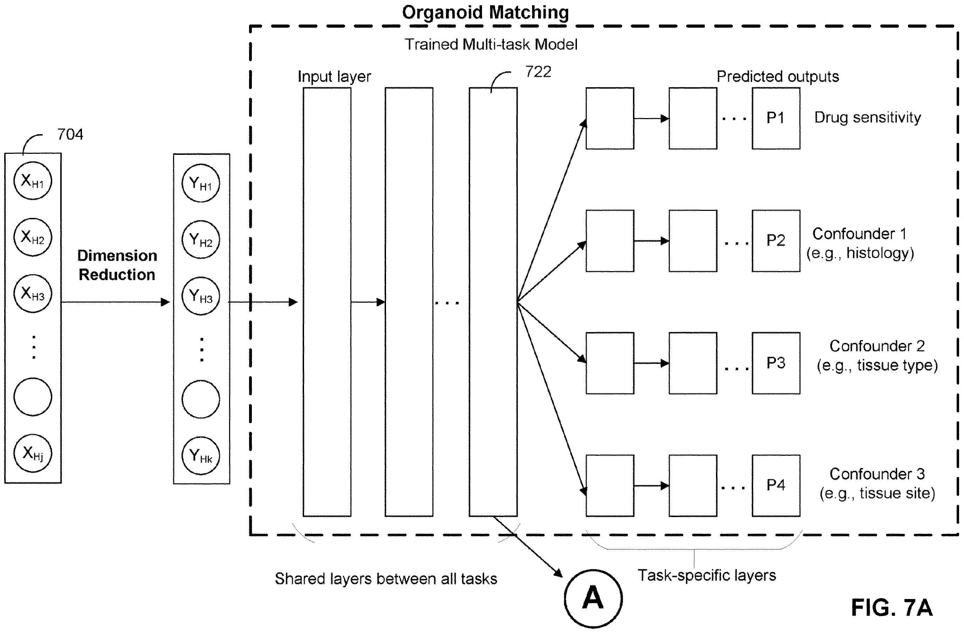

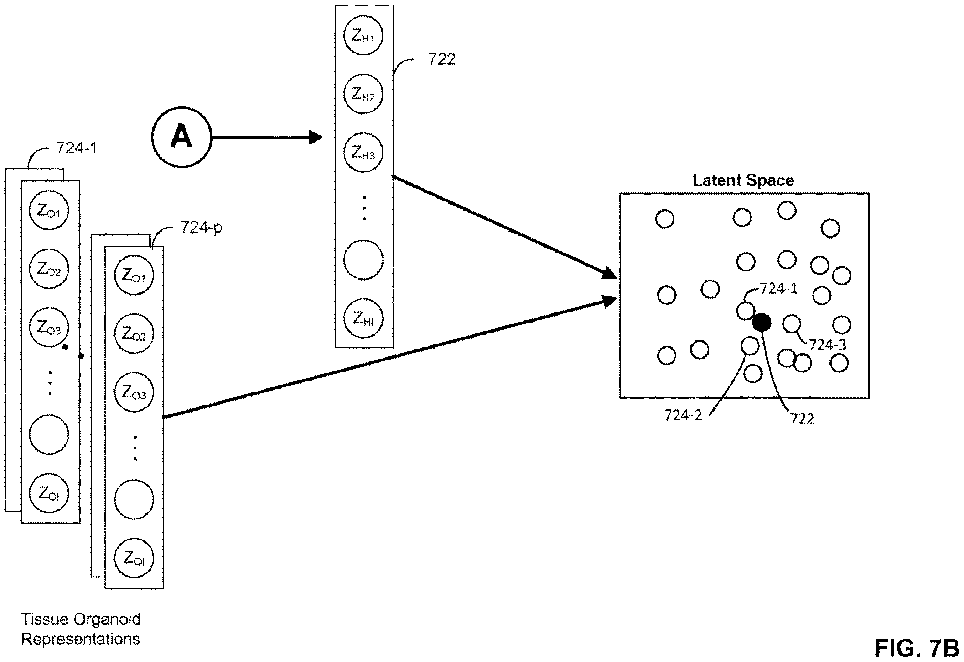

Figures 7A and 7B collectively illustrate processes and features for identifying one or more tissue organoids in a plurality of tissue organoids matching a biological property of a tissue in a subject, in accordance with some embodiments of the present disclosure.

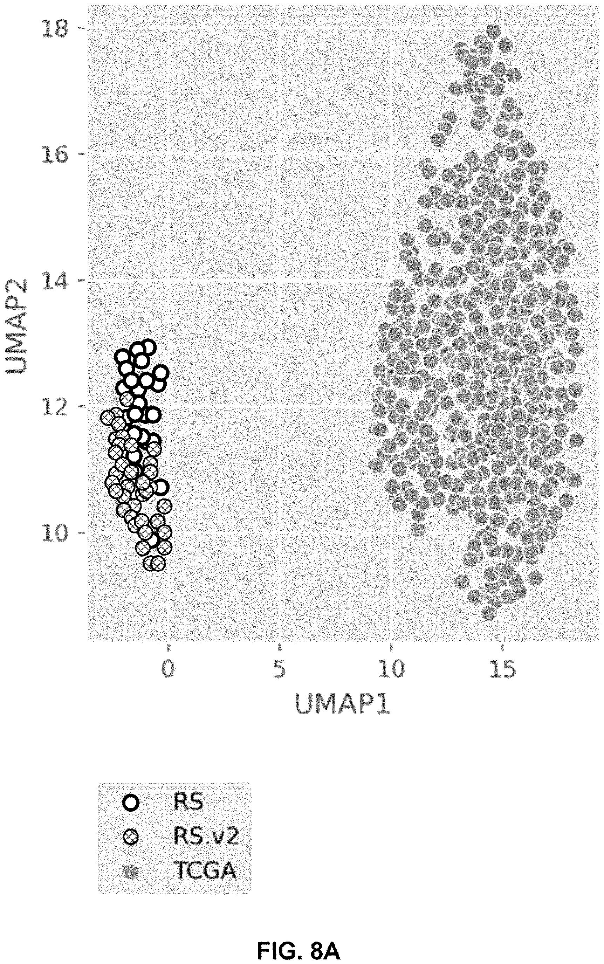

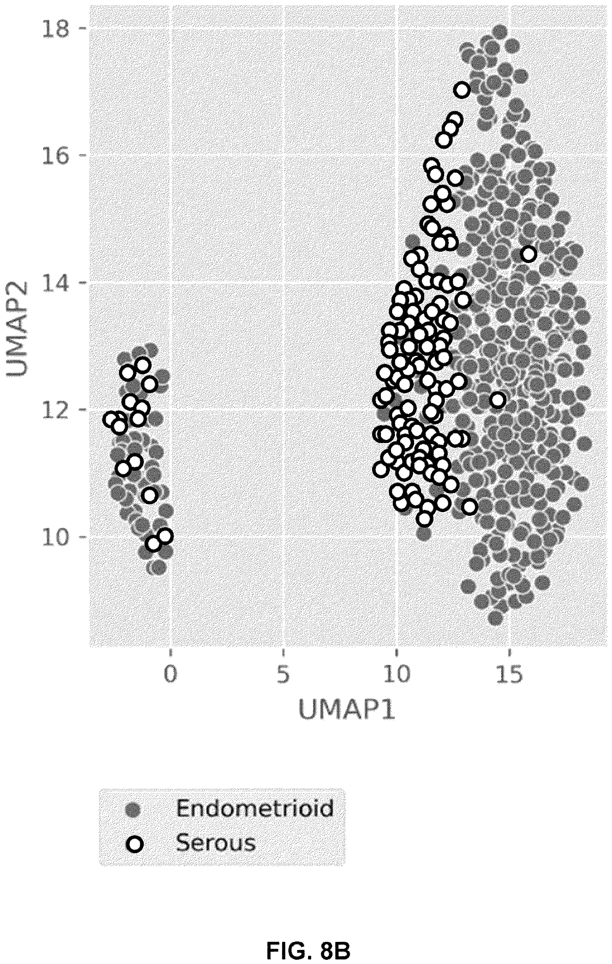

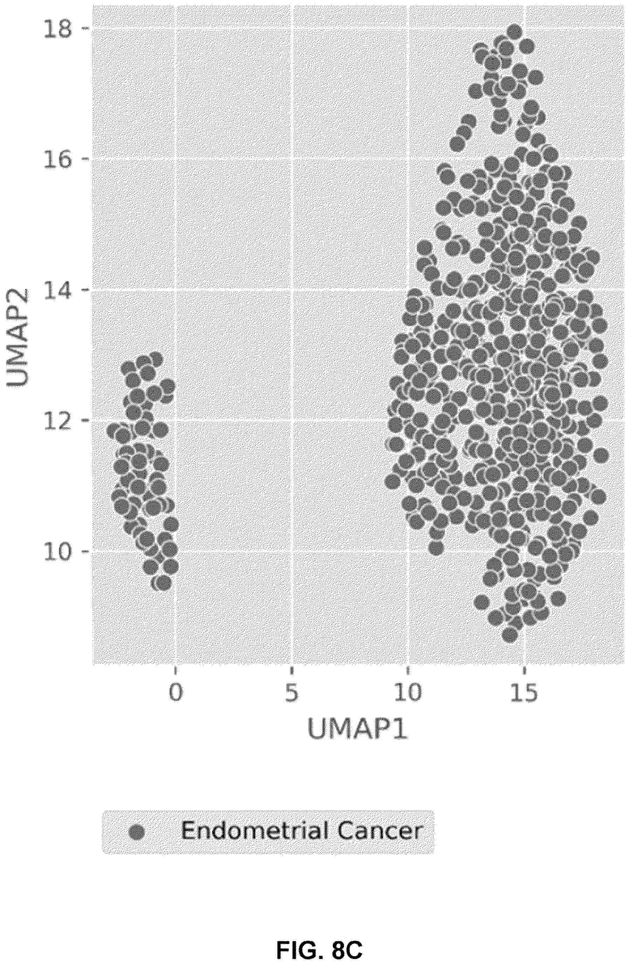

Figures 8A, 8B, and 8C collectively illustrate the results of Uniform Manifold Approximation and Projection (UMAP) dimensionality reduction analysis of gene expression profiles for tumor organoid models of endometrial cancer (RS and RS.v2) and primary human endometrial cancer tissues (TCGA), labeled according to the source of the gene expression profile (8A), tissue histology (8B), or cancer type (8C) as shown in the legend of each respective figure.

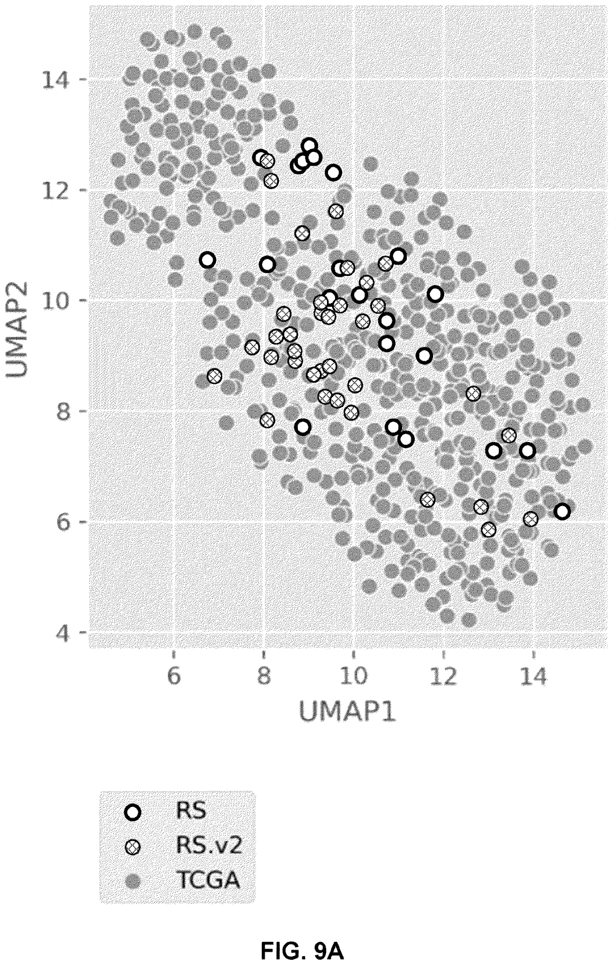

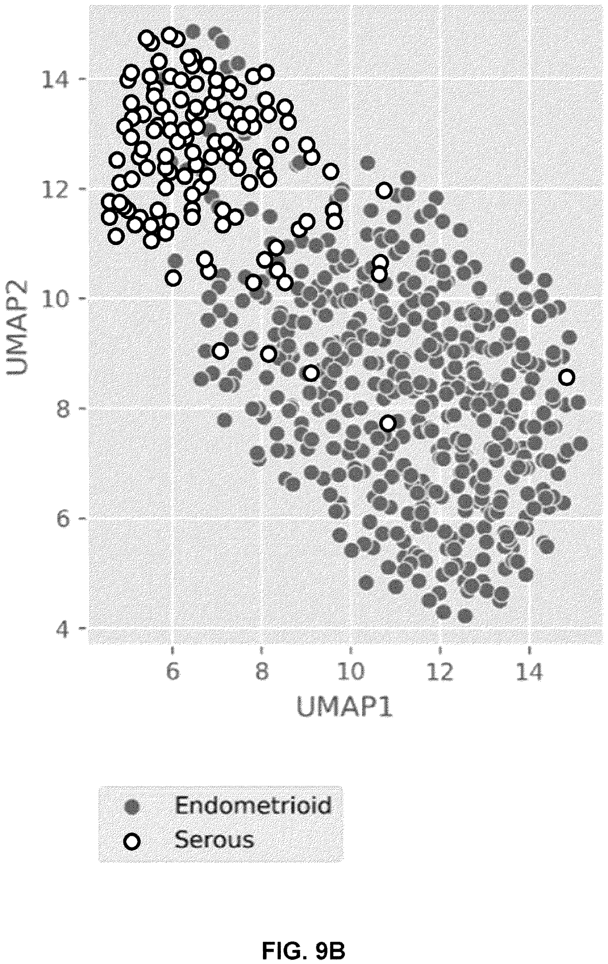

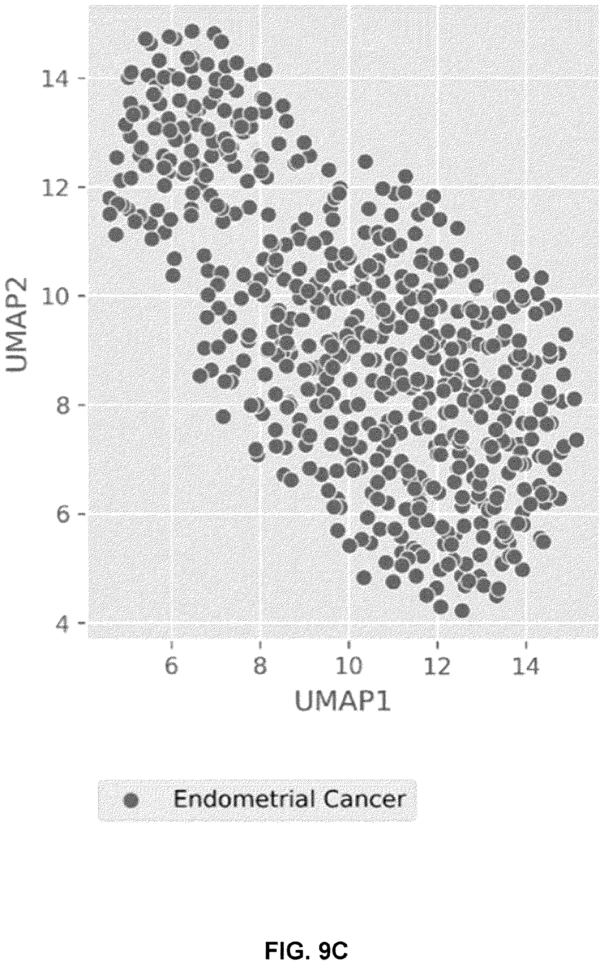

Figures 9A, 9B, and 9C collectively illustrate the results of Uniform Manifold Approximation and Projection (UMAP) dimensionality reduction analysis of latent representations, from a multi-task model according to some embodiments of the present disclosure, of the gene expression profiles for tumor organoid models of endometrial cancer (RS and RS.v2) and primary human endometrial cancer tissues (TCGA), labeled according to the source of the gene expression profile (9A), tissue histology (9B), or cancer type (9C) as shown in the legend of each respective figure.

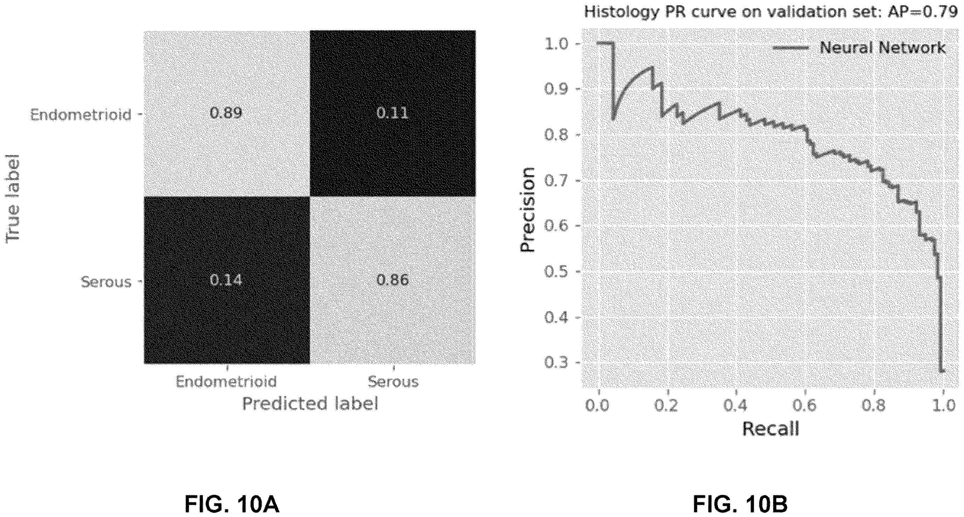

Figures 10A and 10B illustrate a confusion matrix (10A) and a precision-recall curve (10B) for performance of a fully connected neural network trained to distinguish between endometroid and serous histologies of endometrial cancer tissue using gene expression profiles.

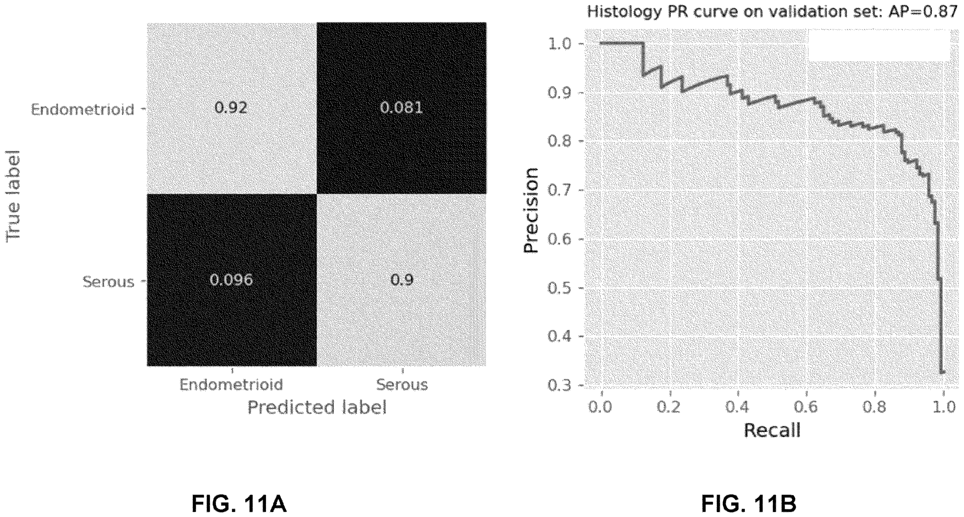

Figures 11A and 11B illustrate a confusion matrix (11A) and a precision-recall curve (11B) for performance of a multi-task model, according to some embodiments of the present disclosure, with respect to distinguishing between endometroid and serous histologies of endometrial cancer tissue using gene expression profiles.

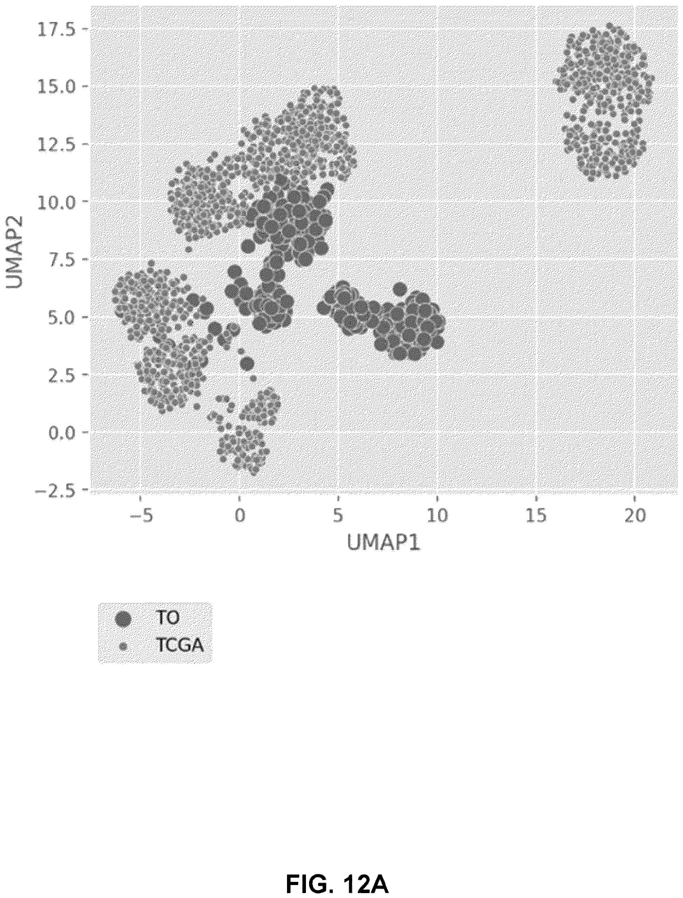

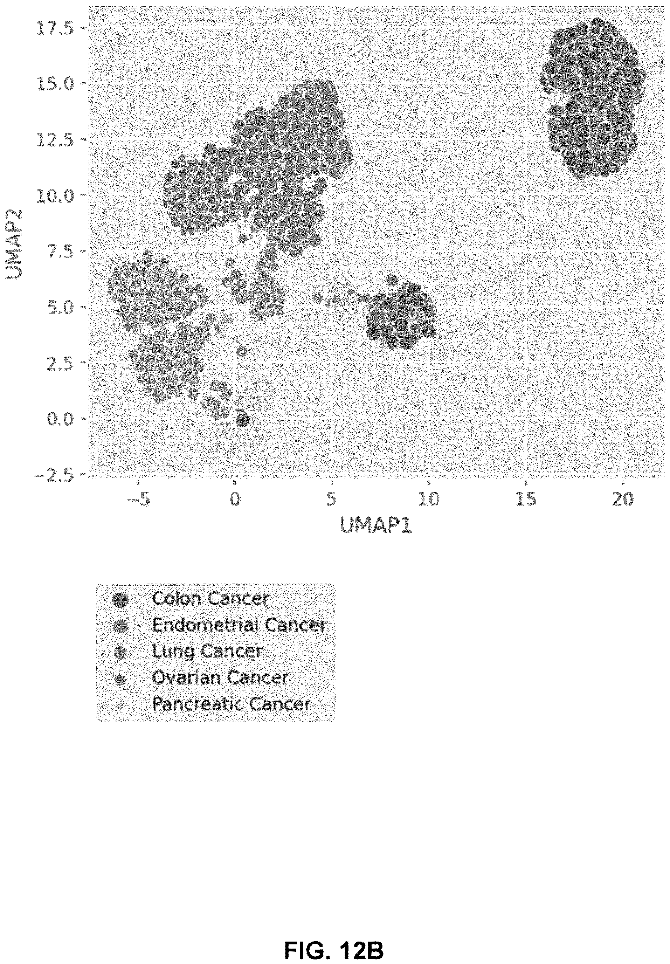

Figures 12A and 12B collectively illustrate the results of Uniform Manifold Approximation and Projection (UMAP) dimensionality reduction analysis of gene expression profiles for tumor organoids (TO) and primary human cancer tissues (TCGA), labeled according to the source of the gene expression profile (12A) or cancer type (12B) as shown in the legend of each respective figure.

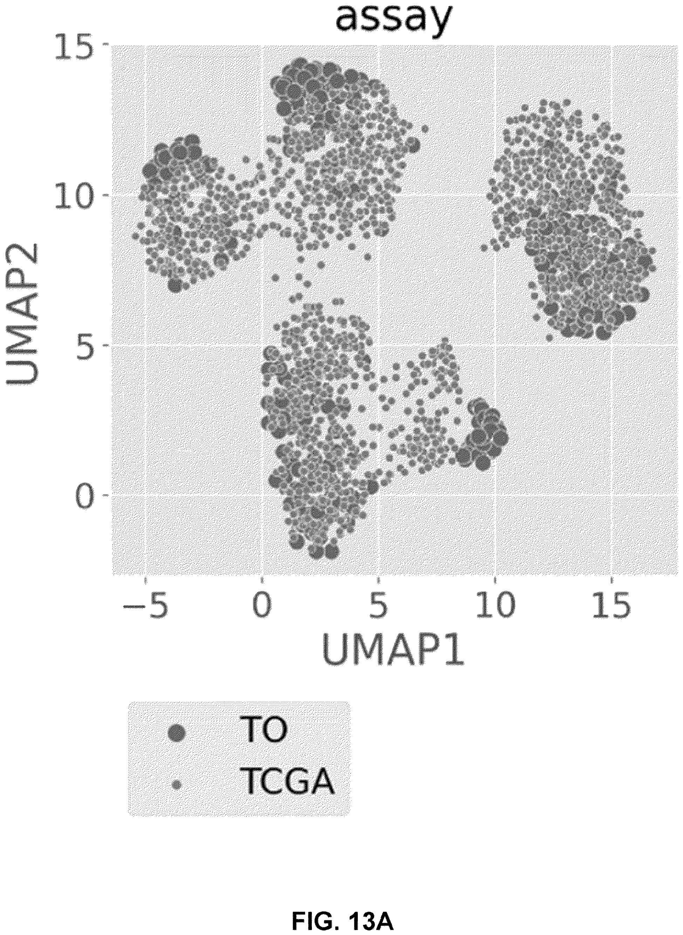

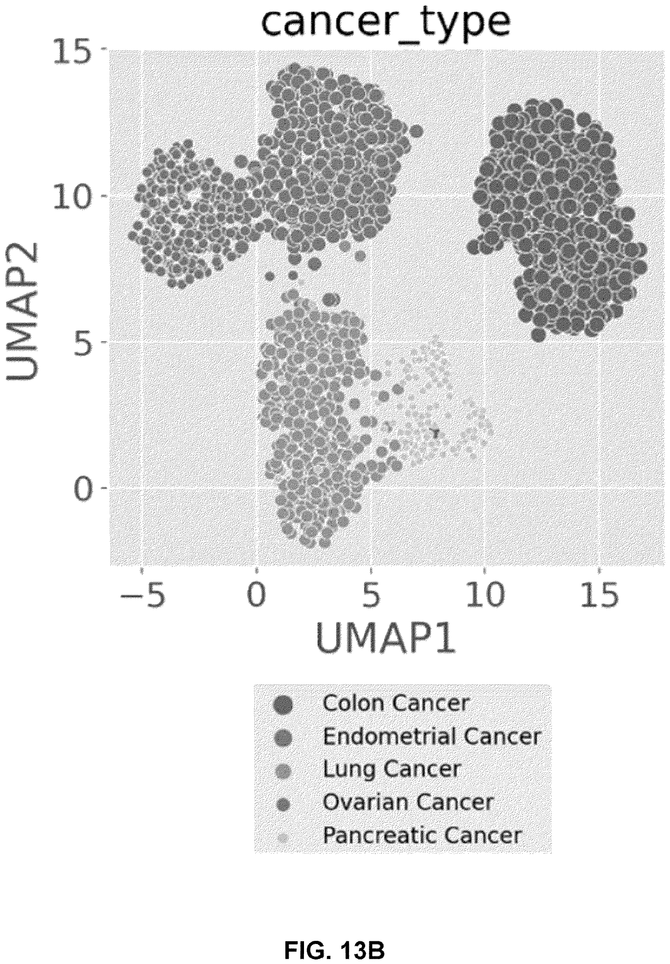

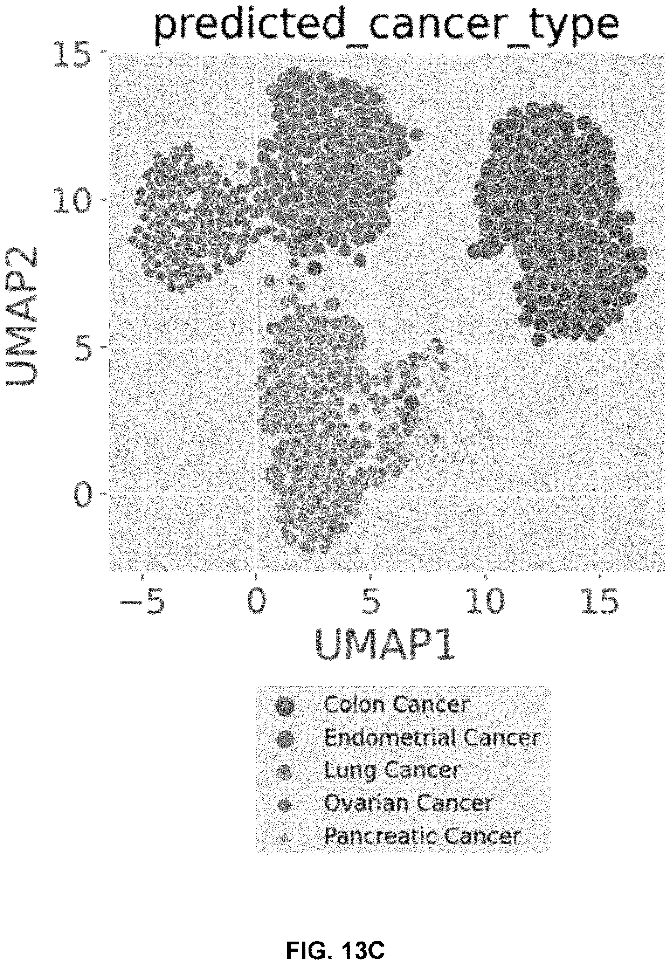

Figures 13A, 13B, and 13C collectively illustrate the results of Uniform Manifold Approximation and Projection (UMAP) dimensionality reduction analysis of latent representations, from the first layer of a multi-task model according to some embodiments of the present disclosure, of the gene expression profiles for tumor organoids (TO) and primary human cancer tissues (TCGA), labeled according to the source of the gene expression profile (13A), cancer type (13B), or cancer type predicted by the multi-task model (13C) as shown in the legend of each respective figure.

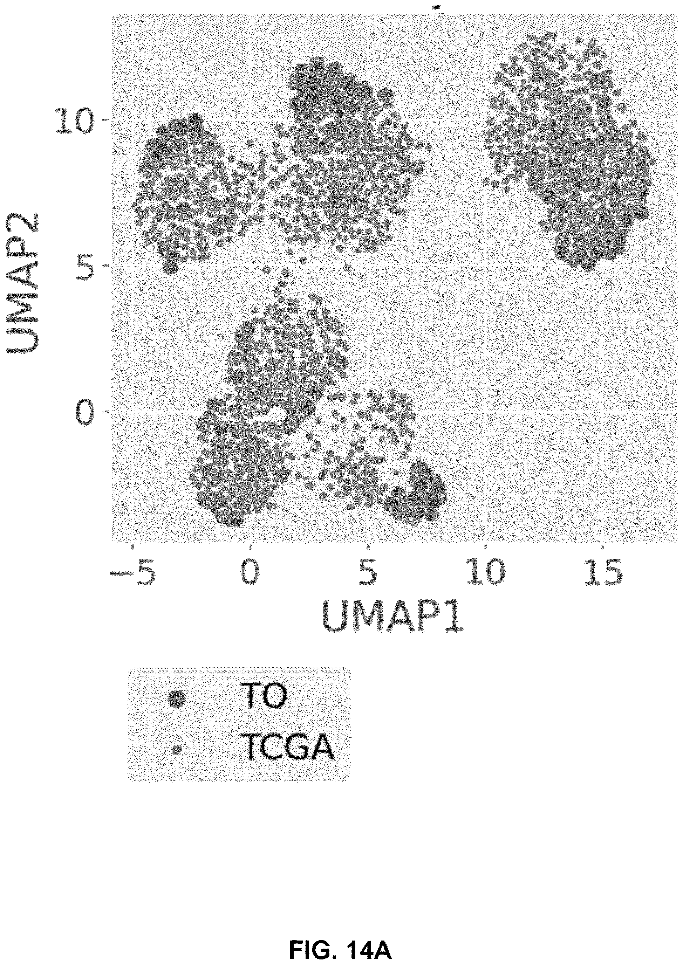

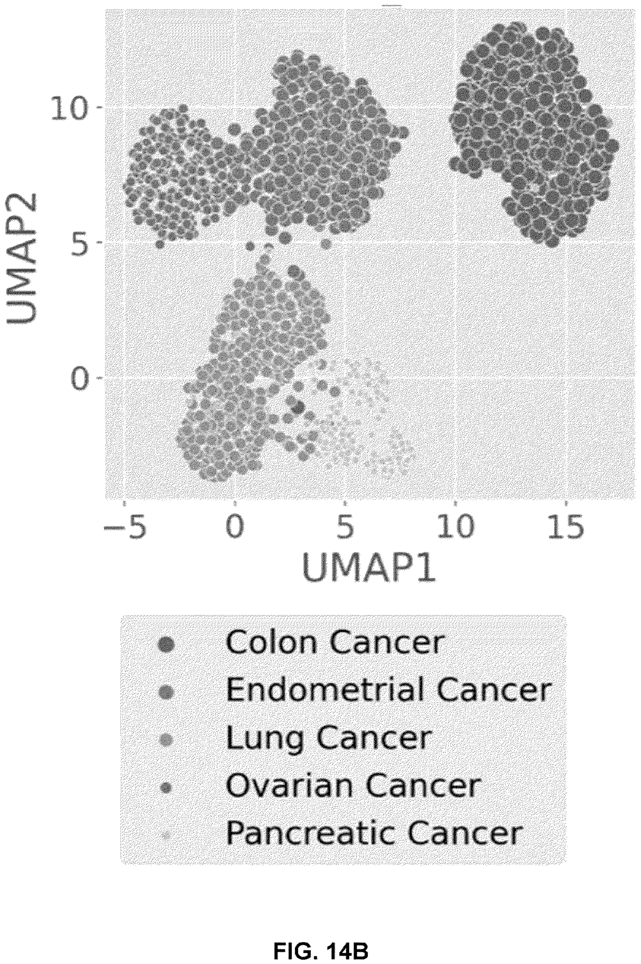

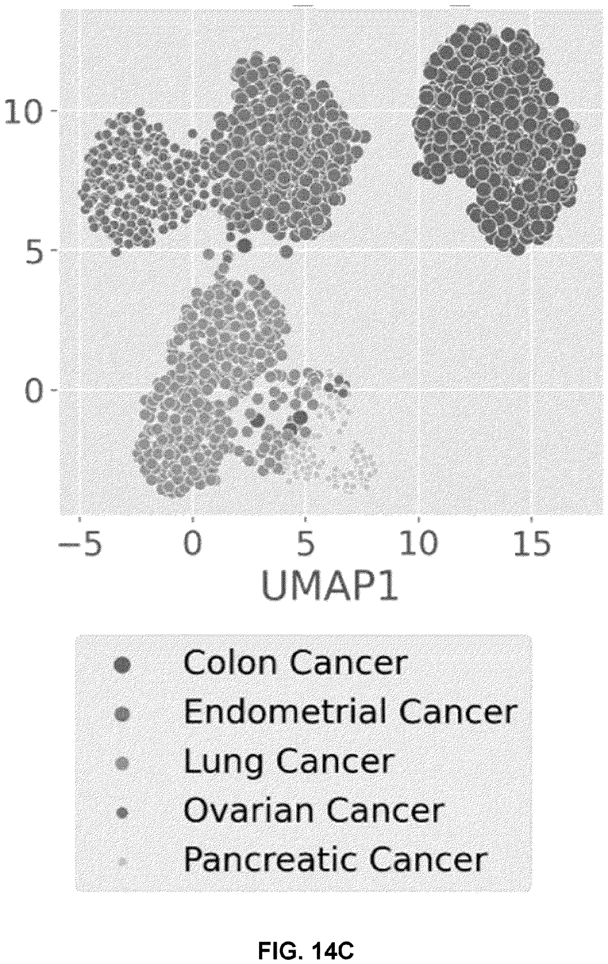

Figures 14A, 14B, and 14C collectively illustrate the results of Uniform Manifold Approximation and Projection (UMAP) dimensionality reduction analysis of latent representations, from the second layer of a multi-task model according to some embodiments of the present disclosure, of the gene expression profiles for tumor organoids (TO) and primary human cancer tissues (TCGA), labeled according to the source of the gene expression profile (14A), cancer type (14B), or cancer type predicted by the multi-task model (14C) as shown in the legend of each respective figure.

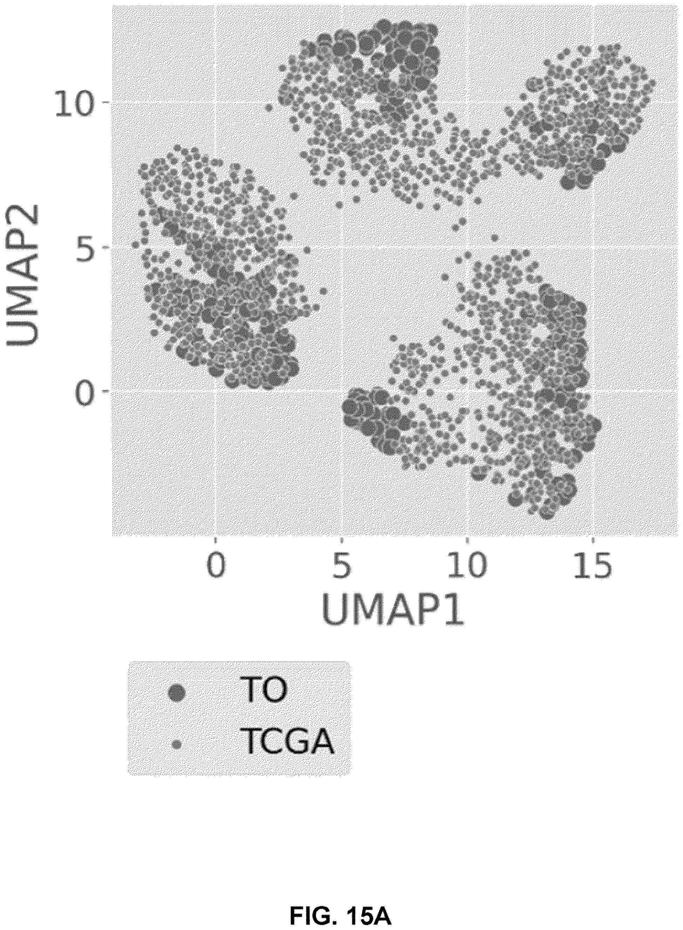

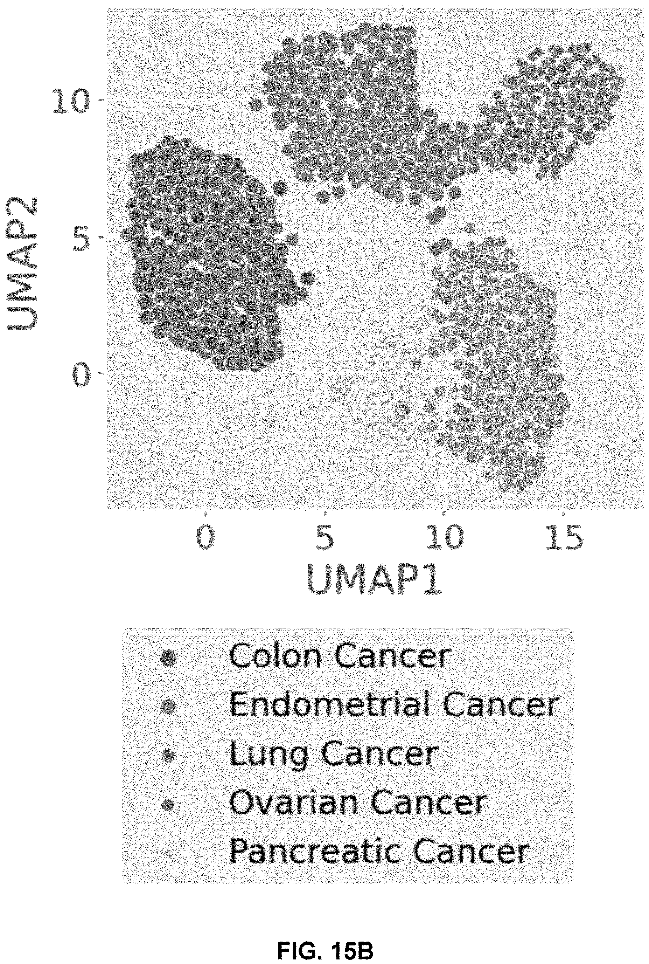

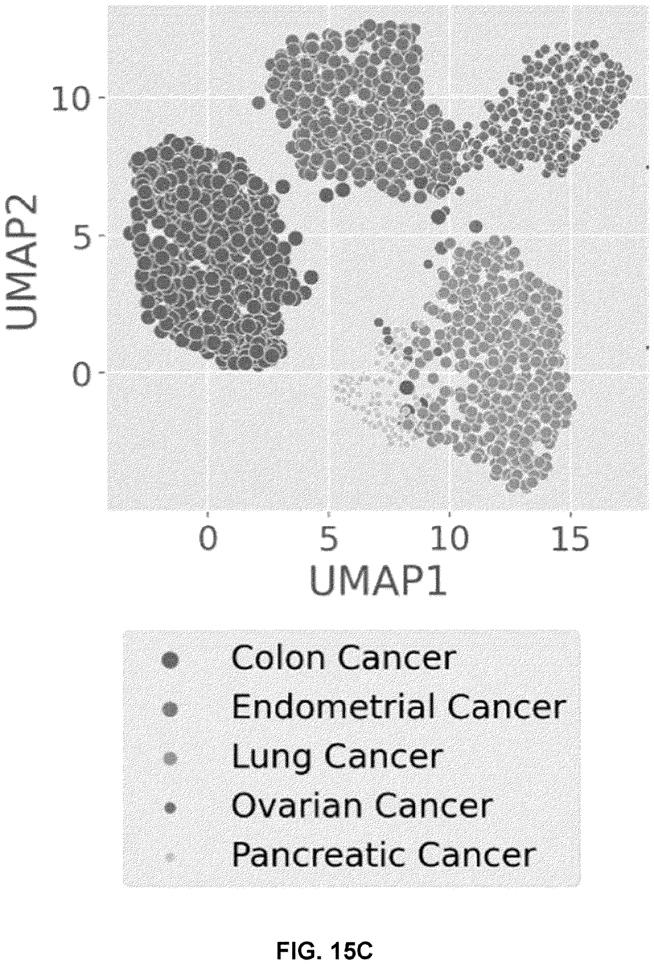

Figures 15A, 15B, and 15C collectively illustrate the results of Uniform Manifold Approximation and Projection (UMAP) dimensionality reduction analysis of latent representations, from the third layer of a multi-task model according to some embodiments of the present disclosure, of the gene expression profiles for tumor organoids (TO) and primary human cancer tissues (TCGA), labeled according to the source of the gene expression profile (15A), cancer type (15B), or cancer type predicted by the multi-task model (15C) as shown in the legend of each respective figure.

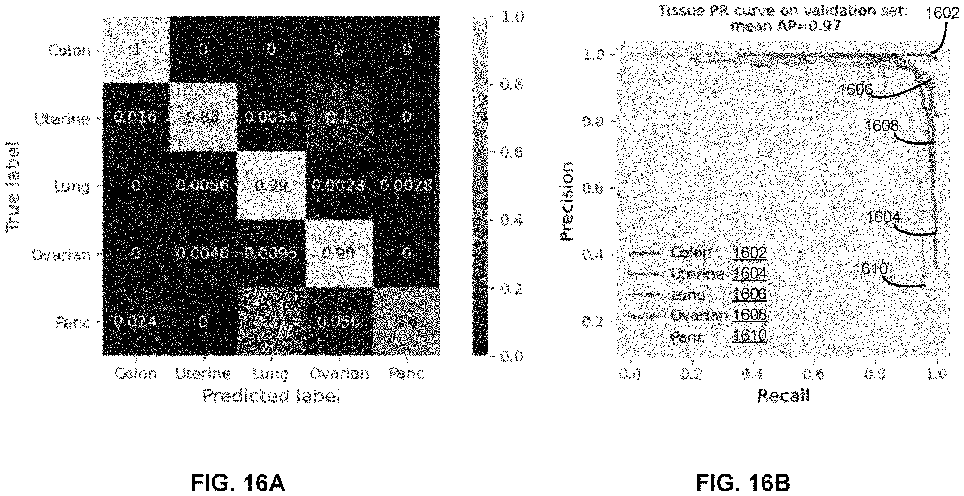

Figures 16A and 16B illustrate a confusion matrix (16A) and a precision-recall curve (16B) for performance of a multi-task model, according to some embodiments of the present disclosure, with respect to distinguishing between cancer types using gene expression profiles.

### DETAILED DESCRIPTION

In some embodiments, the present disclosure provides methods and system that predict human response to medications based on organoid screen data. Recently deep-learning models have been developed to exploit RNA expression data from cell lines to predict patient-specific responses to new drug therapies. Tumor organoids (TO) are increasingly essential in screening drugs for cancer therapy. Identifying patients with biomarkers for anticancer therapy sensitivity is crucial for improving the efficacy of a drug. A significant challenge in discovering biomarkers from organoids is accounting for confounders such as tissue type, tissue sites, stage, and histology. Another challenge is the presence of technical biases between tumor organoids and human expression data. However, models have yet to be developed that utilize organoid screens as well as account for the aforementioned confounders when predicting human clinical response.

Advantageously, the methods and systems described herein (i) correct for technical biases between organoid and human RNA-expression datasets, (ii) exploit organoid-specific-drug responses to predict drug response on human expression data, and (iii) account for confounders within the organoid and human samples. In some embodiments, the confounders are accounted for by modeling confounder variables (e.g., histology, stage, grade, etc.) as tasks similar to the task of predicting drug response. As such, in some embodiments, a multi-task model is provided that not only addresses the confounders and technical biases across organoid and human samples but also learns a transferable drug-response mapping from organoids to patients. In some embodiments, the algorithm's output is a recommendation for patients that are similar to sensitive and resistant organoids. The algorithm allows for the integration of organoid and human molecular datasets into a shared database, increasing the power to detect drug-specific biomarkers.

In some embodiments, the methods and systems described herein.

### Definitions

The terminology used in the present disclosure is for the purpose of describing particular embodiments only and is not intended to be limiting of the invention. As used in the description of the invention and the appended claims, the singular forms "a," "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Furthermore, to the extent that the terms "including," "includes," "having," "has," "with," or variants thereof are used in either the detailed description and/or the claims, such terms are intended to be inclusive in a manner similar to the term "comprising."

As used herein, the term "if' can be construed to mean "when" or "upon" or "in response to determining" or "in response to detecting," depending on the context. Similarly, the phrase "if it is determined" or "if [a stated condition or event] is detected" can be construed to mean "upon determining" or "in response to determining" or "upon detecting [the stated condition or event]" or "in response to detecting [the stated condition or event]," depending on the context.

It will also be understood that, although the terms first, second, *etc.* are used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject. Furthermore, the terms "subject," "user," and "patient" are used interchangeably herein.

The terms "cancer" refers to or describes the physiological condition in mammals that is typically characterized by unregulated cell growth. Included in this definition are benign and malignant cancers as well as dormant tumors or micrometastases.

As used herein, the term "measure of central tendency" refers to a central or representative value for a distribution of values. Non-limiting examples of measures of central tendency include an arithmetic mean, weighted mean, midrange, midhinge, trimean, geometric mean, geometric median, Winsorized mean, median, and mode of the distribution of values.

As used herein, the term "BAM File" or "Binary file containing Alignment Maps" refers to a file storing sequencing data aligned to a reference sequence (e.g., a reference genome or exome). In some embodiments, a BAM file is a compressed binary version of a SAM (Sequence Alignment Map) file that includes, for each of a plurality of unique sequence reads, an identifier for the sequence read, information about the nucleotide sequence, information about the alignment of the sequence to a reference sequence, and optionally metrics relating to the quality of the sequence read and/or the quality of the sequence alignment. While BAM files generally relate to files having a particular format, for simplicity they are used herein to simply refer to a file, of any format, containing information about a sequence alignment, unless specifically stated otherwise.

As used herein, the term "subject" refers to any living or non-living organism including, but not limited to, a human (*e.g.,* a male human, female human, fetus, pregnant female, child, or the like), a non-human mammal, or a non-human animal. Any human or non-human animal can serve as a subject, including but not limited to mammal, reptile, avian, amphibian, fish, ungulate, ruminant, bovine (*e.g.,* cattle), equine (*e.g.,* horse), caprine and ovine (*e.g.,* sheep, goat), swine *(e.g.,* pig), camelid (*e.g.,* camel, llama, alpaca), monkey, ape (*e.g.,* gorilla, chimpanzee), ursid (*e.g.,* bear), poultry, dog, cat, mouse, rat, fish, dolphin, whale and shark. In some embodiments, a subject is a male or female of any age (*e.g.,* a man, a woman, or a child). In some embodiments, a subject is a human.

As used herein, the terms "expression level," "abundance level," or simply "abundance" refers to an amount of a gene product, (an RNA species, *e.g.,* mRNA or miRNA, or protein molecule) transcribed or translated by a cell, or an average amount of a gene product transcribed or translated across multiple cells. In some embodiments, an expression level can refer to the amount of a particular isoform of an mRNA corresponding to a particular gene that gives rise to multiple mRNA isoforms. The genomic locus can be identified using a gene name, a chromosomal location, or any other genetic mapping metric.

As used herein, the terms "sequencing," "sequence determination," and the like refer to any biochemical processes that may be used to determine the order of biological macromolecules such as nucleic acids or proteins. For example, sequencing data can include all or a portion of the nucleotide bases in a nucleic acid molecule such as an mRNA transcript or a genomic locus.

All references cited herein are incorporated by reference in their entirety.

### A. RNA-seq

In some embodiments, the systems and methods disclosed herein utilize data produced by next generation sequencing of RNA (RNA-seq). The original goal of RNA-seq was to identify which genetic loci are expressed in a cell (population) at a given time over the entire expression range without the need to pre-define the sequences of interest as was the case with cDNA microarrays. RNA-seq has proven to be able to identify even lowly expressed transcripts with a very low level of false positives, especially when compared to cDNA microarrays. In addition, RNA-seq can be used not only for the quantification of expression differences between distinct conditions, it also offers the ability to detect and quantify other RNA transcripts present in cells, such as non-protein-coding transcripts, novel transcripts, sites of protein-RNA interactions, and splice isoforms. It is the identification, quantification, categorization, and documentation of this final type of RNA transcript within the RNA-seq data reads that is the focus of the systems and methods disclosed herein.

The present method contemplates starting with some sort of tissue sample of which information about the entire transcriptome is desired without the necessity of identifying target sequences in advance, although such identification can be an optional approach. This is generally done using total RNA sequencing which can accurately measure gene and transcript abundance and identify known and novel features of the transcriptome. The present method is contemplated to be able to be practiced with total RNA sequencing, it can be equally practiced with a probe captured subset of the total set (see, for example probe panels used for whole exome sequencing (WES, as described in Rabbani et al., J. Hum. Genet., 59:5-15 (2014); Suwinski et al., Front. Genet. 12 Feb. 2019), or another targeted panel of selected genes (e.g. various selected subsets of less than the whole transcriptome) or with the RNA obtained through poly-A capture. More details about this approach are provided below. The sample can be derived directly from a patient either at a tissue sample or some sort of bodily fluid sample, or alternatively, an artificial organoid which is grown from tissue or sample provided from a patient. Samples from archival tissues, where exosomes may be the most rich source of RNA are also contemplated by the systems and methods disclosed herein. When RNA-seq data is desired from a patient sample or an organoid, the first step is the isolation of the RNA from that sample. Methods of RNA isolation are well known in the art and vary depending on the precise tissue or sample type involved. Important considerations include stabilization of the RNA after collection, ensuring complete or substantially complete sample lysis, eliminating or substantially eliminating DNA contamination, and choosing from the variety of RNA isolation kits which is highly dependent on the original RNA source. For examples of RNA isolation techniques, see Conesa A et al., Genome Biol.17:13 (2016).

While direct sequencing of RNA molecules is possible, most RNA-Seq experiments are carried out on instruments that sequence DNA molecules due to the technical maturity of commercial instruments designed for DNA-based sequencing. Therefore, cDNA library preparation from RNA is a required step for many embodiments of RNA-Seq. Each cDNA in an RNA-Seq library is composed of a cDNA insert of certain size flanked by adapter sequences, as required for amplification and sequencing on a specific platform. The cDNA library preparation method varies depending on the RNA species under investigation, which can differ in size, sequence, structural features and abundance. Major considerations include (1) how to capture RNA molecules of interest; (2) how to convert RNA to double-stranded cDNAs with defined size ranges; and (3) how to place adapter sequences on the cDNA ends for amplification and sequencing.

In some embodiments, sequencing of polyadenylated RNA is used in the systems and methods disclosed herein, to allow focus on alternative spliced reads. In eukaryotic organisms, most protein-coding RNAs (mRNAs) and many long noncoding RNAs (lncRNAs) (>200 nt) contain a poly(A) tail. The poly(A) tail provides technical convenience for enrichment of poly(A) + RNAs from total cellular RNA, in which they account for approximately 1-5% of the pool. Poly(A) + RNA selection can be carried out with magnetic or cellulose beads coated with oligo-dT molecules. Alternatively, polyadenylated RNAs can be selected using oligo-dT priming for reverse transcription (RT). While efficiently incorporating both poly(A) selection and RT in one step, oligo-dT priming-based methods can exhibit 3' bias, resulting in sequencing reads enriched for the 3' portion of the transcript. In addition, oligo-dT can frequently prime at internal A-rich sequences of transcripts, a phenomenon called internal poly(A) priming, leading to biased RT. Therefore, poly(A) purification is a preferred method to select poly(A) + RNA unless a very low amount of RNA is available. However, it should be noted that non-polyadenylated RNAs such as fragmented mRNAs from formalin-fixed, paraffin-embedded (FFPE) samples could be of interest using the systems and methods disclosed herein and thus specialized methods of isolation should be utilized, such as those described in Pennock et al., BMC Medical Genomics, 12: 195 (2019).

A major issue in sequencing these RNAs is how to eliminate ribosomal RNAs (rRNAs), which are the most abundant RNA species in the cell but of little interest for the systems and methods disclosed herein and their focus on alternative splicing. Several approaches have been developed to deplete them from the RNA pool. One approach to eliminate rRNAs is based on sequence-specific probes that can hybridize to rRNAs. Unwanted rRNAs or their cDNAs are hybridized with biotinylated DNA or locked nucleic acid (LNA) probes, followed by depletion with streptavidin beads. Alternatively, rRNAs are targeted by anti-sense DNA oligos and digested by RNase H, a method also known as probe-directed degradation (PDD). While this approach is less laborious than hybridization, it may require continuous coverage of rRNAs and unique probe sets. A noncontinuous sequence-based method was recently developed which has addressed some of these issues. In this method, all cDNAs, including those of rRNAs and other RNAs, are circularized, and are hybridized to rRNA probes. The hybridized sequences are then digested by duplex-specific nuclease (DSN), making them unusable for amplification. However, this approach requires high input amounts of total RNA, which can be challenging when dealing with clinical samples.

Another approach for rRNA reduction uses specific, not-so-random (NSR) primers which bind to the RNA molecules of interest during RT, thus avoiding rRNAs. This method, commercialized as Ovation RNA-Seq (Tecan, Mannedorf, Switzerland), uses hexamer or heptamer primers whose sequences are absent from rRNAs. Similar to this approach, one study used 44 heptamers to avoid both rRNAs and highly-expressed transcripts. In this way, only 40 primers for RT instead of 700 NSR primers were needed, which works well with partially degraded RNA and low-input samples. In addition to the sequence-based approaches mentioned above, some methods take advantage of certain features of rRNAs for their elimination. The C0T-hybridization method is based on heat denaturation, re-annealing and selective degradation by DSN. Double-stranded cDNAs originating from abundant sequences are preferentially degraded because of their more rapid annealing kinetics compared to less abundant ones. Selective degradation has also been achieved by using the enzyme terminator 5'-phosphate-dependent exonuclease (TEX), which recognizes RNA molecules with 5'-monophosphate, including rRNAs and tRNAs.

A common clinical starting point is a patient blood sample, in which case a frequently used technique is globin depletion, which employs probe-based removal or inhibition of hemoglobin-related transcripts. This can greatly increase the relative number of reads that will be generated from non-globin RNA, since globin transcripts comprise between 50-80% of blood RNA (see, Mastrokolias et al., BMC Genomics, 13:28 (2012)).

In summary, as well known by one of ordinary skill, the selection of an approach for enriching RNA transcripts of interest for sequencing depends on the goal of the experiment and many technical factors. Several studies have compared protocols for removal of rRNA by depletion- and priming-based methods. In eukaryotic cells, oligo-dT bead-based purification of poly(A) + RNA is the method of choice for most applications, because of its ease of use and relatively low cost. For low-input samples, however, oligo-dT priming generally offers better results.

After poly(A) + selection or rRNA depletion, RNA samples are typically subject to RNA fragmentation to a certain size range before RT. In certain embodiments, this is necessary because of the size limitation of most current sequencing platforms. RNAs can be fragmented with alkaline solutions, solutions with divalent cations, such Mg++, Zn++, or enzymes, such as RNase III. Fragmentation with alkaline solutions or divalent cations is typically carried out at an elevated temperature, such as 70°C, to mitigate the effect of RNA structure on fragmentation. Alternatively, intact RNAs can be reverse transcribed, and full-length cDNA can be fragmented. A traditional method to fragment cDNA requires the use of acoustic shearing. Alternatively, full-length double-stranded cDNAs can be fragmented by DNase or a tagmentation method can be used to fragment cDNA and add adapter sequences at the same time. In this method, an active variant of the Tn5 transposase mediates the fragmentation of double-stranded DNA and ligates adapter oligonucleotides at both ends in a quick reaction (~5 min) (see, Picelli et al., Genome Res. 2014;24:2033-2040). However, it is notable that Tn5 and other enzyme-based cDNA fragmentation methods may require a precise enzyme:DNA ratio, making method optimization less straightforward than RNA fragmentation. Consequently, fragmenting RNA is currently still the most frequently used approach in RNA-Seq library preparation.

In a standard RNA-Seq library protocol, cDNAs of a desired size are generated from RT of fragmented RNAs with random hexamer primers or from fragmented full-length cDNAs that are ligated to DNA adapters before amplification and sequencing. Due to the detection limit of most sequencers, cDNA libraries may need to be amplified by a polymerase chain reaction (PCR) process before sequencing. While only a small number of amplification cycles (8-12) are used during most embodiments of PCR, variations in cDNA size and composition can result in uneven amplification. Amplification of some cDNAs plateau while others continue to amplify exponentially. To correct for PCR amplification bias, methods that eliminate PCR duplicates from sequencing results may be used . In one method, under the assumption of random RNA fragmentation, final sequencing reads having the same start and stop coordinates are considered as PCR duplicates and are merged. Another method is to use molecular labels, also known as unique molecular identifiers (UMIs), to distinguish PCR products. Molecular labels are typically introduced within the adapter sequence, prior to PCR amplification. In a modified protocol for making cDNAs from single cells, molecular labels are introduced by the Tn5 transposase during fragmentation of double-stranded, amplified cDNA. However, in some applications, such as digital counting of targeted RNAs, molecular labels are added during RT. Molecular labels differ in size (number of bases) and complexity. In principle, they comprise either defined sequences or random nucleotides. Defined sequences, chosen for their even distribution in final libraries, are more technically challenging to make in some embodiments because of sequence selection and manufacturing complexity. By contrast, random sequences, while easy to implement, give high variability among molecular labels. Molecular labeling is particularly valuable in situations where input RNA is scarce and a large number of PCR cycles is required for sequencing, such as single-cell RNA-seq. Although the present methods anticipate the utilization of traditional RNA-seq approaches as described above, it is also anticipated that single-cell RNA-seq and related methods, for example but not limited to those that begin with less input material, could be the source of reads for use in the present method.

A further method that can be utilized is a combination of RNA-Seq with exome enrichment (see, for example Cieslik et al., Genome Res. 25(9):1372-81 (2015)). This method involves utilizing a panel of complementary capture probes that has been developed for whole exome sequencing. This method differs from traditional RNA-seq sample preparation in that there is no poly-A selection. Instead, enrichment is generally done after the main enzymatic steps of library construction and a subset of PCR cycles. Unique to these approaches is a capture reaction (RNA-DNA hybridization) using exon-targeting RNA probes, followed by a washing step, and an additional set of PCR cycles. A motivation for utilizing such an approach with the systems and methods disclosed herein is the observation that coverage of splice junctions is quite high when utilizing a capture library step. There are a number of commercial sources for whole exome sequencing kits that can be used in the capture reaction of this approach such as Integrated DNA Technologies' (IDT) xGen Exome Research Panel v2 (Coralville, IA); Qiagen's QIAseq Human Exome Kits (Venlo, Netherlands); and Agilent's SureSelect Human All Exon (Santa Clara, CA).

Data produced by the sequencers are produced in a format called Binary Base Call (BCL). BCL files are stored in binary format and represent raw data output of a sequencing run. Ultimately, the BCL file is converted for use and storage in a format called FASTQ. This is a text-based format for storing both a biological sequence, in this case a nucleotide sequence and its corresponding quality scores, see Cock et al. Nuc. Acids Res. 38(6): 1767-71 (2009). As is well known to one of ordinary skill, the sequence letter and quality scores are each encoded using a single ASCII character for brevity. Although originally developed by the Wellcome Trust Sanger Institute to bundle FASTA format sequence data with quality information, it is now the de facto standard for storing the output of high-throughput sequencing instruments. As such, it is contemplated in embodiments of the systems and methods disclosed herein that this standard format will generally be used for the RNA-seq output files.

### B. Systems

Figure 1 shows a block diagram illustrating a system 100 predicting an effect of a pharmaceutical agent in a test subject and/or for identifying one or more tissue organoids in a plurality of tissue organoids matching a biological property of a tissue in a subject, in accordance with some embodiments of the present disclosure. The device 100 in some implementations includes one or more central processing units (CPU(s)) 102 (also referred to as processors), one or more network interfaces 104, a user interface 106, a non-persistent memory 111, a persistent memory 112, and one or more communication buses 110 for interconnecting these components. The one or more communication buses 110 optionally include circuitry (sometimes called a chipset) that interconnects and controls communications between system components. The non-persistent memory 111 typically includes high-speed random access memory, such as DRAM, SRAM, DDR RAM, ROM, EEPROM, flash memory, whereas the persistent memory 112 typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid state storage devices. The persistent memory 112 optionally includes one or more storage devices remotely located from the CPU(s) 102. The persistent memory 112, and the non-volatile memory device(s) within the non-persistent memory 112, comprises non-transitory computer readable storage medium. In some implementations, the non-persistent memory 111 or alternatively the non-transitory computer readable storage medium stores the following programs, modules and data structures, or a subset thereof, sometimes in conjunction with the persistent memory 112:
- an optional operating system 30, which includes procedures for handling various basic system services and for performing hardware dependent tasks;
- an optional network communication module (or instructions) for connecting the system 100 with other devices, or a communication network;
- an optional sequence read analysis module 40 for determining mRNA abundance values from sequence reads (e.g., sequence reads 72), e.g., including:
   ∘ a sequence alignment algorithm 42 for aligning sequence reads (e.g., sequence reads 72), e.g., generated from an mRNA sample, to a reference construct for the species of the subject, e.g., a reference genome, exome, transcriptome, or other partial genomic construct, and/or
   ∘ an abundance value extraction algorithm 44 for determining abundance values for mRNA species, e.g., based on aligned sequence reads (e.g., sequence reads 72);
- a prediction module 50 for predicting an effect of a pharmaceutical agent in a test subject and/or for identifying one or more tissue organoids in a plurality of tissue organoids matching a biological property of a tissue in a subject, including a multi-task prediction model 52 with a dimensionality reduction mapping function 54 and model parameters 56 (e.g., weights, biases, cluster centroids, etc.);
- an optional model interpretation module 60 for generating advice based on the output of a multi-task prediction model (e.g., prediction model 52), e.g., including:
   ∘ a therapy matching algorithm 62 for recommending a therapy for a test subject based on a model output,
   ∘ a clinical trial matching algorithm 64 for matching a test subject to a clinical trial based on a model output, and/or
   ∘ a tissue organoid matching module 66 for identifying one or more tissue organoids in a plurality of tissue organoids matching a biological property of a tissue in a subject, e.g., including a clustering algorithm 67 (e.g., for clustering a latent representation of gene expression data from the subject with latent representations of gene expression data for the tissue organoids), a geometric distance algorithm 68 (e.g., for determining a distance between a latent representation of gene expression data from the subject and a latent representation of gene expression data for a tissue organoid), and/or matching criterion 69 (e.g., for use in determining whether a tissue organoid in the plurality of tissue organoids matches the test subject);
- a test subject data store 70 for storing inputs and/or outputs from a multi-task predictive model described herein (e.g., predictive model 52), e.g.,
   ∘ sequence reads 72 for determining mRNA abundance values,
   ∘ cellular constituent abundance values 74 (e.g., mRNA abundance values) used as inputs to a multi-task predictive model described herein (e.g., predictive model 52),
   ∘ model outputs 76, e.g., predicted pharmaceutical effects 77, cell type classifications 78, and/or latent representation coordinates; and
- A reporting module 80, e.g., for generating a report for a clinician or patient based on at least an output or a latent representation from a multi-task predictive model (e.g., predictive model 52).

In some implementations, one or more of the above identified elements are stored in one or more of the previously mentioned memory devices and correspond to a set of instructions for performing a function described above. The above identified modules, data, or programs (*e.g.,* sets of instructions) need not be implemented as separate software programs, procedures, data sets, or modules, and thus various subsets of these modules and data may be combined or otherwise re-arranged in various implementations. In some implementations, the non-persistent memory 111 optionally stores a subset of the modules and data structures identified above. Furthermore, in some embodiments, the memory stores additional modules and data structures not described above. In some embodiments, one or more of the above identified elements is stored in a computer system, other than that of system 100, that is addressable by system 100 so that system 100 may retrieve all or a portion of such data when needed.

Although Figure 1 depicts a "system 100," the figure is intended more as a functional description of the various features which may be present in computer systems than as a structural schematic of the implementations described herein. In practice, and as recognized by those of ordinary skill in the art, items shown separately could be combined and some items could be separated. Moreover, although Figure 1 depicts certain data and modules in non-persistent memory 111, some or all of these data and modules may be in persistent memory 112.

Some embodiments of the systems and methods disclosed herein involve systems that have been configured for the performance of steps of the present methods. Such systems can be described as comprising primarily a computational device. At a minimum, the systems will comprise at least one processor and at least one memory. The device in some implementations includes one or more processing units CPU(s) (also referred to as processors), one or more network interfaces, a user interface, for example, including a display and/or an input (for example, a mouse, touchpad, keyboard, etc.), a non-persistent memory, a persistent memory, and one or more communication buses for interconnecting these components. The one or more communication buses optionally include circuitry (sometimes called a chipset) that interconnects and controls communications between system components. The non-persistent memory typically includes high-speed random-access memory, such as DRAM, SRAM, DDR RAM, ROM, EEPROM, flash memory, whereas the persistent memory typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid state storage devices.

The persistent memory optionally includes one or more storage devices remotely located from the CPU(s). The persistent memory, and the non-volatile memory device(s) within the non-persistent memory, comprise non-transitory computer readable storage medium. In some implementations, the non-persistent memory or alternatively the non-transitory computer readable storage medium stores the following programs, modules and data structures, or a subset thereof, sometimes in conjunction with the persistent memory: an operating system, which includes procedures for handling various basic system services and for performing hardware dependent tasks; a network communication module (or instructions) for connecting the system with other devices and/or a communication network; a test patient data store for storing one or more collections of features from patients (for example, subjects); a bioinformatics module for processing sequencing data and extracting features from sequencing data, for example, from liquid biopsy, solid tumor, or other sequencing assays, including next generation sequencing assays; a feature analysis module for evaluating patient features, for example, genomic alterations, compound genomic features, and clinical features; and a reporting module for generating and transmitting reports that provide clinical support for personalized cancer therapy.

Although the above description depicts a "system," this description is intended more as a functional description of the various features that may be present in computer systems than as a structural schematic of the implementations described herein. In practice, and as recognized by those of ordinary skill in the art, items shown separately could be combined and some items could be separated. The relationship between persistent and non-persistent memory described in possible association that is not intended to be limiting. For example, in various implementations, one or more of the above identified elements are stored in one or more of the previously mentioned memory devices and correspond to a set of instructions for performing a function described above. The above identified modules, data, or programs (for example, sets of instructions) need not be implemented as separate software programs, procedures, datasets, or modules, and thus various subsets of these modules and data may be combined or otherwise re-arranged in various implementations.

In some implementations, the non-persistent memory optionally stores a subset of the modules and data structures identified above. Furthermore, in some embodiments, the memory stores additional modules and data structures not described above. In some embodiments, one or more of the above-identified elements is stored in a computer system, other than that of the system, that is addressable by the system so that the system may retrieve all or a portion of such data when needed.

One such illustrative example is the system as a single computer that includes all of the functionality for providing methods of detecting alternative splicing variants. However, while a single machine is possible, the term "system" shall also be taken to include any collection of machines that individually or jointly execute a set (or multiple sets) of instructions to perform any one or more of the methodologies discussed herein.

For example, in some embodiments, the system includes one or more computers. In some embodiments, the functionality for detecting, classifying, and documenting alternative splicing variants is spread across any number of networked computers and/or resides on each of several networked computers and/or is hosted on one or more virtual machines at a remote location accessible across the communications network. For example, different portions of the various modules and data stores can be stored and/or executed on the various instances of a processing device and/or processing server/database in the distributed diagnostic environment (for example, multiple processing devices, a processing server, and a database).

The system may operate in the capacity of a server or a client machine in client-server network environment, as a peer machine in a peer-to-peer (or distributed) network environment, or as a server or a client machine in a cloud computing infrastructure or environment. The system may be a personal computer (PC), a tablet PC, a set-top box (STB), a Personal Digital Assistant (PDA), a cellular telephone, a web appliance, a server, a network router, a switch or bridge, or any machine capable of executing a set of instructions (sequential or otherwise) that specify actions to be taken by that machine.

In another implementation, the system comprises a virtual machine that includes a module for executing instructions for performing any one or more of the methodologies disclosed herein. In computing, a virtual machine (VM) is an emulation of a computer system that is based on computer architectures and provides functionality of a physical computer. Some such implementations may involve specialized hardware, software, or a combination of hardware and software.

While systems in accordance with the present disclosure have been disclosed with reference to Figure 1, methods in accordance with the present disclosure are now detailed with reference to Figures 2A-2E, 3A-3F, and 4A-4G.

In one aspect, the disclosure provides a method 200 for predicting an effect of a pharmaceutical agent in a test subject of a first species. In some embodiments, such a method is performed at a computer system (e.g., system 100) comprising at least one processor and a memory storing at least one program for execution by the at least one processor.

Referring to block 201, in some embodiments, the method includes obtaining, in electronic form, a plurality of nucleic acid sequences for mRNA from the biological sample of the test subject. Referring to block 202, in some embodiments, the plurality of nucleic acid sequences is at least 1000 nucleic acid sequences, at least 10,000 nucleic acid sequences, at least 100,000 nucleic acid sequences, at least 250,000 nucleic acid sequences, at least 500,000 nucleic acid sequences, at least 1,000,000 nucleic acid sequences, or more nucleic acid sequences. Referring to block 204, in some embodiments, the method includes sequencing the mRNA from the biological sample of the test subject, thereby obtaining the plurality of nucleic acid sequences.

Referring to block 206, in some embodiments, the method includes determining, for each respective cellular constituent in the plurality of cellular constituents, the corresponding abundance value from the plurality of nucleic acid sequences. In some embodiments, the plurality of cellular constituents is at least 5 cellular constituents, at least 10 cellular constituents, at least 25 cellular constituents, at least 50 cellular constituents, at least 100 cellular constituents, at least 250 cellular constituents, at least 500 cellular constituents, at least 1000 cellular constituents, at least 2500 cellular constituents, at least 5000 cellular constituents, at least 10,000 cellular constituents, at least 15,000 cellular constituents, at least 20,000 cellular constituents, or more cellular constituents.

Referring to block 207, in some embodiments, the method includes inputting information about the test subject into a multi-task model including a plurality of parameters, where the multi-task model applies the plurality of parameters to the information about the test subject through a plurality of instructions to generate, as output from the multi-task model, a plurality of outputs including (i) a predicted effect of the pharmaceutical agent in the test subject and (ii) for each respective cell type variable in a set of one or more cell type variables, a corresponding cell type classification, where the information about the test subject includes a plurality of abundance values, the plurality of abundance values including, for each respective cellular constituent in a plurality of cellular constituents, a corresponding abundance value for the abundance of the respective cellular constituent in a biological sample of the test subject. Referring to block 208, in some embodiments, the predicted effect includes a prediction for cell death of a cancer cell in the subject in response to administration of the pharmaceutical agent to the subject.

Referring to block 210, in some embodiments, the pharmaceutical agent is a chemotherapeutic agent. Referring to block 212, in some embodiments, the pharmaceutical agent is selected from the group consisting of lenalidomid, pembrolizumab, trastuzumab, bevacizumab, rituximab, ibrutinib, human papillomavirus quadrivalent (types 6, 11, 16, and 18) vaccine, pertuzumab, pemetrexed, nilotinib, denosumab, abiraterone acetate, promacta, imatinib, everolimus, palbociclib, erlotinib, bortezomib, bortezomib, nivolumab, atezolizumab, daratumumab, enzalutamide, obinutuzumab, ruxolitinib, venetoclax, osimertinib, and pomalidomide.

Referring to block 214, in some embodiments, the multi-task model includes a partially connected neural network defining a plurality of tasks, each respective task in the plurality of tasks corresponding to a respective output in the plurality of outputs, where the partially-connected neural network includes (a) a first set of layers shared between the plurality of tasks and (b) for each respective task in the plurality of tasks a corresponding second set of layers unique to the respective task. Referring to block 216, in some embodiments, the multi-task model includes a linear mapping function that transforms the plurality of abundance values into a first latent feature space including fewer dimensions than the number of respective cellular constituents in the plurality of cellular constituents.

Referring to block 218, in some embodiments, the plurality of parameters is at least 1000 parameters, at least 10,000 parameters, at least 100,000 parameters, at least 250,000 parameters, at least 500,000 parameters, at least 1,000,000 parameters, at least 2,500,000 parameters, at least 5,000,000 parameters, at least 10,000,000 parameters, at least 25,000,000 parameters, at least 50,000,000 parameters, at least 100,000,000 parameters, or more parameters.

Referring to block 220, in some embodiments, the plurality of instructions is at least 1000 instructions, at least 10,000 instructions, at least 100,000 instructions, at least 250,000 instructions, at least 500,000 instructions, at least 1,000,000 instructions, at least 2,500,000 instructions, at least 5,000,000 instructions, at least 10,000,000 instructions, at least 25,000,000 instructions, at least 50,000,000 instructions, at least 100,000,000 instructions, or more instructions.

Referring to block 222, in some embodiments, the set of one or more cell type variables includes a variable selected from the group consisting of cell histology, disease type (e.g., cancer type), disease stage (e.g., cancer stage), disease grade (e.g., cancer grade), tissue type, and tissue site.

Referring to block 224, in some embodiments, the plurality of cellular constituents is at least 10 cellular constituents, at least 25 cellular constituents, at least 50 cellular constituents, at least 100 cellular constituents, at least 250 cellular constituents, at least 500 cellular constituents, at least 1000 cellular constituents, at least 2500 cellular constituents, at least 5000 cellular constituents, at least 10,000 cellular constituents, at least 20,000 cellular constituents, or more cellular constituents.

Referring to block 226, in some embodiments, each respective cellular constituent in the plurality of cellular constituents is a different mRNA species. In some embodiments, the plurality of cellular constituents includes at least 5 mRNA species, at least 10 mRNA species, at least 25 mRNA species, at least 50 mRNA species, at least 100 mRNA species, at least 250 mRNA species, at least 500 mRNA species, at least 1000 mRNA species, at least 2500 mRNA species, at least 5000 mRNA species, at least 10,000 mRNA species, at least 15,000 mRNA species, at least 20,000 mRNA species, or more mRNA species. In some embodiments, the plurality of cellular constituents includes the mRNA species in a transcriptome. In some embodiments, mRNA abundance values (expression levels) are adjusted to be expressed as a value relative to total expression levels in a sample and/or to a reference expression level. In some embodiments, mRNA abundance values are normalized. In some embodiments, mRNA abundance values are expressed as absolute abundance values.

Referring to block 228, in some embodiments, the biological sample of the subject is a diseased tissue of the subject. Referring to block 230, in some embodiments, the diseased tissue of the subject is a cancerous tissue. In some embodiments, the cancerous tissue is a solid tissue biopsy. Referring to block 232, in some embodiments, the biological sample of the subject includes a biological fluid from the subject. In some embodiments, the biological sample is a liquid biopsy sample.

Referring to block 234, in some embodiments, the subject has a cancer selected from the group consisting of a carcinoma, lymphoma, blastoma, glioblastoma, sarcoma, leukemia, breast cancer, squamous cell cancer, lung cancer, small-cell lung cancer, non-small cell lung cancer (NSCLC), adenocarcinoma of the lung, squamous carcinoma of the lung, head and neck cancer, cancer of the peritoneum, hepatocellular cancer, gastric or stomach cancer, pancreatic cancer, ovarian cancer, cervical cancer, liver cancer, bladder cancer, hepatoma, colon cancer, colorectal cancer, endometrial or uterine carcinoma, salivary gland carcinoma, kidney or renal cancer, liver cancer, prostate cancer, vulval cancer, thyroid cancer, hepatic carcinoma, B-cell lymphoma, low grade/follicular non-Hodgkin's lymphoma (NHL), small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, Waldenstrom's Macroglobulinemia, chronic lymphocytic leukemia (CLL), acute lymphoblastic leukemia (ALL), hairy cell leukemia, and chronic myeloblastic leukemia.

Referring to block 235, in some embodiments, when the predicted effect of the pharmaceutical agent in the test subject satisfies a first set of one or more criterion, the method includes recommending a first therapy that includes administration of the pharmaceutical agent to the subject, and when the predicted effect of the pharmaceutical agent in the test subject does not satisfy the first set of one or more criterion, the method includes recommending a second therapy that is different from the first therapy.

Referring to block 236, in some embodiments, when the predicted effect of the pharmaceutical agent in the test subject satisfies a first set of one or more criterion, the method includes administering a first therapy to the subject, where the first therapy that includes administration of the pharmaceutical agent, and when the predicted effect of the pharmaceutical agent in the test subject does not satisfy the first set of one or more criterion, the method includes administering a second therapy to the subject, where the second therapy is different from the first therapy.

Referring to block 238, in some embodiments, When the predicted effect of the pharmaceutical agent in the test subject satisfies a first set of one or more criterion, the method includes recommending the test subject for a clinical trial of the pharmaceutical agent to the subject, and when the predicted effect of the pharmaceutical agent in the test subject does not satisfy the first set of one or more criterion, the method includes not recommending the test subject for the clinical trial.

In some embodiments, the method includes generating a clinical report based on at least one or more outputs from the model. Report generation may further comprise variant science analysis, including the interpretation of variants (including somatic and germline variants as applicable) for pathogenic and biological significance. The variant science analysis may also estimate, e.g., microsatellite instability (MSI) or tumor mutational burden. Targeted treatments may be identified based on one or more outputs from the model and/or variant science analysis, e.g., for further consideration and review by a physician. In some aspects, clinical trials may be identified for which the patient may be eligible, based on one or more outputs from the model, variant science analysis, and/or clinical history. A validation step may occur, after which the report may be finalized for sign-out and delivery. In some embodiments, a first or second report may include additional data provided through a clinical dataflow, such as patient progress notes, pathology reports, imaging reports, and other relevant documents. Such clinical data is ingested, reviewed, and abstracted based on a predefined set of curation rules. The clinical data is then populated into the patient's clinical history timeline for report generation. Further examples of methods and systems for generating clinical reports are disclosed in US Patent Publication No. 2020/0255909, which is hereby incorporated herein by reference in its entirety.

In one aspect, the disclosure provides a method 300 for identifying one or more tissue organoids in a plurality of tissue organoids matching a biological property of a tissue in a subject. In some embodiments, such a method is performed at a computer system (e.g., system 100) comprising at least one processor and a memory storing at least one program for execution by the at least one processor.

Referring to block 302, in some embodiments, the method includes obtaining, in electronic form, a plurality of nucleic acid sequences for mRNA from the biological sample of the test subject. Referring to block 304, in some embodiments, the plurality of nucleic acid sequences is at least 1000 nucleic acid sequences, at least 10,000 nucleic acid sequences, at least 100,000 nucleic acid sequences, at least 250,000 nucleic acid sequences, at least 500,000 nucleic acid sequences, at least 1,000,000 nucleic acid sequences, or more nucleic acid sequences. Referring to block 306, in some embodiments, sequencing the mRNA from the biological sample of the test subject, thereby obtaining the plurality of nucleic acid sequences.

In some embodiments, mRNA abundance values (expression levels) are adjusted to be expressed as a value relative to total expression levels in a sample and/or to a reference expression level. In some embodiments, mRNA abundance values are normalized. In some embodiments, mRNA abundance values are expressed as absolute abundance values.

Referring to block 308, in some embodiments, the method includes determining, for each respective cellular constituent in the plurality of cellular constituents, the corresponding abundance value from the plurality of nucleic acid sequences.

Referring to block 309, in some embodiments, the method includes inputting information about the test subject into a multi-task model comprising a plurality of parameters and one or more hidden layers, wherein the multi-task model is trained to apply the plurality of parameters to the information about the test subject through a plurality of instructions to generate, as output from the multi-task model, a plurality of outputs comprising (i) a predicted effect of the pharmaceutical agent in the test subject and (ii) for each respective cell type variable in a set of one or more cell type variables, a corresponding cell type classification, wherein the information about the test subject comprises, for each respective cellular constituent in a plurality of cellular constituents, a corresponding abundance value for the abundance of the respective cellular constituent in a biological sample of the test subject.

Referring to block 310, in some embodiments, the multi-task model comprises a partially connected neural network defining a plurality of tasks, each respective task in the plurality of tasks corresponding to a respective output in the plurality of outputs, wherein the partially-connected neural network comprises (a) a first set of layers shared between the plurality of tasks and (b) for each respective task in the plurality of tasks a corresponding second set of layers unique to the respective task.

Referring to block 318, in some embodiments, the multi-task model includes a linear mapping function that transforms the plurality of abundance values into a first latent feature space including fewer dimensions than the number of respective cellular constituents in the plurality of cellular constituents.

Referring to block 320, in some embodiments, the plurality of parameters is at least 1000 parameters, at least 10,000 parameters, at least 100,000 parameters, at least 250,000 parameters, at least 500,000 parameters, at least 1,000,000 parameters, at least 2,500,000 parameters, at least 5,000,000 parameters, at least 10,000,000 parameters, at least 25,000,000 parameters, at least 50,000,000 parameters, at least 100,000,000 parameters, or more parameters.

Referring to block 322, in some embodiments, the plurality of instructions is at least 1000 instructions, at least 10,000 instructions, at least 100,000 instructions, at least 250,000 instructions, at least 500,000 instructions, at least 1,000,000 instructions, at least 2,500,000 instructions, at least 5,000,000 instructions, at least 10,000,000 instructions, at least 25,000,000 instructions, at least 50,000,000 instructions, at least 100,000,000 instructions, or more instructions.

Referring to block 324, in some embodiments, the set of one or more cell type variables includes a variable selected from the group consisting of cell histology, disease type (e.g., cancer type), disease stage (e.g., cancer stage), disease grade (e.g., cancer grade), tissue type, and tissue site.

Referring to block 326, in some embodiments, the plurality of cellular constituents is at least 10 cellular constituents, at least 25 cellular constituents, at least 50 cellular constituents, at least 100 cellular constituents, at least 250 cellular constituents, at least 500 cellular constituents, at least 1000 cellular constituents, at least 2500 cellular constituents, at least 5000 cellular constituents, at least 10,000 cellular constituents, at least 20,000 cellular constituents, or more cellular constituents. Referring to block 328, in some embodiments, each respective cellular constituent in the plurality of cellular constituents is a different mRNA species.

Referring to block 330, in some embodiments, the biological sample of the subject includes a diseased tissue of the subject. Referring to block 332, in some embodiments, the diseased tissue of the subject is a cancerous tissue. In some embodiments, the cancerous tissue is a solid tissue biopsy. Referring to block 334, in some embodiments, the biological sample of the subject includes a biological fluid from the subject. In some embodiments, the biological sample is a liquid biopsy sample.

Referring to block 336, in some embodiments, the subject has a cancer selected from the group consisting of a carcinoma, lymphoma, blastoma, glioblastoma, sarcoma, leukemia, breast cancer, squamous cell cancer, lung cancer, small-cell lung cancer, non-small cell lung cancer (NSCLC), adenocarcinoma of the lung, squamous carcinoma of the lung, head and neck cancer, cancer of the peritoneum, hepatocellular cancer, gastric or stomach cancer, pancreatic cancer, ovarian cancer, cervical cancer, liver cancer, bladder cancer, hepatoma, colon cancer, colorectal cancer, endometrial or uterine carcinoma, salivary gland carcinoma, kidney or renal cancer, liver cancer, prostate cancer, vulval cancer, thyroid cancer, hepatic carcinoma, B-cell lymphoma, low grade/follicular non-Hodgkin's lymphoma (NHL), small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, Waldenstrom's Macroglobulinemia, chronic lymphocytic leukemia (CLL), acute lymphoblastic leukemia (ALL), hairy cell leukemia, and chronic myeloblastic leukemia.

Referring to block 338, in some embodiments, the method includes obtaining a latent representation of the information about the test subject from a respective hidden layer in the one or more hidden layers. Referring to block 312, in some embodiments, the respective hidden layer is a respective layer in the first set of layers shared between the plurality of tasks. Referring to block 314, in some embodiments, the respective hidden layer is a respective layer in a corresponding second set of layers unique to a respective task in the plurality of tasks. Referring to block 316, in some embodiments, the output corresponding to the respective task is the predicted effect of the pharmaceutical agent in the test subject.

Referring to block 339, in some embodiments, the method includes comparing the latent representation of the information about the test subject to a plurality of latent representations, wherein each respective latent representation in the plurality of latent representations is of information about a respective tissue organoid, in a plurality of tissue organoids, obtained from the same hidden layer of the multi-task model.

Referring to block 340, in some embodiments, comparing the latent representation of the information about the test subject to the plurality of latent representations comprises clustering the latent representation of the information about the test subject with the plurality of latent representations. Referring to block 342, in some embodiments, the clustering generates a set of clusters including a first respective cluster comprising the latent representation of the information about the test subject, and the set of one or more similarity criterion comprises a criteria that the latent representation of information about the respective tissue organoid is in the first respective cluster.

Referring to block 344, in some embodiments, comparing the latent representation of the information about the test subject to the plurality of latent representations comprises determining, for each respective tissue organoid in at least a subset of the plurality of tissue organoids, a corresponding geometric distance between the latent representation of the information about the test subject and the corresponding latent representation of the information about the respective tissue organoid, and the set of one or more similarity criterion comprises a distance criteria. Referring to block 346, in some embodiments, the distance criteria is that the latent representation of information about the respective tissue organoid is within a threshold geometric distance of the latent representation of the information about the test subject. Referring to block 348, in some embodiments, the distance criteria is that the latent representation of information about the respective tissue organoid is within a threshold number of closest latent representations, in the plurality of latent representations, to the latent representation of the information about the test subject. Referring to block 350, in some embodiments, the distance criteria is that the latent representation of information about the respective tissue organoid is the closest latent representation, in the plurality of latent representations, to the latent representation of the information about the test subject.

Referring to block 352, in some embodiments, the method includes identifying one or more respective tissue organoids, in the plurality of tissue organoids, that satisfy a set of one or more similarity criterion based on the comparing, thereby identifying the one or more tissue organoids matching the biological property of the tissue in the subject. Referring to block 354, in some embodiments, the plurality of tissue organoids comprises a plurality of tumor organoids.

In one aspect, the disclosure provides a method 400 for training a model to predict an effect of a candidate pharmaceutical agent in a test subject of a first species. In some embodiments, such a method is performed at a computer system (e.g., system 100) comprising at least one processor and a memory storing at least one program for execution by the at least one processor.

Referring to block 402, in some embodiments, the method includes obtaining, for each respective training sample in a first plurality of training samples, where each respective training sample in the first plurality of training samples includes a tissue organoid or tissue organoid culture, formed from cells of the first species, that has been exposed to a perturbation: (i) a corresponding plurality of abundance values including, for each respective cellular constituent in a plurality of cellular constituents, a corresponding abundance value for the abundance of the respective cellular constituent in the respective training sample after exposure to the candidate pharmaceutical agent, (ii) a corresponding experimentally measured effect of the candidate pharmaceutical agent on the respective training sample, and (iii) a corresponding set of one or more cell type classifications including, for each respective cell type variable in a set of one or more cell type variables, a corresponding cell type classification for the respective cell type variable.

Referring to block 404, in some embodiments, the pharmaceutical agent is a chemotherapeutic agent. Referring to block 406, in some embodiments, the pharmaceutical agent is selected from the group consisting of lenalidomid, pembrolizumab, trastuzumab, bevacizumab, rituximab, ibrutinib, human papillomavirus quadrivalent (types 6, 11, 16, and 18) vaccine, pertuzumab, pemetrexed, nilotinib, denosumab, abiraterone acetate, promacta, imatinib, everolimus, palbociclib, erlotinib, bortezomib, bortezomib, nivolumab, atezolizumab, daratumumab, enzalutamide, obinutuzumab, ruxolitinib, venetoclax, osimertinib, and pomalidomide.

Referring to block 408, in some embodiments, the first plurality of training samples is at least 25 training samples, at least 50 training samples, at least 100 training samples, at least 250 training samples, at least 500 training samples, at least 1000 training samples, at least 2500 training samples, at least 5000 training samples, at least 1000 training samples, or more training samples.

Referring to block 410, in some embodiments, for a respective training sample in the first plurality of training samples, the corresponding tissue organoid or tissue organoid culture is a tumor organoid or a tumor organoid culture.

Referring to block 412, in some embodiments, the plurality of cellular constituents is at least 10 cellular constituents, at least 25 cellular constituents, at least 50 cellular constituents, at least 100 cellular constituents, at least 250 cellular constituents, at least 500 cellular constituents, at least 1000 cellular constituents, at least 2500 cellular constituents, at least 5000 cellular constituents, at least 10,000 cellular constituents, at least 20,000 cellular constituents, or more cellular constituents. Referring to block 414, in some embodiments, each respective cellular constituent in the plurality of cellular constituents is a different mRNA species.

In some embodiments, mRNA abundance values (expression levels) are adjusted to be expressed as a value relative to total expression levels in a sample and/or to a reference expression level. In some embodiments, mRNA abundance values are normalized. In some embodiments, mRNA abundance values are expressed as absolute abundance values.

Referring to block 416, in some embodiments, the set of one or more cell type variables includes a variable selected from the group consisting of cell histology, disease type (e.g., cancer type), disease stage (e.g., cancer stage), disease grade (e.g., cancer grade), tissue type, and tissue site.

Referring to block 417, in some embodiments, the method includes obtaining, for each respective training sample in a second plurality of training samples, where each respective training sample in the second plurality of training samples includes a biological sample from a respective subject in a plurality of subjects of the first species: (i) a corresponding plurality of abundance values including, for each respective cellular constituent in the plurality of cellular constituents, a corresponding abundance value for the abundance of the respective cellular constituent in the respective training sample, and (ii) a corresponding set of one or more cell type classifications including, for each respective cell type variable in the set of one or more cell type variables, a corresponding cell type classification for the respective cell type variable.

Referring to block 418, in some embodiments, for a respective training sample in the second plurality of training samples, the corresponding biological sample includes a diseased tissue of the corresponding subject. Referring to block 420, in some embodiments, the diseased tissue of the corresponding subject is a cancerous tissue. In some embodiments, the cancerous tissue is a solid tissue biopsy. Referring to block 422, in some embodiments, for a respective training sample in the second plurality of training samples, the corresponding biological sample includes a biological fluid from the subject. In some embodiments, the biological sample is a liquid biopsy sample.

Referring to block 424, in some embodiments, for a respective training sample in the second plurality of training samples, the corresponding the subject has a cancer selected from the group consisting of a carcinoma, lymphoma, blastoma, glioblastoma, sarcoma, leukemia, breast cancer, squamous cell cancer, lung cancer, small-cell lung cancer, non-small cell lung cancer (NSCLC), adenocarcinoma of the lung, squamous carcinoma of the lung, head and neck cancer, cancer of the peritoneum, hepatocellular cancer, gastric or stomach cancer, pancreatic cancer, ovarian cancer, cervical cancer, liver cancer, bladder cancer, hepatoma, colon cancer, colorectal cancer, endometrial or uterine carcinoma, salivary gland carcinoma, kidney or renal cancer, liver cancer, prostate cancer, vulval cancer, thyroid cancer, hepatic carcinoma, B-cell lymphoma, low grade/follicular non-Hodgkin's lymphoma (NHL), small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, Waldenstrom's Macroglobulinemia, chronic lymphocytic leukemia (CLL), acute lymphoblastic leukemia (ALL), hairy cell leukemia, and chronic myeloblastic leukemia.

Referring to block 425, in some embodiments, the method includes performing a first dimensionality reduction analysis across the corresponding plurality of abundance values for each respective training sample in the first plurality of training samples, thereby: learning a first mapping function that maps a corresponding plurality of abundance values including, for each respective cellular constituent in the plurality of cellular constituents, a corresponding abundance value for the abundance of the respective cellular constituent in a tissue organoid or tissue organoid culture, formed from cells of the first species, that has been exposed to a perturbation, into a first latent feature space including a first plurality of dimensions that is less than the number of cellular constituents in the plurality of constituents, and generating, for each respective training sample in the first plurality of training samples, a first corresponding representation of the corresponding plurality of abundance values in the first latent feature space according to the first mapping function.

Referring to block 426, in some embodiments, the method includes performing a second dimensionality reduction analysis across the corresponding plurality of abundance values for each respective training sample in the second plurality of training samples, thereby: learning a second mapping function that maps a corresponding plurality of abundance values including, for each respective cellular constituent in the plurality of cellular constituents, a corresponding abundance value for the abundance of the respective cellular constituent in a biological sample of the first species, into a second latent feature space including the first plurality of dimensions, and generating, for each respective training sample in the second plurality of training samples, a corresponding representation of the corresponding plurality of abundance values in the second latent feature space according to the second mapping function.

Referring to block 428, in some embodiments, the first dimensionality reduction analysis and the second dimensionality reduction analysis are principal component analysis. In some embodiments, the dimensionality reduction analysis is principal component analysis (PCA), non-negative matrix factorization (NMF), kernel principal component analysis (Kernel PCA), graph-based kernel PCA, linear discriminant analysis (LDA), generalized discriminant analysis (GDA), T-distributed stochastic neighbor embedding (t-SNE), or uniform manifold approximation and projection (UMAP).

Referring to block 429, in some embodiments, the method includes learning a third mapping function that maps a representation of a corresponding plurality of abundance values in the first latent feature space to the second latent feature space. Referring to block 430, in some embodiments, the third mapping function is a linear transformation.

Referring to block 432, in some embodiments, the method includes generating, for each respective training sample in the first plurality of training samples, a second corresponding representation of the corresponding plurality of abundance values in the second latent feature space according to the third mapping function.

Referring to block 433, in some embodiments, the method includes inputting, for each respective training sample in the first plurality of training samples, corresponding information about the respective training sample into a multi-task model including a plurality of parameters, where the multi-task model applies the plurality of parameters to the information about the training subject through a plurality of instructions to generate, as output from the multi-task model, a corresponding plurality of outputs, where: the corresponding plurality of outputs includes (i) a predicted effect of the candidate pharmaceutical agent on the respective training sample and (ii) for each respective cell type variable in the set of one or more cell type variables, a corresponding cell type classification for the respective training sample, and the information about the respective training sample includes the second corresponding representation of the corresponding plurality of abundance values in the second latent feature space.

Referring to block 434, in some embodiments, the multi-task model includes a partially connected neural network defining a plurality of tasks, each respective task in the plurality of tasks corresponding to a respective output in the plurality of outputs, where the partially-connected neural network includes (a) a first set of layers shared between the plurality of tasks and (b) for each respective task in the plurality of tasks a corresponding second set of layers unique to the respective task.

Referring to block 436, in some embodiments, the plurality of parameters is at least 1000 parameters, at least 10,000 parameters, at least 100,000 parameters, at least 250,000 parameters, at least 500,000 parameters, at least 1,000,000 parameters, at least 2,500,000 parameters, at least 5,000,000 parameters, at least 10,000,000 parameters, at least 25,000,000 parameters, at least 50,000,000 parameters, at least 100,000,000 parameters, or more parameters.

Referring to block 438, in some embodiments, the plurality of instructions is at least 1000 instructions, at least 10,000 instructions, at least 100,000 instructions, at least 250,000 instructions, at least 500,000 instructions, at least 1,000,000 instructions, at least 2,500,000 instructions, at least 5,000,000 instructions, at least 10,000,000 instructions, at least 25,000,000 instructions, at least 50,000,000 instructions, at least 100,000,000 instructions, or more instructions.

Referring to block 438, in some embodiments, the method includes inputting, for each respective training sample in the second plurality of training samples, corresponding information about the respective training sample into the multi-task model, where the information about the respective training sample includes the corresponding representation of the corresponding plurality of abundance values in the second latent feature space.

Referring to block 440, in some embodiments, the method includes adjusting the plurality of parameters based on: for each respective training sample in the first plurality of training samples, one or more differences between (i) the corresponding plurality of outputs and (ii) a set of labels including (a) the corresponding experimentally measured effect of the candidate pharmaceutical agent on the respective training sample and (b) the corresponding cell type classification for each respective cell type variable in the set of one or more cell type variables, and for each respective training sample in the second plurality of training samples, one or more differences between (i) the corresponding plurality of outputs and (ii) a set of labels including the corresponding cell type classification for each respective cell type variable in the set of one or more cell type variables.

Figures 5A-5D illustrate an example schema for training a multi-task model as described herein. Briefly, as illustrated in Figure 5A, data sets from a plurality of tumor organoids (502-1, ...., 502-n) and data sets from a plurality of human subjects (504-1, ..., 504-m) contain abundance values (X) for a plurality of cellular constituents (1, ..., J). In some embodiments, the linear transform(s) is learned using data from matched tumor organoid tissues and human tissues, e.g., a training set of tumor organoids and a training set of human tissues with similar cellular characteristics across the respective training sets. For example, a tumor organoid training set with respective percentages of a first set of cancer types and a human tissue training set having a substantially similar percentages of the first set of cancer types. In addition to cancer types, the training sets can be matched according any number of confounders, as described herein. The training sets used to learn the linear transform are not necessarily the same training sets that are used to train the multi-task model. For example, in some embodiments, the training sets used to train the transform(s) do not include tumor organoids that have been perturbed with a candidate pharmaceutical agent, while training sets used to train the multi-task model do include tumor organoids that have been perturbed with a candidate pharmaceutical agent.

In some embodiments, the cellular constituents are mRNAs and the abundance values represent relative or absolute gene expression levels. However, the disclosure is not limited to the use of mRNA as cellular constituents. In some embodiments, one or more cellular constituent is a protein species, a metabolite, a lipid, an miRNA, and the like.

A dimensionality reduction technique is used to transform the tumor organoid data sets (502) and the human data sets (504) into a lower dimensionality representations in respective latent spaces 506 and 508, respectively. Joint learning is used to learn a transform, e.g., a linear transform, between the tumor organoid latent space 506 and the human latent space 508.

While Figure 5A illustrates an embodiment where the linear transform is applied to the tumor organoid representations to bring them into the human latent space 508, the opposite could also be used. That is, a linear transform could be learned to transform the human representations into the tumor organoid latent space. Similarly, separate linear transforms could be learned from each of the tumor organoid latent space 506 and human latent space 508 into a third latent space. The key, in each of these embodiments, is that the tumor organoid data and the human data are present in the same latent space, such that model training performed on the more easily obtainable tumor organoid data can be used to train a predictive model for the effects of drugs on humans.

Application of the learned linear transform(s) results in tumor organoid data that has been transformed into the same latent space as the human data (e.g., into the human latent space, as illustrated in Figure 5B, although other latent spaces may be used). The transformed tumor organoid data can then be used, with or without the corresponding human data, to train a multi-task model, with multiple outputs, as illustrated in Figure 5C. In some embodiments, both the tumor organoid embeddings and the human embeddings are used for training. In some embodiments, only the tumor organoid embeddings are used for training. Supervised or semi-supervised training may be used to train the multi-task model. As labels for some of the outputs may not be available for all samples, and particularly for the drug sensitivities of human training samples, semi-supervised learning may be used to account for missing labels. Back propagation can be used to refine the model. In some embodiments, as illustrated in Figure 5D, back propagation is applied to both the model and the transform(s). In other embodiments, various components of the overall model are separately trained. For example, in some embodiments, the multi-task model and the transform(s) are learned separately.

The resulting model, which was trained using tumor organoid response data to candidate pharmaceutical agents, which is much easier to obtain than human response data, can then be applied to human test subjects to predict their response to a candidate pharmaceutical agent, as illustrated in Figure 6.

In some embodiments, the multi-task model is trained to predict sensitivity to a single drug. In other embodiments, the multi-task model is trained to predict sensitivity to a plurality of drugs. For example, in some embodiments, the multi-task model predicts drug sensitivity to at least 2 drugs, at least 3 drugs, at least 4 drugs, at least 5 drugs, at least 6 drugs, at least 7 drugs, at least 8 drugs, at least 9 drugs, at least 10 drugs, at least 15 drugs, at least 20 drugs, at least 25 drugs, or more.

In some embodiments, the multi-task model is trained to predict sensitivity to a class of drugs, instead or in addition to predicting sensitivity to one or more particular drugs. In some embodiments, the multi-task model is trained to predict sensitivity to a single class of drugs. In other embodiments, the multi-task model is trained to predict sensitivity to a plurality of classes of drugs. For example, in some embodiments, the multi-task model predicts drug sensitivity to at least 2 drug classes, at least 3 drug classes, at least 4 drug classes, at least 5 drug classes, at least 6 drug classes, at least 7 drug classes, at least 8 drug classes, at least 9 drug classes, at least 10 drug classes, at least 15 drug classes, at least 20 drug classes, at least 25 drug classes, or more.

Accordingly, in some embodiments, a multi-task model as described herein is used to determine whether a test subject will be sensitive to a drug. Similarly, in some embodiments, a multi-task model as described herein is used to determine whether a test subject will be sensitive to a class of drugs. Likewise, in some embodiments, a multi-task model as described herein is used to determine whether a test subject will be resistant to a drug. Similarly, in some embodiments, a multi-task model as described herein is used to determine whether a test subject will be resistant to a class of drugs.

In some embodiments, as illustrated in Figure 7, a multi-task model as described herein is used to match a tumor organoid strain to a test subject. Tumor organoid culture has the potential to significantly improve precision oncology. By culturing tumor cells from a test subject, various drugs and combinations thereof can be tested on the organoids that closely resemble the cancer in the subject. In this fashion, improved treatment regimens can be identified for individual patients. However, culturing tumor organoids from primary cells from the subject takes significant time, delaying treatment of the patient. As the timing of treatment can play a significant role in patient outcomes, culturing organoids for each patient may hinder patient outcome.

However, if patients could be matched to existing tumor organoid culture lines and/or data collected from previous tumor organoids, a clinician may be able to derive important response data from a surrogate tumor organoid culture without slowing down treatment. Accordingly, in some embodiments, a multi-task model as described herein can be used to match a test patient to a pre-existing tumor organoid line and/or data generated for a previous tumor organoid culture. In some embodiments, as illustrated in Figures 7A-7B, this is accomplished by inputting data from the test subject (e.g., gene expression data 704) into the model and extracting a hidden layer (e.g., hidden layer 722) from the model and comparing the extracted hidden layer to hidden layers generated by inputting data from previous tumor organoid cultures into the same model (e.g., hidden layers 724-1, ..., 724-p). In some embodiments, as illustrated in Figure 7B, the comparing is done by clustering the hidden layers and identifying which hidden layers (e.g., hidden layers 724-1, 724-2, and 724-3) cluster closest to the hidden layer 722 from the test subject.

One of skill in the art will appreciate that any of a wide array of different computer topologies are used for the application and all such topologies are within the scope of the present disclosure.

The systems and methods disclosed herein are further illustrated by the following non-limiting examples.

### Digital and Laboratory Health Care Platform:

The methods and systems described above may be utilized in combination with or as part of a digital and laboratory health care platform that is generally targeted to medical care and research. It should be understood that many uses of the methods and systems described above, in combination with such a platform, are possible. One example of such a platform is described in U.S. Patent Publication No. 2021/0090694, titled "Data Based Cancer Research and Treatment Systems and Methods", and published March 25, 2021, which is incorporated herein by reference and in its entirety for any and all purposes.

For example, an implementation of one or more embodiments of the methods and systems as described above may include microservices constituting a digital and laboratory health care platform supporting prediction of a therapeutic response or identification of a matching tissue organoid. Embodiments may include a single microservice for executing and delivering abundance analysis of mRNA sequencing data or may include a plurality of microservices each having a particular role which together implement one or more of the embodiments above. In one example, a first microservice may execute mRNA sequencing in order to deliver mRNA sequencing data to a second microservice for abundance analysis of mRNA sequencing data. Similarly, the second microservice may execute mRNA sequencing to deliver abundance analysis of mRNA sequencing data, according to an embodiment above.

Where embodiments above are executed in one or more micro-services with or as part of a digital and laboratory health care platform, one or more of such micro-services may be part of an order management system that orchestrates the sequence of events as needed at the appropriate time and in the appropriate order necessary to instantiate embodiments above. A micro-services based order management system is disclosed, for example, in U.S. Patent Publication No. 2020/80365232, titled "Adaptive Order Fulfillment and Tracking Methods and Systems", and published November 19, 2020, which is incorporated herein by reference and in its entirety for all purposes.

For example, continuing with the above first and second microservices, an order management system may notify the first microservice that an order for mRNA sequencing has been received and is ready for processing. The first microservice may execute and notify the order management system once the delivery of mRNA sequencing data is ready for the second microservice. Furthermore, the order management system may identify that execution parameters (prerequisites) for the second microservice are satisfied, including that the first microservice has completed, and notify the second microservice that it may continue processing the order to analyze abundance of mRNA in the sequencing data, according to an embodiment above.

Where the digital and laboratory health care platform further includes a genetic analyzer system, the genetic analyzer system may include targeted panels and/or sequencing probes. An example of a targeted panel is disclosed, for example, in U.S. Patent Publication No. 2021/0090694, titled "Data Based Cancer Research and Treatment Systems and Methods", and published March 25, 2021, which is incorporated herein by reference and in its entirety for all purposes. An example of a targeted panel for sequencing cell-free (cf) DNA and determining various characteristics of a specimen based on the sequencing is disclosed, for example, in U.S. Patent Publication No. 2021/0343372, titled "Methods And Systems For Dynamic Variant Thresholding In A Liquid Biopsy Assay", and published November 4, 2021, U.S. Patent Publication No. 2021/0257055, titled "Estimation Of Circulating Tumor Fraction Using Off-Target Reads Of Targeted-Panel Sequencing", published August 19, 2021, and issued as U.S. Patent No. 11,211,147, and U.S. Patent Publication No. 2021/0257047, titled "Methods And Systems For Refining Copy Number Variation In A Liquid Biopsy Assay", published August 19, 2021, and issued as U.S. Patent No. 11,211,144, which are each incorporated herein by reference and in their entireties for all purposes. In one example, targeted panels may enable the delivery of next generation sequencing results (including sequencing of DNA and/or RNA from solid or cell-free specimens) for splicing analysis of mRNA sequencing data according to an embodiment, above. An example of the design of next-generation sequencing probes is disclosed, for example, in U.S. Patent Publication No. 2021/0115511, titled "Systems and Methods for Next Generation Sequencing Uniform Probe Design", and published June 22, 2021, and U.S. Patent Publication No. 2021/0269878, titled "Systems and Methods for Next Generation Sequencing Uniform Probe Design", and published September 2, 2021, which are each incorporated herein by reference and in their entireties for all purposes.

Where the digital and laboratory health care platform further includes an epigenetic analyzer system, the epigenetic analyzer system may analyze specimens to determine their epigenetic characteristics and may further use that information for monitoring a patient over time. An example of an epigenetic analyzer system is disclosed, for example, in U.S. Patent Publication No. 2021/0398617, titled "Molecular Response And Progression Detection From Circulating Cell Free DNA", and published December 23, 2021, which is incorporated herein by reference and in its entirety for all purposes.

Where the digital and laboratory health care platform further includes a bioinformatics pipeline, the methods and systems described above may be utilized after completion or substantial completion of the systems and methods utilized in the bioinformatics pipeline. As one example, the bioinformatics pipeline may receive next-generation genetic sequencing results and return a set of binary files, such as one or more BAM files, reflecting DNA and/or RNA read counts aligned to a reference genome. The methods and systems described above may be utilized, for example, to ingest the DNA and/or RNA read counts and produce abundance values for mRNA in sequencing data as a result.

When the digital and laboratory health care platform further includes an RNA data normalizer, any RNA read counts may be normalized before processing embodiments as described above. An example of an RNA data normalizer is disclosed, for example, in U.S. Patent Publication No. 2020/0098448, titled "Methods of Normalizing and Correcting RNA Expression Data", and published March 26, 2020, which is incorporated herein by reference and in its entirety for all purposes.

When the digital and laboratory health care platform further includes a genetic data deconvolver, any system and method for deconvolving may be utilized for analyzing genetic data associated with a specimen having two or more biological components to determine the contribution of each component to the genetic data and/or determine what genetic data would be associated with any component of the specimen if it were purified. An example of a genetic data deconvolver is disclosed, for example, in U.S. Patent Publication No. 2020/0210852, published July 2, 2020, and PCT/US19/69161, filed December 31, 2019, both titled "Transcriptome Deconvolution of Metastatic Tissue Samples"; and U.S. Patent Publication No. 2021/0118526, titled "Calculating Cell-type RNA Profiles for Diagnosis and Treatment", and published April 22, 2021, the contents of each of which are incorporated herein by reference and in their entireties for all purposes.

RNA expression levels may be adjusted to be expressed as a value relative to a reference expression level. Furthermore, multiple RNA expression data sets may be adjusted, prepared, and/or combined for analysis and may be adjusted to avoid artifacts caused when the data sets have differences because they have not been generated by using the same methods, equipment, and/or reagents. An example of RNA data set adjustment, preparation, and/or combination is disclosed, for example, in U.S. Patent Publication No. 2022/0059190, titled "Systems and Methods for Homogenization of Disparate Datasets", and published February 24, 2022, which is incorporated herein by reference and in its entirety for all purposes.

When the digital and laboratory health care platform further includes an automated RNA expression caller, RNA expression levels associated with multiple samples may be compared to determine whether an artifact is causing anomalies in the data. An example of an automated RNA expression caller is disclosed, for example, in U.S. Patent No. 11,043,283, titled "Systems and Methods for Automating RNA Expression Calls in a Cancer Prediction Pipeline", and issued June 22, 2021, which is incorporated herein by reference and in its entirety for all purposes.

The digital and laboratory health care platform may further include one or more insight engines to deliver information, characteristics, or determinations related to a disease state that may be based on genetic and/or clinical data associated with a patient, specimen and/or organoid. Exemplary insight engines may include a tumor of unknown origin (tumor origin) engine, a human leukocyte antigen (HLA) loss of homozygosity (LOH) engine, a tumor mutational burden engine, a PD-L1 status engine, a homologous recombination deficiency engine, a cellular pathway activation report engine, an immune infiltration engine, a microsatellite instability engine, a pathogen infection status engine, a T cell receptor or B cell receptor profiling engine, a line of therapy engine, a metastatic prediction engine, an IO progression risk prediction engine, and so forth.

An example tumor origin or tumor of unknown origin engine is disclosed, for example, in U.S. Patent Publication No. 2020/0365268, titled "Systems and Methods for Multi-Label Cancer Classification", and published November 19, 2020, which is incorporated herein by reference and in its entirety for all purposes.

An example of an HLA LOH engine is disclosed, for example, in U.S. Patent No. 11,081,210, titled "Detection of Human Leukocyte Antigen Class I Loss of Heterozygosity in Solid Tumor Types by NGS DNA Sequencing", and issued August 3, 2021, which is incorporated herein by reference and in its entirety for all purposes. An additional example of an HLA LOH engine is disclosed, for example, in U.S. Patent Publication No. 2021/0327536, titled "Detection of Human Leukocyte Antigen Loss of Heterozygosity", and published October 21, 2021, which is incorporated herein by reference and in its entirety for all purposes.

An example of a tumor mutational burden (TMB) engine is disclosed, for example, in U.S. Patent Publication No. 2020/0258601, titled "Targeted-Panel Tumor Mutational Burden Calculation Systems and Methods", and published August 13, 2020, which is incorporated herein by reference and in its entirety for all purposes.

An example of a PD-L1 status engine is disclosed, for example, in U.S. Patent Publication No. 2020/0395097, titled "A Pan-Cancer Model to Predict The PD-L1 Status of a Cancer Cell Sample Using RNA Expression Data and Other Patient Data", and published December 17, 2020, which is incorporated herein by reference and in its entirety for all purposes. An additional example of a PD-L1 status engine is disclosed, for example, in U.S. Patent No. 10,957,041, titled "Determining Biomarkers from Histopathology Slide Images", issued March 23, 2021, which is incorporated herein by reference and in its entirety for all purposes.

An example of a homologous recombination deficiency engine is disclosed, for example, in U.S. Patent No. 10,975,445, titled "An Integrative Machine-Learning Framework to Predict Homologous Recombination Deficiency", and issued April 13, 2021, which is incorporated herein by reference and in its entirety for all purposes. An additional example of a homologous recombination deficiency engine is disclosed, for example, in U.S. Patent No. 11,164,655, titled "Systems and Methods for Predicting Homologous Recombination Deficiency Status of a Specimen", and issued November 2, 2021, which is incorporated herein by reference and in its entirety for all purposes.

An example of a cellular pathway activation report engine is disclosed, for example, in U.S. Patent Publication No. 2021/0057042, titled "Systems And Methods For Detecting Cellular Pathway Dysregulation In Cancer Specimens", and published February 25, 2021, which is incorporated herein by reference and in its entirety for all purposes.

An example of an immune infiltration engine is disclosed, for example, in U.S. Patent Publication No. 2020/0075169, titled "A Multi-Modal Approach to Predicting Immune Infiltration Based on Integrated RNA Expression and Imaging Features", and published March 5, 2020, which is incorporated herein by reference and in its entirety for all purposes.

An example of an MSI engine is disclosed, for example, in U.S. Patent Publication No. 2020/0118644, titled "Microsatellite Instability Determination System and Related Methods", and published April 16, 2020, which is incorporated herein by reference and in its entirety for all purposes. An additional example of an MSI engine is disclosed, for example, in U.S. Patent Publication No. 2021/0098078, titled "Systems and Methods for Detecting Microsatellite Instability of a Cancer Using a Liquid Biopsy", and published April 1, 2021, which is incorporated herein by reference and in its entirety for all purposes.

An example of a pathogen infection status engine is disclosed, for example, in U.S. Patent No. 11,043,304, titled "Systems And Methods For Using Sequencing Data For Pathogen Detection", and issued June 22, 2021, which is incorporated herein by reference and in its entirety for all purposes. Another example of a pathogen infection status engine is disclosed, for example, in WO 2021/168143, titled "Systems And Methods For Detecting Viral DNA From Sequencing", and filed February 18, 2021, which is incorporated herein by reference and in its entirety for all purposes.

An example of a T cell receptor or B cell receptor profiling engine is disclosed, for example, in U.S. Patent No. 11,414,700, titled "TCR/BCR Profiling Using Enrichment with Pools of Capture Probes", and issued November 18, 2021, which is incorporated herein by reference and in its entirety for all purposes.

An example of a line of therapy engine is disclosed, for example, in U.S. Patent Publication No. 2021/0057071, titled "Unsupervised Learning And Prediction Of Lines Of Therapy From High-Dimensional Longitudinal Medications Data", and published February 25, 2021, which is incorporated herein by reference and in its entirety for all purposes.

An example of a metastatic prediction engine is disclosed, for example, in U.S. Patent No. 11,145,416, titled "Predicting likelihood and site of metastasis from patient records", and issued October 12, 2021, which is incorporated herein by reference and in its entirety for all purposes.

An example of an IO progression risk prediction engine is disclosed, for example, in U.S. Patent Publication No. 2022/0154284, titled "Determination of Cytotoxic Gene Signature and Associated Systems and Methods For Response Prediction and Treatment", and published May 19, 2022, which is incorporated herein by reference and in its entirety for all purposes.

When the digital and laboratory health care platform further includes a report generation engine, the methods and systems described above may be utilized to create a summary report of a patient's genetic profile and the results of one or more insight engines for presentation to a physician. For instance, the report may provide to the physician information about the extent to which the specimen that was sequenced contained tumor or normal tissue from a first organ, a second organ, a third organ, and so forth. For example, the report may provide a genetic profile for each of the tissue types, tumors, or organs in the specimen. The genetic profile may represent genetic sequences present in the tissue type, tumor, or organ and may include variants, expression levels, information about gene products, or other information that could be derived from genetic analysis of a tissue, tumor, or organ.

The report may include therapies and/or clinical trials matched based on a portion or all of the genetic profile or insight engine findings and summaries. For example, the therapies may be matched according to the systems and methods disclosed in U.S. Patent Publication No. 2022/0208305, titled "Artificial Intelligence Driven Therapy Curation and Prioritization", and published June 30, 2022, which is incorporated herein by reference and in its entirety for all purposes. For example, the clinical trials may be matched according to the systems and methods disclosed in U.S. Patent Publication No. 2020/0381087, titled "Systems and Methods of Clinical Trial Evaluation", published December 3, 2020, which is incorporated herein by reference and in its entirety for all purposes.

The report may include a comparison of the results (for example, molecular and/or clinical patient data) to a database of results from many specimens. An example of methods and systems for comparing results to a database of results are disclosed in U.S. Patent Publication No. 2020/0135303 titled "User Interface, System, And Method For Cohort Analysis" and published April 30, 2020, and U.S. Patent Publication No. 2020/0211716 titled "A Method and Process for Predicting and Analyzing Patient Cohort Response, Progression and Survival", and published July 2, 2020, which is incorporated herein by reference and in its entirety for all purposes. The information may be used, sometimes in conjunction with similar information from additional specimens and/or clinical response information, to match therapies likely to be successful in treating a patient, discover biomarkers or design a clinical trial.

Any data generated by the systems and methods and/or the digital and laboratory health care platform may be downloaded by the user. In one example, the data may be downloaded as a CSV file comprising clinical and/or molecular data associated with tests, data structuring, and/or other services ordered by the user. In various embodiments, this may be accomplished by aggregating clinical data in a system backend and making it available via a portal. This data may include not only variants and RNA expression data, but also data associated with immunotherapy markers such as MSI and TMB, as well as RNA fusions.

When the digital and laboratory health care platform further includes a device comprising a microphone and speaker for receiving audible queries or instructions from a user and delivering answers or other information, the methods and systems described above may be utilized to add data to a database the device can access. An example of such a device is disclosed, for example, in U.S. Patent Publication No. 2020/0335102, titled "Collaborative Artificial Intelligence Method And System", and published October 22, 2020, which is incorporated herein by reference and in its entirety for all purposes.

When the digital and laboratory health care platform further includes a mobile application for ingesting patient records, including genomic sequencing records and/or results even if they were not generated by the same digital and laboratory health care platform, the methods and systems described above may be utilized to receive ingested patient records. An example of such a mobile application is disclosed, for example, in U.S. Patent No. 10,395,772, titled "Mobile Supplementation, Extraction, And Analysis Of Health Records", and issued August 27, 2019, which is incorporated herein by reference and in its entirety for all purposes. Another example of such a mobile application is disclosed, for example, in U.S. Patent No. 10,902,952, titled "Mobile Supplementation, Extraction, And Analysis Of Health Records", and issued January 26, 2021, which is incorporated herein by reference and in its entirety for all purposes. Another example of such a mobile application is disclosed, for example, in U.S. Patent Publication No. 2021/0151192, titled "Mobile Supplementation, Extraction, And Analysis Of Health Records", and filed May 20, 2021, which is incorporated herein by reference and in its entirety for all purposes.

When the digital and laboratory health care platform further includes organoids developed in connection with the platform (for example, from the patient specimen), the methods and systems may be used to further evaluate genetic sequencing data derived from an organoid and/or the organoid sensitivity, especially to therapies matched based on a portion or all of the information determined by the systems and methods, including predicted cancer type(s), likely tumor origin(s), etc. These therapies may be tested on the organoid, derivatives of that organoid, and/or similar organoids to determine an organoid's sensitivity to those therapies. Any of the results may be included in a report. If the organoid is associated with a patient specimen, any of the results may be included in a report associated with that patient and/or delivered to the patient or patient's physician or clinician. In various examples, organoids may be cultured and tested according to the systems and methods disclosed in U.S. Patent Publication No. 2021/0155989, titled "Tumor Organoid Culture Compositions, Systems, and Methods", published May 27, 2021; WO2021081253, titled "Systems and Methods for Predicting Therapeutic Sensitivity", published April 29, 2021; U.S. Patent Publication No. 2021/0172931, titled "Large Scale Organoid Analysis", published June 10, 2021; WO 2021/113821, titled "Systems and Methods for High Throughput Drug Screening", and published June 10, 2021, and U.S. Patent Publication No. 2021/0325308, titled "Artificial Fluorescent Image Systems and Methods", and published October 21, 2021, which are each incorporated herein by reference and in their entirety for all purposes. In one example, the drug sensitivity assays may be especially informative if the systems and methods return results that match with a variety of therapies, or multiple results (for example, multiple equally or similarly likely cancer types or tumor origins), each matching with at least one therapy.

When the digital and laboratory health care platform further includes application of one or more of the above in combination with or as part of a medical device or a laboratory developed test that is generally targeted to medical care and research, such laboratory developed test or medical device results may be enhanced and personalized through the use of artificial intelligence. An example of laboratory developed tests, especially those that may be enhanced by artificial intelligence, is disclosed, for example, in U.S. Patent Publication No. 2021/0118559, titled "Artificial Intelligence Assisted Precision Medicine Enhancements to Standardized Laboratory Diagnostic Testing", and published April 22, 2021, which is incorporated herein by reference and in its entirety for all purposes.

### Example Embodiments

Embodiment 1 - A method for predicting an effect of a pharmaceutical agent in a test subject of a first species, the method comprising: at a computer system comprising at least one processor and a memory storing at least one program for execution by the at least one processor: inputting information about the test subject into a multi-task model comprising a plurality of parameters, wherein the multi-task model applies the plurality of parameters to the information about the test subject through a plurality of instructions to generate, as output from the multi-task model, a plurality of outputs comprising (i) a predicted effect of the pharmaceutical agent in the test subject and (ii) for each respective cell type variable in a set of one or more cell type variables, a corresponding cell type classification, wherein the information about the test subject comprises a plurality of abundance values, the plurality of abundance values comprising, for each respective cellular constituent in a plurality of cellular constituents, a corresponding abundance value for the abundance of the respective cellular constituent in a biological sample of the test subject.

Embodiment 2 - The method of embodiment 1, wherein the predicted effect comprises a prediction for cell death of a cancer cell in the subject in response to administration of the pharmaceutical agent to the subject.

Embodiment 3 - The method of embodiment 1 or 2, wherein the pharmaceutical agent is a chemotherapeutic agent.

Embodiment 4 - The method of embodiment 1 or 2, wherein the pharmaceutical agent is selected from the group consisting of lenalidomid, pembrolizumab, trastuzumab, bevacizumab, rituximab, ibrutinib, human papillomavirus quadrivalent (types 6, 11, 16, and 18) vaccine, pertuzumab, pemetrexed, nilotinib, denosumab, abiraterone acetate, promacta, imatinib, everolimus, palbociclib, erlotinib, bortezomib, bortezomib, nivolumab, atezolizumab, daratumumab, enzalutamide, obinutuzumab, ruxolitinib, venetoclax, osimertinib, and pomalidomide.

Embodiment 5 - The method of any one of embodiments 1-4, wherein the multi-task model comprises a partially connected neural network defining a plurality of tasks, each respective task in the plurality of tasks corresponding to a respective output in the plurality of outputs, wherein the partially-connected neural network comprises (a) a first set of layers shared between the plurality of tasks and (b) for each respective task in the plurality of tasks a corresponding second set of layers unique to the respective task.

Embodiment 6 - The method of any one of embodiments 1-5, the method further comprising: when the predicted effect of the pharmaceutical agent in the test subject satisfies a first set of one or more criterion, recommending a first therapy that comprises administration of the pharmaceutical agent to the subject; and when the predicted effect of the pharmaceutical agent in the test subject does not satisfy the first set of one or more criterion, recommending a second therapy that is different from the first therapy.

Embodiment 7 - The method of any one of embodiments 1-6, the method further comprising: when the predicted effect of the pharmaceutical agent in the test subject satisfies a first set of one or more criterion, administering a first therapy to the subject, wherein the first therapy that comprises administration of the pharmaceutical agent; and when the predicted effect of the pharmaceutical agent in the test subject does not satisfy the first set of one or more criterion, administering a second therapy to the subject, wherein the second therapy is different from the first therapy.

Embodiment 8 - The method of any one of embodiments 1-4, the method further comprising: when the predicted effect of the pharmaceutical agent in the test subject satisfies a first set of one or more criterion, recommending the test subject for a clinical trial of the pharmaceutical agent to the subject; and when the predicted effect of the pharmaceutical agent in the test subject does not satisfy the first set of one or more criterion, not recommending the test subject for the clinical trial.

Embodiment 9 - A method for identifying one or more tissue organoids in a plurality of tissue organoids matching a biological property of a tissue in a subject, the method comprising: at a computer system comprising at least one processor and a memory storing at least one program for execution by the at least one processor: inputting information about the test subject into a multi-task model comprising a plurality of parameters and one or more hidden layers, wherein the multi-task model is trained to apply the plurality of parameters to the information about the test subject through a plurality of instructions to generate, as output from the multi-task model, a plurality of outputs comprising (i) a predicted effect of the pharmaceutical agent in the test subject and (ii) for each respective cell type variable in a set of one or more cell type variables, a corresponding cell type classification, wherein the information about the test subject comprises, for each respective cellular constituent in a plurality of cellular constituents, a corresponding abundance value for the abundance of the respective cellular constituent in a biological sample of the test subject; obtaining a latent representation of the information about the test subject from a respective hidden layer in the one or more hidden layers; and comparing the latent representation of the information about the test subject to a plurality of latent representations, wherein each respective latent representation in the plurality of latent representations is of information about a respective tissue organoid, in a plurality of tissue organoids, obtained from the multi-task model; and identifying one or more respective tissue organoids, in the plurality of tissue organoids, that satisfy a set of one or more similarity criterion based on the comparing, thereby identifying the one or more tissue organoids matching the biological property of the tissue in the subject.

Embodiment 10 - The method of embodiment 9, wherein the multi-task model comprises a partially connected neural network defining a plurality of tasks, each respective task in the plurality of tasks corresponding to a respective output in the plurality of outputs, wherein the partially-connected neural network comprises (a) a first set of layers shared between the plurality of tasks and (b) for each respective task in the plurality of tasks a corresponding second set of layers unique to the respective task.

Embodiment 11 - The method of embodiment 10, wherein the respective hidden layer is a respective layer in the first set of layers shared between the plurality of tasks.

Embodiment 12 - The method of embodiment 10, wherein the respective hidden layer is a respective layer in a corresponding second set of layers unique to a respective task in the plurality of tasks.

Embodiment 13 - The method of embodiment 12, wherein the output corresponding to the respective task is the predicted effect of the pharmaceutical agent in the test subject.

Embodiment 14 - The method of any one of embodiments 9-13, wherein comparing the latent representation of the information about the test subject to the plurality of latent representations comprises clustering the latent representation of the information about the test subject with the plurality of latent representations.

Embodiment 15 - The method of embodiment 14, wherein: the clustering generates a set of clusters including a first respective cluster comprising the latent representation of the information about the test subject; and the set of one or more similarity criterion comprises a criteria that the latent representation of information about the respective tissue organoid is in the first respective cluster.

Embodiment 16 - The method of any one of embodiments 9-15, wherein: comparing the latent representation of the information about the test subject to the plurality of latent representations comprises determining, for each respective tissue organoid in at least a subset of the plurality of tissue organoids, a corresponding geometric distance between the latent representation of the information about the test subject and the corresponding latent representation of the information about the respective tissue organoid; and the set of one or more similarity criterion comprises a distance criteria.

Embodiment 17 - The method of embodiment 16, wherein the distance criteria is that the latent representation of information about the respective tissue organoid is within a threshold geometric distance of the latent representation of the information about the test subj ect.

Embodiment 18 - The method of embodiment 16, wherein the distance criteria is that the latent representation of information about the respective tissue organoid is within a threshold number of closest latent representations, in the plurality of latent representations, to the latent representation of the information about the test subject.

Embodiment 19 - The method of embodiment 16, wherein the distance criteria is that the latent representation of information about the respective tissue organoid is the closest latent representation, in the plurality of latent representations, to the latent representation of the information about the test subject.

Embodiment 20 - The method of any one of embodiments 1-19, wherein the plurality of tissue organoids comprises a plurality of tumor organoids.

Embodiment 21 - The method of any one of embodiments 1-20, wherein the multi-task model comprises a linear mapping function that transforms the plurality of abundance values into a first latent feature space comprising fewer dimensions than the number of respective cellular constituents in the plurality of cellular constituents.

Embodiment 22 - The method of any one of embodiments 1-21, wherein the plurality of parameters is at least 1000 parameters, at least 10,000 parameters, at least 100,000 parameters, at least 250,000 parameters, at least 500,000 parameters, at least 1,000,000 parameters, at least 2,500,000 parameters, at least 5,000,000 parameters, at least 10,000,000 parameters, at least 25,000,000 parameters, at least 50,000,000 parameters, at least 100,000,000 parameters, or more parameters.

Embodiment 23 - The method of any one of embodiments 1-22, wherein the plurality of instructions is at least 1000 instructions, at least 10,000 instructions, at least 100,000 instructions, at least 250,000 instructions, at least 500,000 instructions, at least 1,000,000 instructions, at least 2,500,000 instructions, at least 5,000,000 instructions, at least 10,000,000 instructions, at least 25,000,000 instructions, at least 50,000,000 instructions, at least 100,000,000 instructions, or more instructions.

Embodiment 24 - The method of any one of embodiments 1-23, wherein the set of one or more cell type variables comprises a variable selected from the group consisting of cell histology, disease type, disease stage, disease grade, tissue type, and tissue site.

Embodiment 25 - The method of any one of embodiments 1-24, wherein the plurality of cellular constituents is at least 10 cellular constituents, at least 25 cellular constituents, at least 50 cellular constituents, at least 100 cellular constituents, at least 250 cellular constituents, at least 500 cellular constituents, at least 1000 cellular constituents, at least 2500 cellular constituents, at least 5000 cellular constituents, at least 10,000 cellular constituents, at least 20,000 cellular constituents, or more cellular constituents.

Embodiment 26 - The method of any one of embodiments 1-25, wherein each respective cellular constituent in the plurality of cellular constituents is a different mRNA species.

Embodiment 27 - The method of embodiment 26, further comprising: (i) obtaining, in electronic form, a plurality of nucleic acid sequences for mRNA from the biological sample of the test subject; and (ii) determining, for each respective cellular constituent in the plurality of cellular constituents, the corresponding abundance value from the plurality of nucleic acid sequences.

Embodiment 28 - The method of embodiment 27, wherein the obtaining (i) comprises sequencing the mRNA from the biological sample of the test subject, thereby obtaining the plurality of nucleic acid sequences.

Embodiment 29 - The method of embodiment 27 or 28, wherein the plurality of nucleic acid sequences is at least 1000 nucleic acid sequences, at least 10,000 nucleic acid sequences, at least 100,000 nucleic acid sequences, at least 250,000 nucleic acid sequences, at least 500,000 nucleic acid sequences, at least 1,000,000 nucleic acid sequences, or more nucleic acid sequences.

Embodiment 30 - The method of any one of embodiments 1-29, wherein the biological sample of the subject comprises a diseased tissue of the subject.

Embodiment 31 - The method of embodiment 30, wherein the diseased tissue of the subject is a cancerous tissue.

Embodiment 32 - The method of any one of embodiments 1-29, wherein the biological sample of the subject comprises a biological fluid from the subject.

Embodiment 33 - The method of any one of embodiments 1-32, wherein the subject has a cancer selected from the group consisting of a carcinoma, lymphoma, blastoma, glioblastoma, sarcoma, leukemia, breast cancer, squamous cell cancer, lung cancer, small-cell lung cancer, non-small cell lung cancer (NSCLC), adenocarcinoma of the lung, squamous carcinoma of the lung, head and neck cancer, cancer of the peritoneum, hepatocellular cancer, gastric or stomach cancer, pancreatic cancer, ovarian cancer, cervical cancer, liver cancer, bladder cancer, hepatoma, colon cancer, colorectal cancer, endometrial or uterine carcinoma, salivary gland carcinoma, kidney or renal cancer, liver cancer, prostate cancer, vulval cancer, thyroid cancer, hepatic carcinoma, B-cell lymphoma, low grade/follicular non-Hodgkin's lymphoma (NHL), small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, Waldenstrom's Macroglobulinemia, chronic lymphocytic leukemia (CLL), acute lymphoblastic leukemia (ALL), hairy cell leukemia, and chronic myeloblastic leukemia.

Embodiment 34 - A method for training a model to predict an effect of a candidate pharmaceutical agent in a test subject of a first species, the method comprising: at a computer system comprising at least one processor and a memory storing at least one program for execution by the at least one processor: A) obtaining, for each respective training sample in a first plurality of training samples, wherein each respective training sample in the first plurality of training samples comprises a tissue organoid or tissue organoid culture, formed from cells of the first species, that has been exposed to a perturbation: (i) a corresponding plurality of abundance values comprising, for each respective cellular constituent in a plurality of cellular constituents, a corresponding abundance value for the abundance of the respective cellular constituent in the respective training sample after exposure to the candidate pharmaceutical agent, (ii) a corresponding experimentally measured effect of the candidate pharmaceutical agent on the respective training sample, and (iii) a corresponding set of one or more cell type classifications comprising, for each respective cell type variable in a set of one or more cell type variables, a corresponding cell type classification for the respective cell type variable; B) obtaining, for each respective training sample in a second plurality of training samples, wherein each respective training sample in the second plurality of training samples comprises a biological sample from a respective subject in a plurality of subjects of the first species: (i) a corresponding plurality of abundance values comprising, for each respective cellular constituent in the plurality of cellular constituents, a corresponding abundance value for the abundance of the respective cellular constituent in the respective training sample, and (ii) a corresponding set of one or more cell type classifications comprising, for each respective cell type variable in the set of one or more cell type variables, a corresponding cell type classification for the respective cell type variable; C) performing a first dimensionality reduction analysis across the corresponding plurality of abundance values for each respective training sample in the first plurality of training samples, thereby: learning a first mapping function that maps a corresponding plurality of abundance values comprising, for each respective cellular constituent in the plurality of cellular constituents, a corresponding abundance value for the abundance of the respective cellular constituent in a tissue organoid or tissue organoid culture, formed from cells of the first species, that has been exposed to a perturbation, into a first latent feature space comprising a first plurality of dimensions that is less than the number of cellular constituents in the plurality of constituents, and generating, for each respective training sample in the first plurality of training samples, a first corresponding representation of the corresponding plurality of abundance values in the first latent feature space according to the first mapping function; D) performing a second dimensionality reduction analysis across the corresponding plurality of abundance values for each respective training sample in the second plurality of training samples, thereby: learning a second mapping function that maps a corresponding plurality of abundance values comprising, for each respective cellular constituent in the plurality of cellular constituents, a corresponding abundance value for the abundance of the respective cellular constituent in a biological sample of the first species, into a second latent feature space comprising the first plurality of dimensions, and generating, for each respective training sample in the second plurality of training samples, a corresponding representation of the corresponding plurality of abundance values in the second latent feature space according to the second mapping function; E) learning a third mapping function that maps a representation of a corresponding plurality of abundance values in the first latent feature space to the second latent feature space; F) generating, for each respective training sample in the first plurality of training samples, a second corresponding representation of the corresponding plurality of abundance values in the second latent feature space according to the third mapping function; G) inputting, for each respective training sample in the first plurality of training samples, corresponding information about the respective training sample into a multi-task model comprising a plurality of parameters, wherein the multi-task model applies the plurality of parameters to the information about the training subject through a plurality of instructions to generate, as output from the multi-task model, a corresponding plurality of outputs, wherein: the corresponding plurality of outputs comprises (i) a predicted effect of the candidate pharmaceutical agent on the respective training sample and (ii) for each respective cell type variable in the set of one or more cell type variables, a corresponding cell type classification for the respective training sample, and the information about the respective training sample comprises the second corresponding representation of the corresponding plurality of abundance values in the second latent feature space; H) inputting, for each respective training sample in the second plurality of training samples, corresponding information about the respective training sample into the multi-task model, wherein the information about the respective training sample comprises the corresponding representation of the corresponding plurality of abundance values in the second latent feature space; and I) adjusting the plurality of parameters based on: for each respective training sample in the first plurality of training samples, one or more differences between (i) the corresponding plurality of outputs and (ii) a set of labels comprising (a) the corresponding experimentally measured effect of the candidate pharmaceutical agent on the respective training sample and (b) the corresponding cell type classification for each respective cell type variable in the set of one or more cell type variables, and for each respective training sample in the second plurality of training samples, one or more differences between (i) the corresponding plurality of outputs and (ii) a set of labels comprising the corresponding cell type classification for each respective cell type variable in the set of one or more cell type variables.

Embodiment 35 - The method of embodiment 34, wherein the pharmaceutical agent is a chemotherapeutic agent.

Embodiment 36 - The method of embodiment 34, wherein the pharmaceutical agent is selected from the group consisting of lenalidomid, pembrolizumab, trastuzumab, bevacizumab, rituximab, ibrutinib, human papillomavirus quadrivalent (types 6, 11, 16, and 18) vaccine, pertuzumab, pemetrexed, nilotinib, denosumab, abiraterone acetate, promacta, imatinib, everolimus, palbociclib, erlotinib, bortezomib, bortezomib, nivolumab, atezolizumab, daratumumab, enzalutamide, obinutuzumab, ruxolitinib, venetoclax, osimertinib, and pomalidomide.

Embodiment 37 - The method of any one of embodiments 34-36, wherein the first plurality of training samples is at least 25 training samples, at least 50 training samples, at least 100 training samples, at least 250 training samples, at least 500 training samples, at least 1000 training samples, at least 2500 training samples, at least 5000 training samples, at least 1000 training samples, or more training samples.

Embodiment 38 - The method of any one of embodiments 34-37, wherein, for a respective training sample in the first plurality of training samples, the corresponding tissue organoid or tissue organoid culture is a tumor organoid or a tumor organoid culture.

Embodiment 39 - The method of any one of embodiments 34-38, wherein the plurality of cellular constituents is at least 10 cellular constituents, at least 25 cellular constituents, at least 50 cellular constituents, at least 100 cellular constituents, at least 250 cellular constituents, at least 500 cellular constituents, at least 1000 cellular constituents, at least 2500 cellular constituents, at least 5000 cellular constituents, at least 10,000 cellular constituents, at least 20,000 cellular constituents, or more cellular constituents.

Embodiment 40 - The method of any one of embodiments 34-39, wherein each respective cellular constituent in the plurality of cellular constituents is a different mRNA species.

Embodiment 41 - The method of any one of embodiments 34-40, wherein the set of one or more cell type variables comprises a variable selected from the group consisting of cell histology, disease type, disease stage, disease grade, tissue type, and tissue site.

Embodiment 42 - The method of any one of embodiments 34-41, wherein, for a respective training sample in the second plurality of training samples, the corresponding biological sample comprises a diseased tissue of the corresponding subject.

Embodiment 43 - The method of embodiment 42, wherein the diseased tissue of the corresponding subject is a cancerous tissue.

Embodiment 44 - The method of any one of embodiments 34-41, wherein, for a respective training sample in the second plurality of training samples, the corresponding biological sample comprises a biological fluid from the subject.

Embodiment 45 - The method of any one of embodiments 34-41, wherein, for a respective training sample in the second plurality of training samples, the corresponding the subject has a cancer selected from the group consisting of a carcinoma, lymphoma, blastoma, glioblastoma, sarcoma, leukemia, breast cancer, squamous cell cancer, lung cancer, small-cell lung cancer, non-small cell lung cancer (NSCLC), adenocarcinoma of the lung, squamous carcinoma of the lung, head and neck cancer, cancer of the peritoneum, hepatocellular cancer, gastric or stomach cancer, pancreatic cancer, ovarian cancer, cervical cancer, liver cancer, bladder cancer, hepatoma, colon cancer, colorectal cancer, endometrial or uterine carcinoma, salivary gland carcinoma, kidney or renal cancer, liver cancer, prostate cancer, vulval cancer, thyroid cancer, hepatic carcinoma, B-cell lymphoma, low grade/follicular non-Hodgkin's lymphoma (NHL), small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, Waldenstrom's Macroglobulinemia, chronic lymphocytic leukemia (CLL), acute lymphoblastic leukemia (ALL), hairy cell leukemia, and chronic myeloblastic leukemia.

Embodiment 46 - The method of any one of embodiments 34-45, wherein the first dimensionality reduction analysis and the second dimensionality reduction analysis are principal component analysis.

Embodiment 47 - The method of any one of embodiments 34-46, wherein the third mapping function is a linear transformation.

Embodiment 48 - The method of any one of embodiments 34-47, wherein the multi-task model comprises a partially connected neural network defining a plurality of tasks, each respective task in the plurality of tasks corresponding to a respective output in the plurality of outputs, wherein the partially-connected neural network comprises (a) a first set of layers shared between the plurality of tasks and (b) for each respective task in the plurality of tasks a corresponding second set of layers unique to the respective task.

Embodiment 49 - The method of any one of embodiments 34-48, wherein the plurality of parameters is at least 1000 parameters, at least 10,000 parameters, at least 100,000 parameters, at least 250,000 parameters, at least 500,000 parameters, at least 1,000,000 parameters, at least 2,500,000 parameters, at least 5,000,000 parameters, at least 10,000,000 parameters, at least 25,000,000 parameters, at least 50,000,000 parameters, at least 100,000,000 parameters, or more parameters.

Embodiment 50 - The method of any one of embodiments 34-49, wherein the plurality of instructions is at least 1000 instructions, at least 10,000 instructions, at least 100,000 instructions, at least 250,000 instructions, at least 500,000 instructions, at least 1,000,000 instructions, at least 2,500,000 instructions, at least 5,000,000 instructions, at least 10,000,000 instructions, at least 25,000,000 instructions, at least 50,000,000 instructions, at least 100,000,000 instructions, or more instructions.

Embodiment 51 - A computer system, comprising: one or more processors; and a non-transitory computer-readable medium including computer-executable instructions that, when executed by the one or more processors, cause the processors to perform the method according to any one of embodiments 1-50.

Embodiment 52 - A non-transitory computer-readable storage medium having stored thereon program code instructions that, when executed by a processor, cause the processor to perform the method according to any one of embodiments 1-50.

It should be understood that the examples provided herein are illustrative and do not limit the uses of the systems and methods described herein in combination with a digital and laboratory health care platform.

### EXAMPLES

### Example 1 - Predicting histology of uterine corpus endometrial carcinoma (UCEC).

It was first investigated whether dimensionally-reduced transcriptional profiles from endometrial tumor organoids and from primary UCEC tissue (tumor biopsies) would cluster together. Briefly, the transcriptional profiles obtained from 61 endometrial tumor organoids (tumor organoids grown from endometrial tumor cells) from two different tumor organoid collections (RS and RS.v2) and from 532 UCEC tumor biopsies in the Cancer Genome Atlas (TCGA) were subject to Uniform Manifold Approximation and Projection (UMAP) dimensionality reduction analysis, the results of which are shown in Figure 8. As shown in Figure 8A, the representations of the transcriptional profiles obtained from the endometrial tumor organoids grouped together and the representations of the transcriptional profiles for the UCEC tumor biopsies grouped together. However, the representations of the transcriptional profiles obtained from the endometrial tumor organoids did not group together with the representations of the transcriptional profiles for the UCEC tumor biopsies. As shown in Figure 8B, within the respective tumor organoid and primary tumor tissue groupings, the UMAP analysis did cluster samples with endometrioid histology together and samples with serous histology together.

To try and correct for technical biases between the organoid and human RNA-expression datasets, and to account for cofounder variables, a multi-task model as described herein was trained. Briefly, the transcriptional profiles obtained from the 61 endometrial tumor organoids were subject to principal component analysis to learn a first set of principal components. The transcriptional profiles obtained from the 532 UCEC tumor biopsies were also subjected to principal component analysis to learn a second set of principal components. A linear function was then learned from the embeddings in a latent space defined by the first set of principal components and the embeddings in a latent space defined by the second set of principal components.

A multi-task neural network having a plurality of tasks including prediction of tumor histology between endometrioid histology and serous histology was then trained against PCA embeddings for the 61 endometrial tumor organoids that had been transformed from the first latent space to the second latent space using the learned linear transformation. The ability of model to predict the histology of the 532 UCEC tissue biopsies (which included 409 endometrioid samples and 114 serous samples) was then determined. Briefly, the PCA embeddings of the 532 UCEC tumor biopsies in the second latent space were then input into the multi-task model to output a predicted histology for each tumor biopsy. As shown in Figure 11A, the sensitivity of the model for predicting the histology of the human tumor biopsies was 0.90 and the specificity was 0.92. As shown in Figure 11B, the mean average precision (AP) of the model for predicting the histology of the human tumor biopsies was 0.87.

As a comparison, a fully connected neural network was trained to predict cell histology using the transcriptional profile for the 61 endometrial tumor organoids. The neural network was then used to predict the histology of the 532 UCEC tissue biopsies. As shown in Figure 10A, the sensitivity of the model for predicting the histology of the human tumor biopsies was 0.89 and the specificity was 0.86. As shown in Figure 10B, the mean average precision (AP) of the model for predicting the histology of the human tumor biopsies was 0.79.

Further, the PCA embeddings for the 61 endometrial tumor organoids transformed into the second latent space and the PCA embeddings for the 532 UCEC tissue biopsies in the second latent space were subject to Uniform Manifold Approximation and Projection (UMAP) dimensionality reduction analysis, the results of which are shown in Figure 9. As shown in Figure 9A, unlike the UMAP representations of the untransformed transcriptional profiles shown in Figure 8, the UMAP representations obtained from the transformed PCA embeddings of the endometrial tumor organoids grouped together with the UMAP representations of the PCA embeddings of the transcriptional profiles for the UCEC tumor biopsies. Further, as shown in Figure 8B, the UMAP analyses clustered both the tumor organoid and the human biopsies by cellular histology.

### Example 2 - Predicting cancer type.

This example describes training of a model that includes a multi-task neural network for predicting, among other tasks, cancer type. Briefly, the model is a semi-supervised end-to-end model that predicts individual patient clinical responses to a drug based on the sensitivity of the tumor organoids to that particular drug. The training and use of the model can be described in three steps: asymmetric domain adaptation, multi-task model training, and inference.

During the domain adaptation stage, a linear transformation is learned between a low-rank organoid subspace and a low-rank patient subspace, such that the resulting low-dimensional organoid embeddings are homogenized with the low-dimensional patient embeddings. The corrected organoid embeddings are used to reconstruct the gene expression profiles of the organoids in the patient embedding space.

During the multi-task model training stage, a semi-supervised neural network model is trained on corrected organoid expression data to predict organoid drug response screen, while modeling for the confounder variables in the organoid expression data (an explicit map is learnt between the expression data and each confounder variable). The correction factors between organoid and human expression data are simultaneously fine-tuned to ensure transferability of the multi-task model from organoid to human expression data.

This strategy ensures that while the organoid and human expression datasets are homogenized via correction of technical biases, each drug-response signature can be transferred in the homogenized space, and the known confounder variables serve to learn robust correction factors and a drug-response predictor. This step ensures the transferability of drug-response mapping from organoids to human expression data, even in the presence of confounders.

During the inference stage, the patient gene expression data is used to predict the patient's response to a drug via the aforementioned neural network model, trained in the multi-task model training stage.

Briefly, a fully-connected neural network is represented as *f^{k}_{W^{k}}*[*xᵢ*] = *f^{k}*₁[*W₁^{k}*[*f*₂*^{k}*[*W*₂*^{k}xᵢ*]]], where *i* = 1, .., *n* samples, *y^{k}ᵢ* is the *i*-th sample and k-th task, *xᵢ* ∈ *R^{p}* is the input with p dimensions, *Wⱼ^{k}* ∈ R^{pⱼ×pⱼ₊₁} are the weight matrices and *fⱼ^{k}*: R^{pⱼ×pⱼ₊₁} are activation functions for *j* = 1,2. Let denote the multi-task loss Σₖ *L*(*y^{k}*, *f^{k}_{W^{k}}* [*x_{sc}*]). *U_{S}* and *Uₜ* represent the source and target bases. A is a linear transformation between source basis and target basis. The corrected source data is *X_{sc}* = *UₜAU^{T}ₛXₛ.* The domain-adaptation loss is denoted as ∥ *U_{S}A* - *Uₜ*∥*_{F}.* The overall objective is *J*(*W, A*) = *min_{W^{k},A}* Σ*ₖ L*(*y^{k} ,f^{k}_{W^{k}}*[*x_{sc}*]) + C₁∥ *U_{S}A* - *Uₜ*∥*_{F}* + *C*₂Σ*ₖ* Σ*ⱼ* ∥*Wⱼ^{k}*∥_{2,1}.

The training includes steps of:

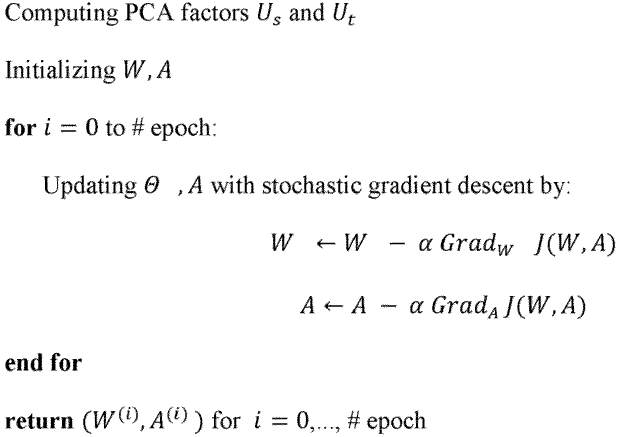

The algorithm was applied to two independent pan-cancer cohorts (2106 TCGA patients and 375 tumor organoids). The TCGA cohort was split into training (631 samples) and validation (1475 samples) sets. A multi-task model, including a task for the prediction of cancer type, was trained on the 375 organoids and the TCGA training samples. Organoids were corrected to TCGA patient samples during training, through a learned linear transformation between latent spaces as described in Example 1.

As shown in Figures 12A and 12B, UMAP analysis of the transcriptional profiles from the pan-cancer validation set and the organoid samples did not cluster the samples together (Figure 12A) and did not cluster similar cancer types together between the two cohorts (Figure 12B). However, when UMAP analysis was applied to embeddings of the pan-cancer validation set and the transformed organoid sample data, from the first layer (Figures 13A-13C), second layer (Figures 14A-14C), and third layer (Figures 15A-15C), the UMAP representations for the pan-cancer validation set and the organoid samples did cluster together (13A, 14A, and 15A), the cancer types clustered together (13B, 14B, and 15B), and the predicted cancer type from the multi-task model clustered together (13C, 14C, and 15C), regardless of the data source.

The performance of the multi-task model on the pan-cancer validation set is further shown in the confusion matrix illustrated in Figure 16A and the precision-recall curve illustrated in Figure 16B.

From the foregoing, it will be appreciated that, although specific embodiments of the systems and methods disclosed herein have been described herein for purposes of illustration, various modifications may be made without deviating from the spirit and scope of the systems and methods disclosed herein. Accordingly, the systems and methods disclosed herein are not limited except as by the appended claims.

## Claims

1. A method for predicting an effect of a pharmaceutical agent in a test subject of a first species, the method comprising:
at a computer system comprising at least one processor and a memory storing at least one program for execution by the at least one processor:
inputting information about the test subject into a multi-task model comprising a plurality of parameters, wherein the multi-task model applies the plurality of parameters to the information about the test subject through a plurality of instructions to generate, as output from the multi-task model, a plurality of outputs comprising (i) a predicted effect of the pharmaceutical agent in the test subject and (ii) for each respective cell type variable in a set of one or more cell type variables, a corresponding cell type classification, wherein the information about the test subject comprises a plurality of abundance values, the plurality of abundance values comprising, for each respective cellular constituent in a plurality of cellular constituents, a corresponding abundance value for the abundance of the respective cellular constituent in a biological sample of the test subject.

2. The method of claim 1, wherein the predicted effect comprises a prediction for cell death of a cancer cell in the subject in response to administration of the pharmaceutical agent to the subject.

3. The method of claim 1 or 2, wherein the pharmaceutical agent is a chemotherapeutic agent, optionally wherein the pharmaceutical agent is selected from the group consisting of lenalidomid, pembrolizumab, trastuzumab, bevacizumab, rituximab, ibrutinib, human papillomavirus quadrivalent (types 6, 11, 16, and 18) vaccine, pertuzumab, pemetrexed, nilotinib, denosumab, abiraterone acetate, promacta, imatinib, everolimus, palbociclib, erlotinib, bortezomib, bortezomib, nivolumab, atezolizumab, daratumumab, enzalutamide, obinutuzumab, ruxolitinib, venetoclax, osimertinib, and pomalidomide.

4. The method of any one of claims 1-3, wherein the multi-task model comprises a partially connected neural network defining a plurality of tasks, each respective task in the plurality of tasks corresponding to a respective output in the plurality of outputs, wherein the partially-connected neural network comprises (a) a first set of layers shared between the plurality of tasks and (b) for each respective task in the plurality of tasks a corresponding second set of layers unique to the respective task.

5. The method of any one of claims 1-4, the method further comprising:
when the predicted effect of the pharmaceutical agent in the test subject satisfies a first set of one or more criterion, recommending a first therapy that comprises administration of the pharmaceutical agent to the subject; and
when the predicted effect of the pharmaceutical agent in the test subject does not satisfy the first set of one or more criterion, recommending a second therapy that is different from the first therapy.

6. The method of any one of claims 1-4, the method further comprising:
when the predicted effect of the pharmaceutical agent in the test subject satisfies a first set of one or more criterion, recommending the test subject for a clinical trial of the pharmaceutical agent to the subject; and
when the predicted effect of the pharmaceutical agent in the test subject does not satisfy the first set of one or more criterion, not recommending the test subject for the clinical trial.

7. A method for identifying one or more tissue organoids in a plurality of tissue organoids matching a biological property of a tissue in a subject, the method comprising:
at a computer system comprising at least one processor and a memory storing at least one program for execution by the at least one processor:
inputting information about the test subject into a multi-task model comprising a plurality of parameters and one or more hidden layers, wherein the multi-task model is trained to apply the plurality of parameters to the information about the test subject through a plurality of instructions to generate, as output from the multi-task model, a plurality of outputs comprising (i) a predicted effect of the pharmaceutical agent in the test subject and (ii) for each respective cell type variable in a set of one or more cell type variables, a corresponding cell type classification, wherein the information about the test subject comprises, for each respective cellular constituent in a plurality of cellular constituents, a corresponding abundance value for the abundance of the respective cellular constituent in a biological sample of the test subject;
obtaining a latent representation of the information about the test subject from a respective hidden layer in the one or more hidden layers; and
comparing the latent representation of the information about the test subject to a plurality of latent representations, wherein each respective latent representation in the plurality of latent representations is of information about a respective tissue organoid, in a plurality of tissue organoids, obtained from the multi-task model; and
identifying one or more respective tissue organoids, in the plurality of tissue organoids, that satisfy a set of one or more similarity criterion based on the comparing, thereby identifying the one or more tissue organoids matching the biological property of the tissue in the subject.

8. The method of claim 8, wherein the multi-task model comprises a partially connected neural network defining a plurality of tasks, each respective task in the plurality of tasks corresponding to a respective output in the plurality of outputs, wherein the partially-connected neural network comprises (a) a first set of layers shared between the plurality of tasks and (b) for each respective task in the plurality of tasks a corresponding second set of layers unique to the respective task, wherein:
the respective hidden layer is a respective layer in the first set of layers shared between the plurality of tasks, or
the respective hidden layer is a respective layer in a corresponding second set of layers unique to a respective task in the plurality of tasks.

9. The method of claim 8, wherein the output corresponding to the respective task is the predicted effect of the pharmaceutical agent in the test subject.

10. The method of any one of claims 8-9, wherein comparing the latent representation of the information about the test subject to the plurality of latent representations comprises clustering the latent representation of the information about the test subject with the plurality of latent representations, optionally wherein:
the clustering generates a set of clusters including a first respective cluster comprising the latent representation of the information about the test subject; and
the set of one or more similarity criterion comprises a criteria that the latent representation of information about the respective tissue organoid is in the first respective cluster.

11. The method of any one of claims 8-10, wherein:
comparing the latent representation of the information about the test subject to the plurality of latent representations comprises determining, for each respective tissue organoid in at least a subset of the plurality of tissue organoids, a corresponding geometric distance between the latent representation of the information about the test subject and the corresponding latent representation of the information about the respective tissue organoid; and
the set of one or more similarity criterion comprises a distance criteria, optionally wherein the distance criteria comprises that:
the latent representation of information about the respective tissue organoid is within a threshold geometric distance of the latent representation of the information about the test subject,
the latent representation of information about the respective tissue organoid is within a threshold number of closest latent representations, in the plurality of latent representations, to the latent representation of the information about the test subject, or
the latent representation of information about the respective tissue organoid is the closest latent representation, in the plurality of latent representations, to the latent representation of the information about the test subject.

12. The method of any one of claims 1-11, wherein the plurality of tissue organoids comprises a plurality of tumor organoids.

13. The method of any one of claims 1-12, wherein the multi-task model comprises a linear mapping function that transforms the plurality of abundance values into a first latent feature space comprising fewer dimensions than the number of respective cellular constituents in the plurality of cellular constituents.

14. The method of any one of claims 1-13, wherein the set of one or more cell type variables comprises a variable selected from the group consisting of cell histology, disease type, disease stage, disease grade, tissue type, and tissue site.

15. The method of any one of claims 1-14, wherein each respective cellular constituent in the plurality of cellular constituents is a different mRNA species.

16. The method of any one of claims 1-15, wherein the biological sample of the subject comprises a diseased tissue of the subject.

17. A method for training a model to predict an effect of a candidate pharmaceutical agent in a test subject of a first species, the method comprising:
at a computer system comprising at least one processor and a memory storing at least one program for execution by the at least one processor:
A) obtaining, for each respective training sample in a first plurality of training samples, wherein each respective training sample in the first plurality of training samples comprises a tissue organoid or tissue organoid culture, formed from cells of the first species, that has been exposed to a perturbation:
(i) a corresponding plurality of abundance values comprising, for each respective cellular constituent in a plurality of cellular constituents, a corresponding abundance value for the abundance of the respective cellular constituent in the respective training sample after exposure to the candidate pharmaceutical agent,
(ii) a corresponding experimentally measured effect of the candidate pharmaceutical agent on the respective training sample, and
(iii) a corresponding set of one or more cell type classifications comprising, for each respective cell type variable in a set of one or more cell type variables, a corresponding cell type classification for the respective cell type variable;
B) obtaining, for each respective training sample in a second plurality of training samples, wherein each respective training sample in the second plurality of training samples comprises a biological sample from a respective subject in a plurality of subjects of the first species:
(i) a corresponding plurality of abundance values comprising, for each respective cellular constituent in the plurality of cellular constituents, a corresponding abundance value for the abundance of the respective cellular constituent in the respective training sample, and
(ii) a corresponding set of one or more cell type classifications comprising, for each respective cell type variable in the set of one or more cell type variables, a corresponding cell type classification for the respective cell type variable;
C) performing a first dimensionality reduction analysis across the corresponding plurality of abundance values for each respective training sample in the first plurality of training samples, thereby:
learning a first mapping function that maps a corresponding plurality of abundance values comprising, for each respective cellular constituent in the plurality of cellular constituents, a corresponding abundance value for the abundance of the respective cellular constituent in a tissue organoid or tissue organoid culture, formed from cells of the first species, that has been exposed to a perturbation, into a first latent feature space comprising a first plurality of dimensions that is less than the number of cellular constituents in the plurality of constituents, and
generating, for each respective training sample in the first plurality of training samples, a first corresponding representation of the corresponding plurality of abundance values in the first latent feature space according to the first mapping function;
D) performing a second dimensionality reduction analysis across the corresponding plurality of abundance values for each respective training sample in the second plurality of training samples, thereby:
learning a second mapping function that maps a corresponding plurality of abundance values comprising, for each respective cellular constituent in the plurality of cellular constituents, a corresponding abundance value for the abundance of the respective cellular constituent in a biological sample of the first species, into a second latent feature space comprising the first plurality of dimensions, and
generating, for each respective training sample in the second plurality of training samples, a corresponding representation of the corresponding plurality of abundance values in the second latent feature space according to the second mapping function;
E) learning a third mapping function that maps a representation of a corresponding plurality of abundance values in the first latent feature space to the second latent feature space;
F) generating, for each respective training sample in the first plurality of training samples, a second corresponding representation of the corresponding plurality of abundance values in the second latent feature space according to the third mapping function;
G) inputting, for each respective training sample in the first plurality of training samples, corresponding information about the respective training sample into a multi-task model comprising a plurality of parameters, wherein the multi-task model applies the plurality of parameters to the information about the training subject through a plurality of instructions to generate, as output from the multi-task model, a corresponding plurality of outputs, wherein:
the corresponding plurality of outputs comprises (i) a predicted effect of the candidate pharmaceutical agent on the respective training sample and (ii) for each respective cell type variable in the set of one or more cell type variables, a corresponding cell type classification for the respective training sample, and
the information about the respective training sample comprises the second corresponding representation of the corresponding plurality of abundance values in the second latent feature space;
H) inputting, for each respective training sample in the second plurality of training samples, corresponding information about the respective training sample into the multi-task model, wherein the information about the respective training sample comprises the corresponding representation of the corresponding plurality of abundance values in the second latent feature space; and
I) adjusting the plurality of parameters based on:
for each respective training sample in the first plurality of training samples, one or more differences between (i) the corresponding plurality of outputs and (ii) a set of labels comprising (a) the corresponding experimentally measured effect of the candidate pharmaceutical agent on the respective training sample and (b) the corresponding cell type classification for each respective cell type variable in the set of one or more cell type variables, and
for each respective training sample in the second plurality of training samples, one or more differences between (i) the corresponding plurality of outputs and (ii) a set of labels comprising the corresponding cell type classification for each respective cell type variable in the set of one or more cell type variables.

18. A computer system, comprising:
one or more processors; and
a non-transitory computer-readable medium including computer-executable instructions that, when executed by the one or more processors, cause the processors to perform the method according to any one of claims 1-17.

19. A non-transitory computer-readable storage medium having stored thereon program code instructions that, when executed by a processor, cause the processor to perform the method according to any one of claims 1-17.
